# EUROPEAN PATENT APPLICATION

(11) **EP 1 031 572 A1**
(43) Date of publication of application: **30.08.2000**
(21) Application number: 98953051.4
(22) Date of filing: 13.11.1998
(51) Int. Cl.: C07D 409/14, C07D 411/14, C07D 417/14, A61K 31/44, A61K 31/54

(54) **PYRIDYLPYRROLE DERIVATIVES**

(30) Priority: 14.11.1997 JP 31398897
(71) Applicant: Sankyo Company Limited, Chuo-ku, Tokyo 103-8426 (JP)
(72) Inventor: KIMURA, Tomio, Sankyo Company, Limited, Shinagawa-ku Tokyo 140-8710 (JP); KAWARA, Akihiro, Sankyo Company, Limited, Shinagawa-ku Tokyo 140-8710 (JP); NAKAO, Akira, Sankyo Company, Limited, Shinagawa-ku Tokyo 140-8710 (JP); SUZUKI, Keisuke, Sankyo Company, Limited, Shinagawa-ku Tokyo 140-8710 (JP); USHIYAMA, Shigeru, Sankyo Company, Limited, Shinagawa-ku Tokyo 140-8710 (JP); SHIMOZATO, Takaichi, Sankyo Company, Limited, Shinagawa-ku Tokyo 140-8710 (JP)
(74) Representative: Gibson, Christian John Robert
(86) International application number: JP9805134
(87) International publication number: WO9925717

(57) **Abstract**

Object: to provide compounds having an inhibitory effect on the production of inflammatory cytokines. Constitution: compounds having general formula (I), pharmacologically acceptable salts thereof or derivatives of the same, wherein A: single bond, oxygen, sulfur, CO, SO, SO₂, C(R⁹)(R¹⁰) [R⁹, R¹⁰: H, OH, halogeno, alkyl], N(R¹¹), C=NOR¹¹ [R¹¹: H, alkyl]; D: single bond, C(R¹²)(R¹³) [R¹², R¹³: H, OH, halogeno, alkyl]; R¹ : optionally substituted pyridyl; R² : optionally substituted phenyl; R³ : halogeno, alkyl, haloalkyl, alkoxy, haloalkoxy; R⁴, R⁵, R⁶, R⁷, R⁸ : H, alkyl; k: 0-3; and m: 1, 2.

## Description

### [Technical Field]

The present invention relates to pyridylpyrrole derivatives useful as a medicament. More specifically, this invention pertains to pyridylpyrrole derivatives which have an inhibitory activity against the production of inflammatory cytokines such as interleukin (IL)-1, IL-6, IL-8 and tumor necrosis factor (TNF) and which are useful as an antipyretic, analgesic, antiinflammatory drug or a medicament for autoimmune diseases such as chronic rheumatism, for bone diseases such as osteoporosis or for treatment of diseases in which the above-described cytokines take part.

### [Background of the Invention]

Nonsteroidal antiinflammatory drugs (NSAID) have been used frequently for treatment of various inflammatory diseases and pain, since they have, as their main pharmacological activity, antipyretic, analgesic, and antiinflammatory activity based on the mechanism of inhibiting the biosynthesis of prostaglandin (PG) through cyclooxygenase inhibitory action. For the treatment of chronic rheumatism, NSAIDs are used nosotropically and immunomodulators (DMARD) are used etiopathically.

Conventional NSAIDs however induce disorders in the digestive tract function such as gastric ulcers owing to their mechanism of action and therefore involve a problem in the continuous administration of the NSAIDs for a long period of time. DMARD has not yet exhibited a definite stable effect. Recently, active substances generally called cytokines, produced by immunocytes, have been found. Among them, interleukin (IL)-1, IL-6, IL-8, tumor necrosis factor (TNF) and the like are called inflammatory cytokines, and their versatile functions as an inflammatory mediator for the activation of an arachidonic acid metabolism system which is a production system of PG, migration of leukocytes, derivation of acute phase protein, activation of osteoclasts and the like have been elucidated. Medicaments to inhibit the production of such inflammatory cytokines are therefore expected to be a new generation antipyretic, analgesic, and antiinflammatory drug or a medicament for autoimmune diseases such as chronic rheumatism, for bone diseases such as osteoporosis and for the diseases in which the above-described cytokines take part, due to their mechanism of action which is different from that of the conventional drugs.

As a heteroaryl compound having inhibitory activity against the production of such inflammatory cytokines, the below-described compounds are, for example, disclosed specifically in the specification of WO97/5878. There is however a demand for the development of further compounds superior to them in activity, pharmacokinetics and safety.

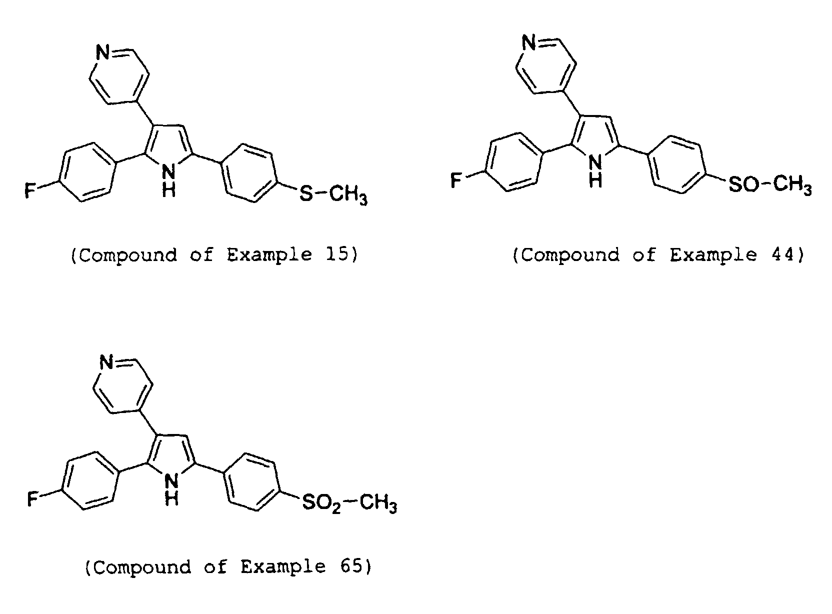

### [Disclosure of the Invention]

The present inventors carried out an intensive investigation for many years on the synthesis and pharmacological activities of pyrrole derivatives capable of inhibiting the production of the above-described inflammatory cytokines. As a result, it has been found that pyridylpyrrole derivatives having a special substituent on the pyrrole ring exhibit excellent inhibitory activity against the production of inflammatory cytokines, leading to the completion of the present invention.

The present invention relates to:
(1) compounds represented by the following formula (I): 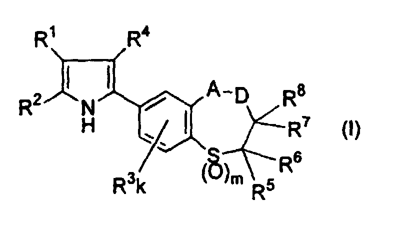 [wherein,
   A represents a single bond, an oxygen atom, a sulfur atom, a carbonyl group. -SO-, -SO₂-, -C(R⁹)(R¹⁰)- (in which, R⁹ and R¹⁰ are the same or different and each independently represents a hydrogen atom, a hydroxyl group, a halogen atom or a lower alkyl group), -N(R¹¹)- (in which, R¹¹ represents a hydrogen atom or a lower alkyl group), or =C=NOR¹¹ (wherein, R¹¹ has the same meaning as described above),
   D represents a single bond or -C(R¹²)(R¹³)- (in which, R¹² and R¹³ are the same or different and each independently represents a hydrogen atom, a hydroxyl group, a halogen atom or a lower alkyl group),
   R¹ represents an unsubstituted pyridyl group or a pyridyl group substituted with at least one group selected from Substituent group α,
   R² represents an unsubstituted phenyl group or a phenyl group substituted with at least one group selected from Substituent group α,
   R³ represents a halogen atom, a lower alkyl group, a lower halogeno alkyl group, a lower alkoxy group or a lower halogeno alkoxy group,
   R⁴, R⁵, R⁶, R⁷ and R⁸ are the same or different and each independently represents a hydrogen atom or a lower alkyl group,
   k is an integer of 0 to 3 (when k is 2 or 3, plural R³ groups may be the same or different) and
   m is 1 or 2,
   Substituent group α:
   a halogen atom, a lower alkyl group, a lower halogeno alkyl group, a lower alkoxy group, a lower halogeno alkoxy group, a lower alkylthio group];
   and pharmacologically acceptable salts or derivatives thereof.

   Among the above-described compounds, preferred are:
(2) the compounds wherein R¹ represents an unsubstituted 4-pyridyl group or a 4-pyridyl group substituted with at least one group selected from Substituent group α.
(3) the compounds wherein R¹ represents an unsubstituted 4-pyridyl group,
(4) the compounds wherein R² represents an unsubstituted phenyl group or a phenyl group substituted with 1 to 3 substituents selected from Substituent group α,
(5) the compounds wherein R² represents an unsubstituted phenyl group or a phenyl group substituted with 1 to 3 substituents selected from below-described Substituent group α¹,
(6) the compounds wherein R² represents an unsubstituted phenyl group or a phenyl group substituted with 1 to 3 substituents selected from below-described Substituent group α²,
(7) the compounds wherein R³ represents a halogen atom, a C₁₋₄ alkyl group, a C₁₋₄ halogenoalkyl group, a C₁₋₄ alkoxy group or a C₁₋₄ halogenoalkoxy group,
(8) the compounds wherein R³ represents a fluorine atom, a chlorine atom, a methyl group, a methoxy group or a difluoromethoxy group,
(9) the compounds wherein k is 0,
(10) the compounds wherein R⁴ represents a hydrogen atom or a C₁₋₄ alkyl group,
(11) the compounds wherein R⁴ represents a hydrogen atom, a methyl group or an ethyl group,
(12) the compounds wherein R⁵, R⁶, R⁷ and R⁸ are the same or different and each independently represents a hydrogen atom or a C₁₋₄ alkyl group,
(13) the compounds wherein R⁵, R⁶, R⁷ and R⁸ are the same or different and each independently represents a hydrogen atom or a methyl group,
(14) the compounds wherein R⁹ and R¹⁰ are the same or different and each independently represents a hydrogen atom, a hydroxyl group, a halogen atom or a C₁₋₄ alkyl group,
(15) the compounds wherein R⁹ and R¹⁰ are the same or different and each independently represents a hydrogen atom, a hydroxyl group, a fluorine atom, a methyl group or an ethyl group,
(16) the compounds wherein R¹¹ represents a hydrogen atom or a C₁₋₄ alkyl group,
(17) the compounds wherein R¹¹ represents a hydrogen atom, a methyl group or an ethyl group,
(18) the compounds wherein A represents a single bond, an oxygen atom, a carbonyl group, -SO-, -SO₂-, -C(R⁹)(R¹⁰)-, -N(R¹¹)- or =C=NOR¹¹,
(19) the compounds wherein A represents a single bond, an oxygen atom, a carbonyl group, -SO₂-, -C(R⁹)(R¹⁰)-, -N(R¹¹)- or =C=NOR¹¹,
(20) the compounds wherein A represents a single bond, an oxygen atom, a carbonyl group, -C(R⁹)(R¹⁰)-, -N(R¹¹)- or =C=NOR¹¹, and
(21) the compounds wherein A represents a single bond, an oxygen atom, a carbonyl group, -C(R⁹)(R¹⁰)- or =C=NOR¹¹,
or pharmacologically acceptable salts or derivatives thereof.
Substituent group α¹:
a halogen atom, a C₁₋₄ alkyl group, a C₁₋₄ halogenoalkyl group, a C₁₋₄ alkoxy group, a C₁₋₄ halogenoalkoxyl group, a C₁₋₄ alkylthio group.
Substituent group α²:
a fluorine atom, a chlorine atom, a difluoromethoxy group.

In addition, among the above-described compounds (1), compounds which comprise any combination of the factors selected freely from nine groups consisting of (2) and (3), (4) to (6), (7) and (8), (9), (10) and (11), (12) and (13), (14) and (15), (16) and (17) and (18) to (21) are also preferred.

Another object of the present invention is to provide a medicament (particularly, a medicament for prevention or treatment of the diseases mediated by inflammatory cytokines) comprising, as an effective ingredient, a compound described in any group selected from the above-described groups (1) to (21) or a pharmacologically acceptable salt or derivative thereof. Examples of such a medicament include analgesics, antiinflammatory drugs, antiviral drugs, and medicaments for the prevention or treatment of chronic rheumatism, osteoarthritis, allergosis, asthma, sepsis, psoriasis, osteoporosis, autoimmune diseases (e.g. systemic lupus erythematosus, ulcerative colitis, and Crohn's disease), diabetes, glomerular nephritis or arteriosclerosis, of which the analgesics, antiinflammatory drugs and medicaments for the prevention or treatment of chronic rheumatism, osteoarthritis, allergosis, sepsis, psoriasis, osteoporosis, ulcerative colitis, diabetes or arteriosclerosis are particularly suited.

In the above-described formula (I), the "lower alkyl group" in the definition of R³, R⁴, R⁵, R⁶, R⁷, R⁸, R⁹, R¹⁰, R¹¹, R¹², R¹³ and Substituent group α signifies a straight or branched C₁₋₆ alkyl group. Examples of the group include methyl, ethyl, propyl, isopropyl, butyl, isobutyl, s-butyl, t-butyl, pentyl, 2-pentyl, 3-pentyl, 2-methylbutyl, 3-methylbutyl, 1,1-dimethylpropyl, 1,2-dimethylpropyl, 2,2-dimethylpropyl, hexyl, 2-hexyl, 3-hexyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1,1,2-trimethylpropyl and 1,2,2-trimethylpropyl groups, of which C₁₋₄ alkyl groups are preferred and methyl, ethyl and propyl groups are more preferred.

When R³ represents a lower alkyl group, particularly preferred are methyl and ethyl groups, of which the most preferred are methyl groups.

When R⁵, R⁶, R⁷ and/or R⁸ represent a lower alkyl group, particularly preferred are methyl groups.

When R⁴, R⁹, R¹⁰, R¹¹, R¹², R¹³ and/or Substituent group a represent a lower alkyl group, particularly preferred are methyl and ethyl groups.

The "halogen atom" in the definition of R³, R⁹, R¹⁰, R¹², R¹³ and Substituent group α signifies a fluorine atom, chlorine atom, bromine atom or iodine atom, of which fluorine and chlorine atoms are preferred. When R⁹ or R¹⁰ represents a halogen atom, fluorine atoms are particularly preferred.

The "lower halogeno alkyl group" in the definition of R³ and Substituent group α means the above-described "lower alkyl group" substituted with the above-described "halogen atom" and signifies a straight or branched C₁₋₆ halogenoalkyl group such as trifluoromethyl, trichloromethyl, difluoromethyl, dichloromethyl, dibromomethyl, fluoromethyl, chloromethyl, bromomethyl, 2,2,2-trichloroethyl, 2,2,2-trifluoroethyl, 2-bromoethyl, 2-chloroethyl, 2-fluoroethyl, 2,2-dibromoethyl, 3-fluoropropyl or 4-fluorobutyl, of which C₁₋₄ halogenoalkyl groups such as trifluoromethyl, trichloromethyl, difluoromethyl, fluoromethyl, chloromethyl, bromomethyl, 2-fluoroethyl, 3-fluoropropyl and 4-fluorobutyl groups are preferred.

The "lower alkoxy group" in the definition of R³ and Substituent group α means the above-described "lower alkyl group" having an oxygen atom attached thereto and signifies a straight or branched C₁₋₆ alkoxy group such as methoxy, ethoxy, propoxy, isopropoxy, butoxy, isobutoxy, s-butoxy, t-butoxy, pentyloxy, 2-pentyloxy, 3-pentyloxy, 2-methylbutoxy, 3-methylbutoxy, 1,1-dimethylpropoxy, 1,2-dimethylpropoxy, 2,2-dimethylpropoxy, hexyloxy, 2-hexyloxy, 3-hexyloxy, 2-methylpentyloxy, 3-methylpentyloxy, 4-methylpentyloxy, 1,1-dimethylbutoxy, 1,2-dimethylbutoxy, 1,3-dimethylbutoxy, 2,2-dimethylbutoxy, 2,3-dimethylbutoxy, 3,3 - dimethylbutoxy, 1,1,2-trimethylpropoxy and 1,2,2-trimethylpropoxy groups, of which C₁₋₄ alkoxy groups are preferred and methoxy groups are more preferred.

The "lower halogeno alkoxy group" in the definition of R³ and Substituent group α means the above-described "lower halogeno alkyl group" having an oxygen atom attached thereto and signifies a straight or branched C₁₋₆ halogenoalkoxy group such as fluoromethoxy, difluoromethoxy, trifluoromethoxy, 2-fluoroethoxy, 3-fluoropropoxy, 4-fluorobutoxy, 2-chloroethoxy and 2-bromoethoxy groups, of which C₁₋₄ halogenoalkoxy groups are preferred, difluoromethoxy and trifluoromethoxy groups are more preferred and difluoromethoxy groups are particularly preferred.

The "lower alkylthio group" in the definition of Substituent group α means the above-described "lower alkyl group" having a sulfur atom attached thereto and signifies a straight or branched C₁₋₆ alkylthio group such as methylthio, ethylthio, propylthio, isopropylthio, butylthio, isobutylthio, s-butylthio, t-butylthio, pentylthio, 2-pentylthio, 3-pentylthio, 2-methylbutylthio, 3-methylbutylthio, 1,1-dimethylpropylthio, 1,2-dimethylpropylthio, 2,2-dimethylpropylthio, hexylthio, 2-hexylthio, 3-hexylthio, 2-methylpentylthio, 3-methylpentylthio, 4-methylpentylthio, 1,1-dimethylbutylthio, 1,2-dimethylbutylthio, 1,3-dimethylbutylthio, 2,2-dimethylbutylthio, 2,3-dimethylbutylthio, 3,3-dimethylbutylthio, 1,1,2-trimethylpropylthio and 1,2,2-trimethylpropylthio groups, of which C₁₋₄ alkylthio groups are preferred and methylthio groups are more preferred.

In the formula (I), when k is 2 or 3, the plural groups of R³ may be the same or different. Preferably, k is 0, 1 or 2 and more preferably k is 0.

Since the compound of the present invention represented by the formula (I) can be converted into its salts if necessary, the term "pharmacologically acceptable salt" means said salts. Examples of such salts include acid addition salts, e.g., salts with a mineral acid such as hydrochloride, hydrobromide, hydroiodide, sulfate and phosphate, and salts with an organic acid such as methanesulfonate, ethanesulfonate, benzenesulfonate, p-toluenesulfonate, oxalate, maleate, fumarate, tartrate and citrate. The compound (I) of the present invention or a pharmacologically acceptable salt or derivative thereof may also exist as a solvate (e.g. hydrate) and the present invention embraces this.

When the compound (I) of the present invention has a hydroxyl group and/or imino group, compound (I) can be converted to a derivative by protecting the group with a "group removable by a chemical process such as hydrolysis, hydrogenolysis, electrolysis or photolysis" or a "group removable by a biological process such as hydrolysis in vivo". The "derivative" in the definition means the above-described derivative.

Whether a compound is a "derivative" protected with the "group removable by a chemical process such as hydrolysis, hydrogenolysis, electrolysis or photolysis" or not can be determined as follows: the compound is placed under the conditions ordinarily employed for a reaction such as hydrolysis, hydrogenolysis, electrolysis or photolysis. After a predetermined time, if the original compound or a pharmacologically acceptable salt thereof can be detected from the reaction phase, the compound thus studied is judged as a derivative.

Whether a compound is a "derivative" protected by a "group removable by a biological process such as hydrolysis in vivo" or not can be determined as follows: the compound is intravenously administered to an experimental animal such as a rat or mouse and the body fluid of the animal is thereafter studied. If the original compound or a pharmacologically acceptable salt thereof can be detected from the body fluid, the compound thus studied is judged as a derivative.

Preferred examples of the group forming a "derivative" based on a hydroxyl group include "aliphatic acyl groups", for example, alkylcarbonyl groups such as formyl, acetyl, propionyl, butyryl, isobutyryl, pentanoyl, pivaloyl, valeryl, isovaleryl, octanoyl, nonylcarbonyl, decylcarbonyl, 3-methylnonylcarbonyl, 8-methylnonylcarbonyl, 3-ethyloctylcarbonyl, 3,7-dimethyloctylcarbonyl, undecylcarbonyl, dodecylcarbonyl, tridecylcarbonyl, tetradecylcarbonyl, pentadecylcarbonyl, hexadecylcarbonyl, 1-methylpentadecylcarbonyl, 14-methylpentadecylcarbonyl, 13,13-dimethyltetradecylcarbonyl, heptadecylcarbonyl, 15-methylhexadecylcarbonyl, octadecylcarbonyl, 1-methylheptadecylcarbonyl, nonadecylcarbonyl, eicosylcarbonyl and heneicosylcarbonyl groups, halogenated alkylcarbonyl groups such as chloroacetyl, dichloroacetyl, trichloroacetyl and trifluoroacetyl groups, lower alkoxyalkylcarbonyl groups such as methoxyacetyl groups and unsaturated alkylcarbonyl groups such as acryloyl, propioloyl, methacryloyl, crotonoyl, isocrotonoyl and (E)-2-methyl-2-butenoyl groups; "aromatic acyl groups", for example, arylcarbonyl groups such as benzoyl, α-naphthoyl and β-naphthoyl groups, halogenated arylcarbonyl groups such as 2-bromobenzoyl and 4-chlorobenzoyl groups, lower alkylated arylcarbonyl groups such as 2,4,6-trimethylbenzoyl and 4-toluoyl groups, lower alkoxylated arylcarbonyl groups such as 4-anisoyl groups, nitrated arylcarbonyl groups such as 4-nitrobenzoyl and 2-nitrobenzoyl groups, lower alkoxycarbonylated arylcarbonyl groups such as 2-(methoxycarbonyl)benzoyl groups and arylated arylcarbonyl groups such as 4-phenylbenzoyl groups; "tetrahydropyranyl or tetrahydrothiopyranyl groups" such as tetrahydropyran-2-yl, 3-bromotetrahydropyran-2-yl, 4-methoxytetrahydropyran-4-yl groups, tetrahydrothiopyran-2-yl and 4-methoxytetrahydrothiopyran-4-yl groups; "tetrahydrofuranyl or tetrahydrothiofuranyl groups" such as tetrahydrofuran-2-yl groups and tetrahydrothiofuran-2-yl groups; "silyl groups", for example, tri(lower alkyl)silyl groups such as trimethylsilyl, triethylsilyl, isopropyldimethylsilyl, t-butyldimethylsilyl, methyldiisopropylsilyl, methyl-di-t-butylsilyl and triisopropylsilyl groups, and tri(lower alkyl)silyl groups substituted with 1 or 2 aryl groups such as diphenylmethylsilyl, diphenylbutylsilyl, diphenylisopropylsilyl and phenyldiisopropylsilyl groups; "alkoxymethyl groups", for example, lower alkoxymethyl groups such as methoxymethyl, 1,1-dimethyl-1-methoxymethyl, ethoxymethyl, propoxymethyl, isopropoxymethyl, butoxymethyl and t-butoxymethyl groups, lower alkoxylated lower alkoxymethyl groups such as 2-methoxyethoxymethyl groups and lower halogeno alkoxymethyl groups such as 2,2,2-trichloroethoxymethyl and bis(2-chloroethoxy)methyl groups; "substituted ethyl groups", for example, lower alkoxylated ethyl groups such as 1-ethoxyethyl and 1-(isopropoxy)ethyl groups and halogenated ethyl groups such as 2,2,2-trichloroethyl groups; "aralkyl groups", for example, lower alkyl groups substituted with 1 to 3 aryl groups such as benzyl, α-naphthylmethyl, β-naphthylmethyl, diphenylmethyl, triphenylmethyl, α-naphthyldiphenylmethyl and 9-anthrylmethyl groups and lower alkyl groups substituted with 1 to 3 aryl groups each having an aryl ring substituted with a lower alkyl, lower alkoxy, nitro, halogen or cyano group, for example, 4-methylbenzyl, 2,4,6-trimethylbenzyl, 3,4,5-trimethylbenzyl, 4-methoxybenzyl, 4-methoxyphenydiphenylmethyl, 2-nitrobenzyl, 4-nitrobenzyl, 4-chlorobenzyl, 4-bromobenzyl and 4-cyanobenzyl groups, "alkoxycarbonyl groups", for example, lower alkoxycarbonyl groups such as methoxycarbonyl, ethoxycarbonyl, propoxycarbonyl, butoxycarbonyl, s-butoxycarbonyl, t-butoxycarbonyl and isobutoxycarbonyl groups and lower alkoxycarbonyl groups substituted with a halogen or tri(lower alkyl)silyl group such as 2,2,2-trichloroethoxycarbonyl and 2-trimethylsilylethoxycarbonyl groups; "alkenyloxycarbonyl groups" such as vinyloxycarbonyl and allyloxycarbonyl groups; "aralkyloxycarbonyl groups" having an aryl ring which may be substituted with one or two lower alkoxy or nitro groups such as benzyloxycarbonyl, 4-methoxybenzyloxycarbonyl, 3,4-dimethoxybenzyloxycarbonyl, 2-nitrobenzyloxycarbonyl and 4-nitrobenzyloxycarbonyl groups; 1-(acyloxy)"lower alkyl groups", for example, 1-("aliphatic acyl"oxy)"lower alkyl groups" such as formyloxymethyl, acetoxymethyl, dimethylaminoacetoxymethyl, propionyloxymethyl, butyryloxymethyl, pivaloyloxymethyl, valeryloxymethyl, isovaleryloxymethyl, hexanoyloxymethyl, 1-formyloxyethyl, 1-acetoxyethyl, 1-propionyloxyethyl, 1-butyryloxyethyl, 1-pivaloyloxyethyl, 1-valeryloxyethyl, 1-isovaleryloxyethyl, 1-hexanoyloxyethyl, 1-formyloxypropyl, 1-acetoxypropyl, 1-propionyloxypropyl, 1-butyryloxypropyl, 1-pivaloyloxypropyl, 1-valeryloxypropyl, 1-isovaleryloxypropyl, 1-hexanoyloxypropyl, 1-acetoxybutyl, 1-propionyloxybutyl, 1-butyryloxybutyl, 1-pivaloyloxybutyl, 1-acetoxypentyl, 1-propionyloxypentyl, 1-butyryloxypentyl, 1-pivaloyloxypentyl and 1-pivaloyloxyhexyl groups, 1-("cycloalkyl"carbonyloxy)"lower alkyl groups" such as cyclopentylcarbonyloxymethyl, cyclohexylcarbonyloxymethyl, 1-cyclopentylcarbonyloxyethyl, 1-cyclohexylcarbonyloxyethyl, 1-cyclopentylcarbonyloxypropyl, 1-cyclohexylcarbonyloxypropyl, 1-cyclopentylcarbonyloxybutyl and 1-cyclohexylcarbonyloxybutyl groups, and 1-("aromatic acyl"oxy)"lower alkyl groups" such as benzoyloxymethyl groups; "carbonyloxyalkyl groups", for example, (lower alkoxycarbonyloxy)alkyl groups such as methoxycarbonyloxymethyl, ethoxycarbonyloxymethyl, propoxycarbonyloxymethyl, isopropoxycarbonyloxymethyl, butoxycarbonyloxymethyl, isobutoxycarbonyloxymethyl, pentyloxycarbonyloxymethyl, hexyloxycarbonyloxymethyl, cyclohexyloxycarbonyloxymethyl, cyclohexyloxycarbonyloxy(cyclohexyl)methyl, 1-(methoxycarbonyloxy)ethyl, 1-(ethoxycarbonyloxy)ethyl, 1-(propoxycarbonyloxy)ethyl, 1-(isopropoxycarbonyloxy)ethyl, 1-(butoxycarbonyloxy)ethyl, 1-(isobutoxycarbonyloxy)ethyl, 1-(t-butoxycarbonyloxy)ethyl, 1-(pentyloxycarbonyloxy)ethyl, 1-(hexyloxycarbonyloxy)ethyl, 1-(cyclopentyloxycarbonyloxy)ethyl, 1-(cyclopentyloxycarbonyloxy)propyl, 1-(cyclohexyloxycarbonyloxy)propyl, 1-(cyclopentyloxycarbonyloxy)butyl, 1-(cyclohexyloxycarbonyloxy)butyl, 1-(cyclohexyloxycarbonyloxy)ethyl, 1-(ethoxycarbonyloxy)propyl, 2-(methoxycarbonyloxy)ethyl, 2-(ethoxycarbonyloxy)ethyl, 2-(propoxycarbonyloxy)ethyl, 2-(isopropoxycarbonyloxy)ethyl, 2-(butoxycarbonyloxy)ethyl, 2-(isobutoxycarbonyloxy)ethyl, 2-(pentyloxycarbonyloxy)ethyl, 2-(hexyloxycarbonyloxy)ethyl, 1-(methoxycarbonyloxy)propyl, 1-(ethoxycarbonyloxy)propyl, 1-(propoxycarbonyloxy)propyl, 1-(isopropoxycarbonyloxy)propyl, 1-(butoxycarbonyloxy)propyl, 1-(isobutoxycarbonyloxy)propyl, 1-(pentyloxycarbonyloxy)propyl, 1-(hexyloxycarbonyloxy)propyl, 1-(methoxycarbonyloxy)butyl, 1-(ethoxycarbonyloxy)butyl, 1-(propoxycarbonyloxy)butyl, 1-(isopropoxycarbonyloxy)butyl, 1-(butoxycarbonyloxy)butyl, 1-(isobutoxycarbonyloxy)butyl, 1-(methoxycarbonyloxy)pentyl, 1-(ethoxycarbonyloxy)pentyl, 1-(methoxycarbonyloxy)hexyl and 1-(ethoxycarbonyloxy)hexyl groups, and oxodioxolenylmethyl groups such as (5-phenyl-2-oxo-1,3-dioxolen-4-yl)methyl, [5-(4-methylphenyl)-2-oxo-1,3-dioxolen-4-yl]methyl, [5-(4-methoxyphenyl)-2-oxo-1,3-dioxolen-4-yl]methyl, [5-(4-fluorophenyl)-2-oxo-1,3-dioxolen-4-yl]methyl, [5-(4-chlorophenyl)-2-oxo-1,3-dioxolen-4-yl]methyl, (2-oxo-1,3-dioxolen-4-yl)methyl, (5-methyl-2-oxo-1,3-dioxolen-4-yl)methyl, (5-ethyl-2-oxo-1,3-dioxolen-4-yl)methyl, (5-propyl-2-oxo-1,3-dioxolen-4-yl)methyl, (5-isopropyl-2-oxo-1,3-dioxolen-4-yl)methyl and (5-butyl-2-oxo-1,3-dioxolen-4-yl)methyl groups; "phthalidyl groups" such as phthalidyl, dimethylphthalidyl and dimethoxyphthalidyl; "half-ester salt residues of succinic acid"; "phosphate ester salt residues"; "ester-forming residues of an amino acid or the like"; a carbamoyl group and "carbamoyl groups substituted with 1 or 2 lower alkyl groups" such as methylcarbamoyl, ethylcarbamoyl, propylcarbamoyl, isopropylcarbamoyl, butylcarbamoyl, isobutylcarbamoyl, s-butylcarbamoyl, t-butylcarbamoyl, dimethylcarbamoyl, diethylcarbamoyl and dipropylcarbamoyl groups; and "1-(acyloxy)alkyloxycarbonyl groups" such as pivaloyloxymethyloxycarbonyl groups; of which the above-described "aliphatic acyl groups" are preferred and C₁₋₁₀ aliphatic acyl groups are more preferred and C₁₋₄ aliphatic acyl groups are most preferred.

Preferred examples of the group forming a "derivative" based on an imino group include the above-described "alkylcarbonyl groups" (preferably C₁₋₅ alkylcarbonyl groups); C₁₋₄ alkylsulfonyl groups such as methylsulfonyl, ethylsulfonyl, propylsulfonyl, isopropylsulfonyl, butylsulfonyl, isobutylsulfonyl, s-butylsulfonyl and t-butylsulfonyl groups; C₆₋₁₀ arylsulfonyl groups such as phenylsulfonyl, 1-naphthylsulfonyl and 2-naphthylsulfonyl groups: a carbamoyl group; the above-described "carbamoyl groups substituted with 1 to 2 lower alkyl groups"; the above-described "lower alkoxycarbonyl groups"; the above-described "aralkyloxycarbonyl groups"; the above-described "carbonyloxyalkyl groups" (preferably, the above-described 1-("aliphatic acyl"oxy)"lower alkyl groups", the above-described (lower alkoxycarbonyloxy)alkyl groups and the above-described oxodioxolenylmethyl groups); and the above-described "phthalidyl groups". More preferred examples include formyl, acetyl, propionyl, methylsulfonyl, ethylsulfonyl. carbamoyl, methylcarbamoyl, methoxycarbonyl, ethoxycarbonyl, propoxycarbonyl, acetoxymethyl, propionyloxymethy, butyryloxymethyl, pivaloyloxymethyl. methoxycarbonyloxymethyl, ethoxycarbonyloxymethyl, propoxycarbonyloxymethyl and (5-methyl-2-oxo-1,3-dioxolen-4-yl)methyl groups, of which acetyl, propionyl, methylsulfonyl, methoxycarbonyl, ethoxycarbonyl, acetoxymethyl, propionyloxymethyl, butyryloxymethyl, pivaloyloxymethyl and (5-methyl-2-oxo-1,3-dioxolen-4-yl)methyl groups are particularly preferred.

The compound (I) according to the present invention sometimes has asymmetric centers and in such a case, there exist optical isomers (R-form, S-form). The present invention also embraces them.

Preferred examples of the pyridylpyrrole derivatives according to the present invention include the compounds as shown in the below-described tables.

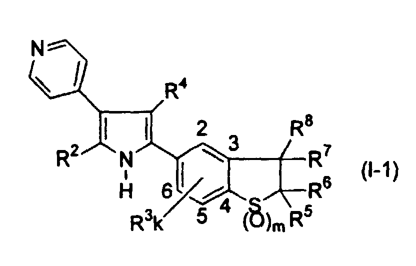

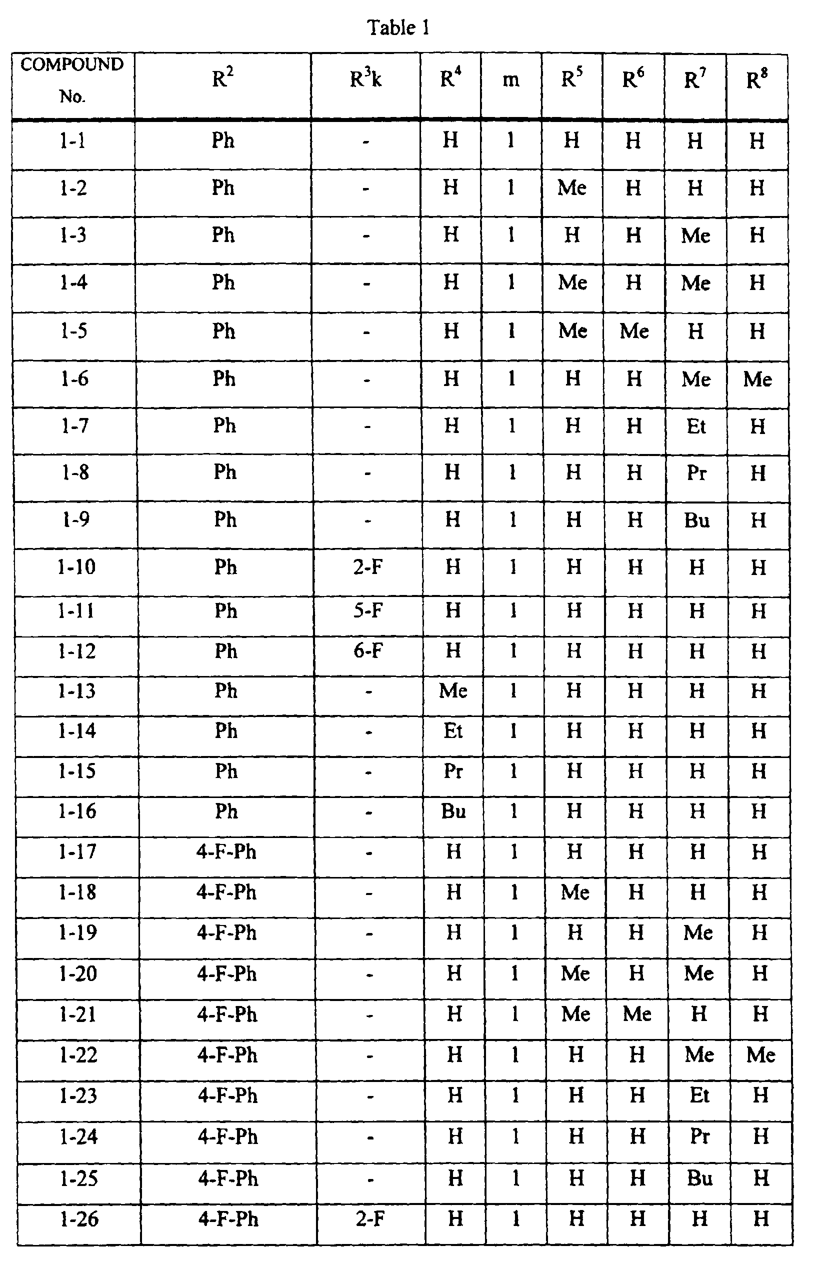

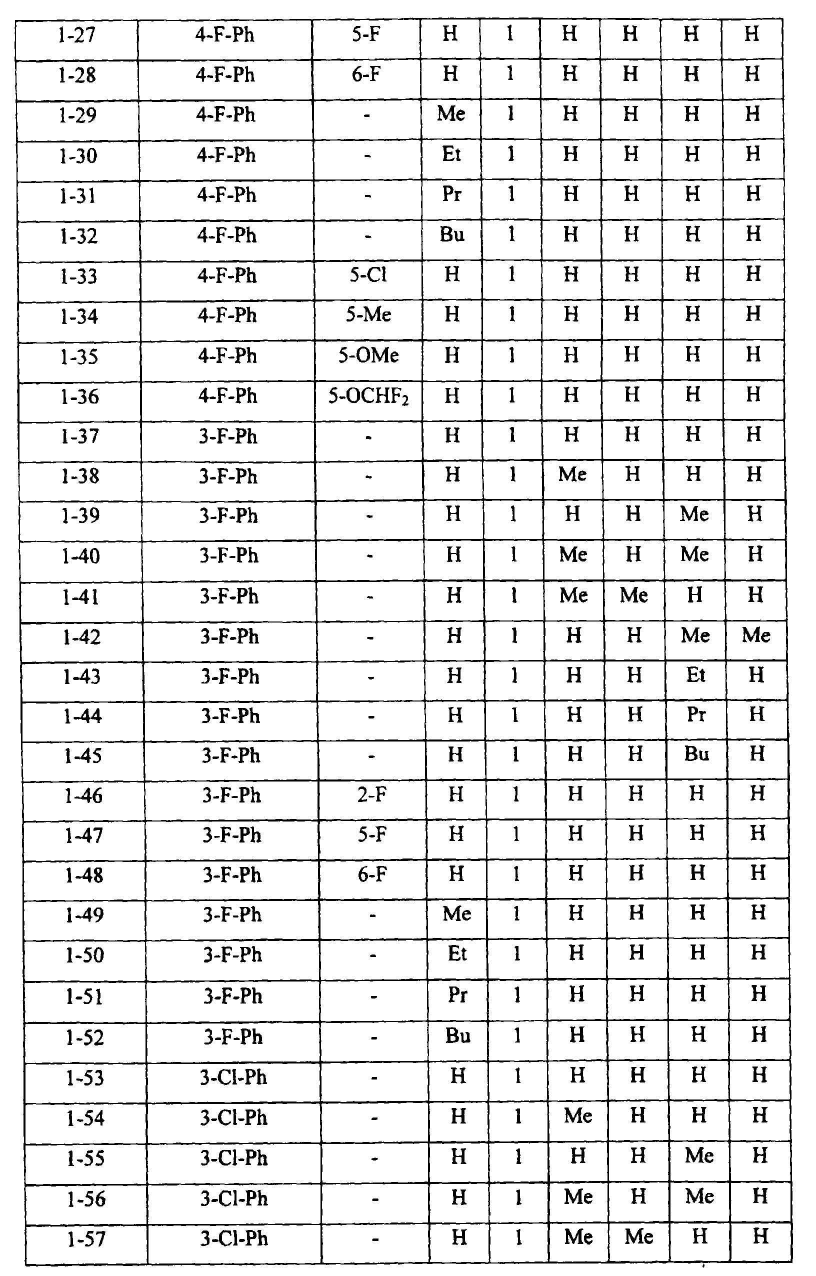

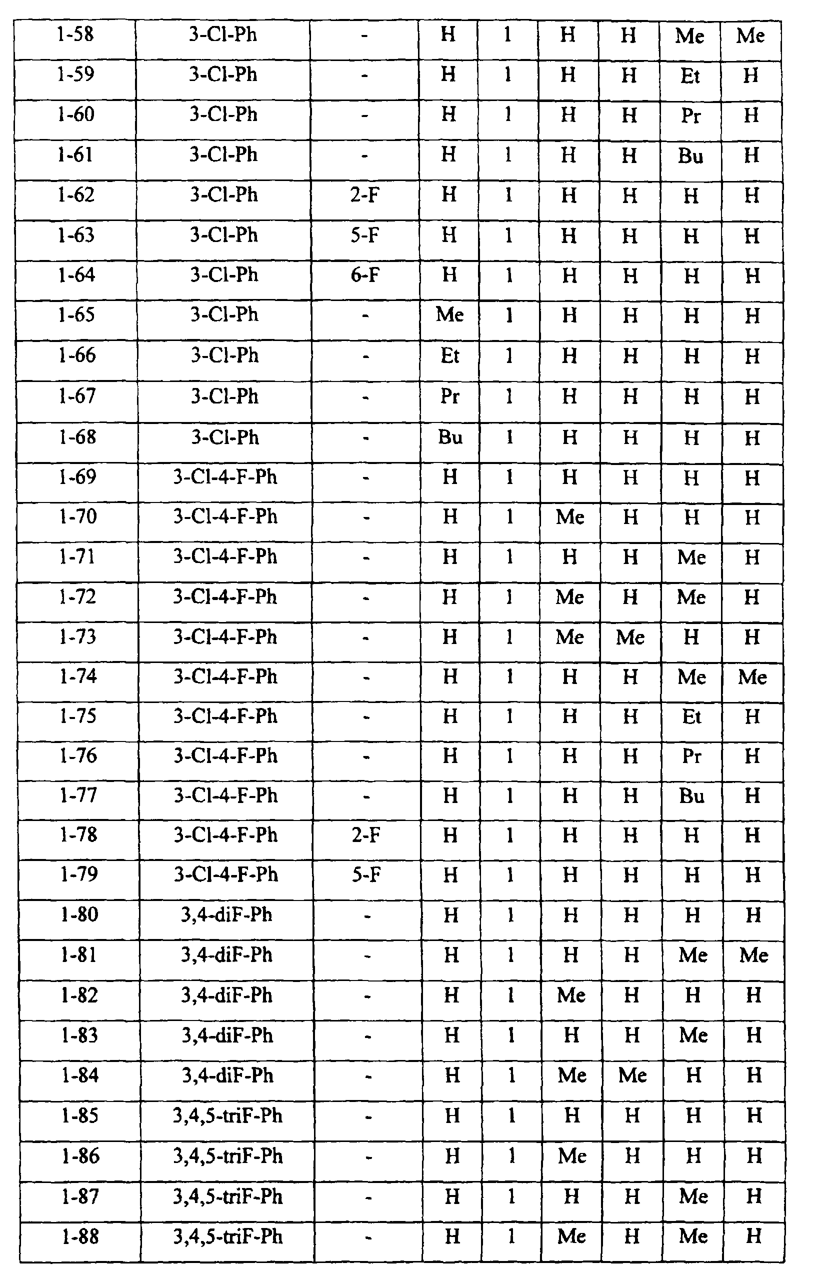

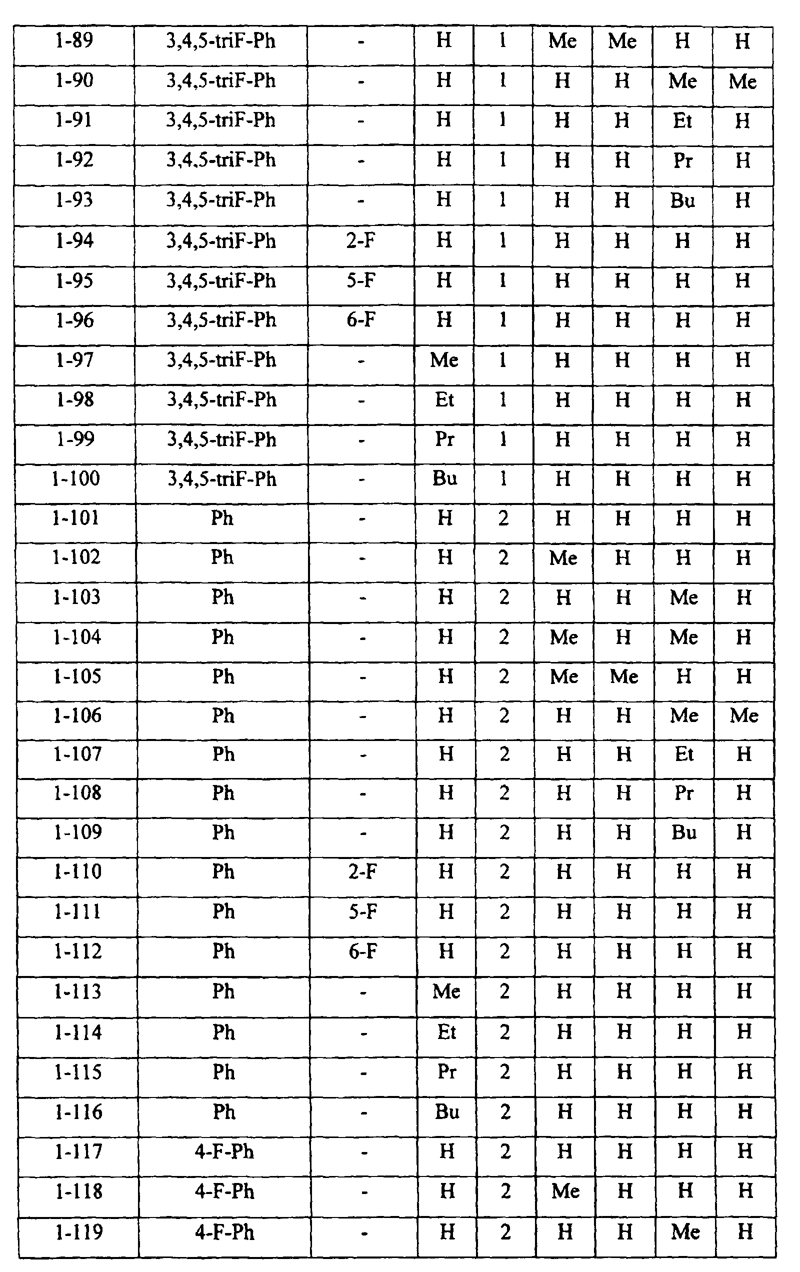

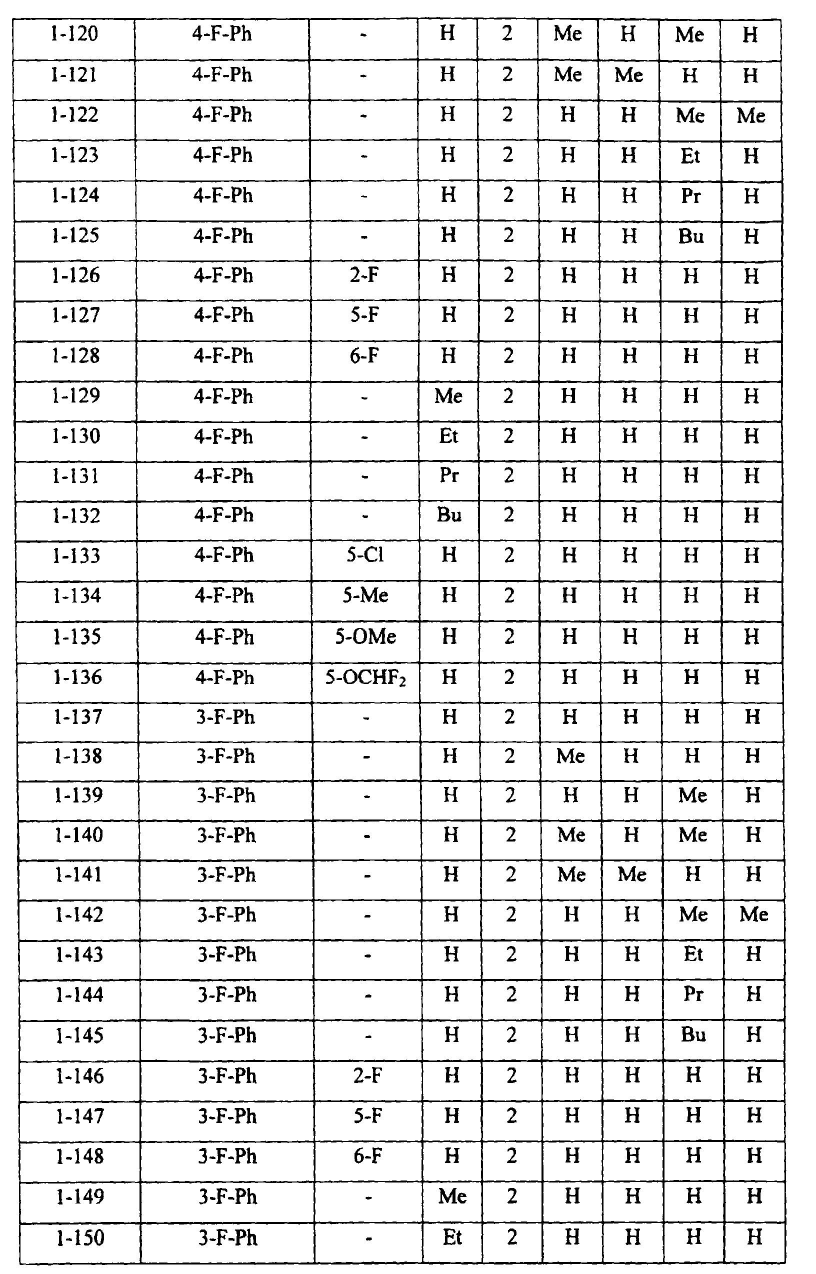

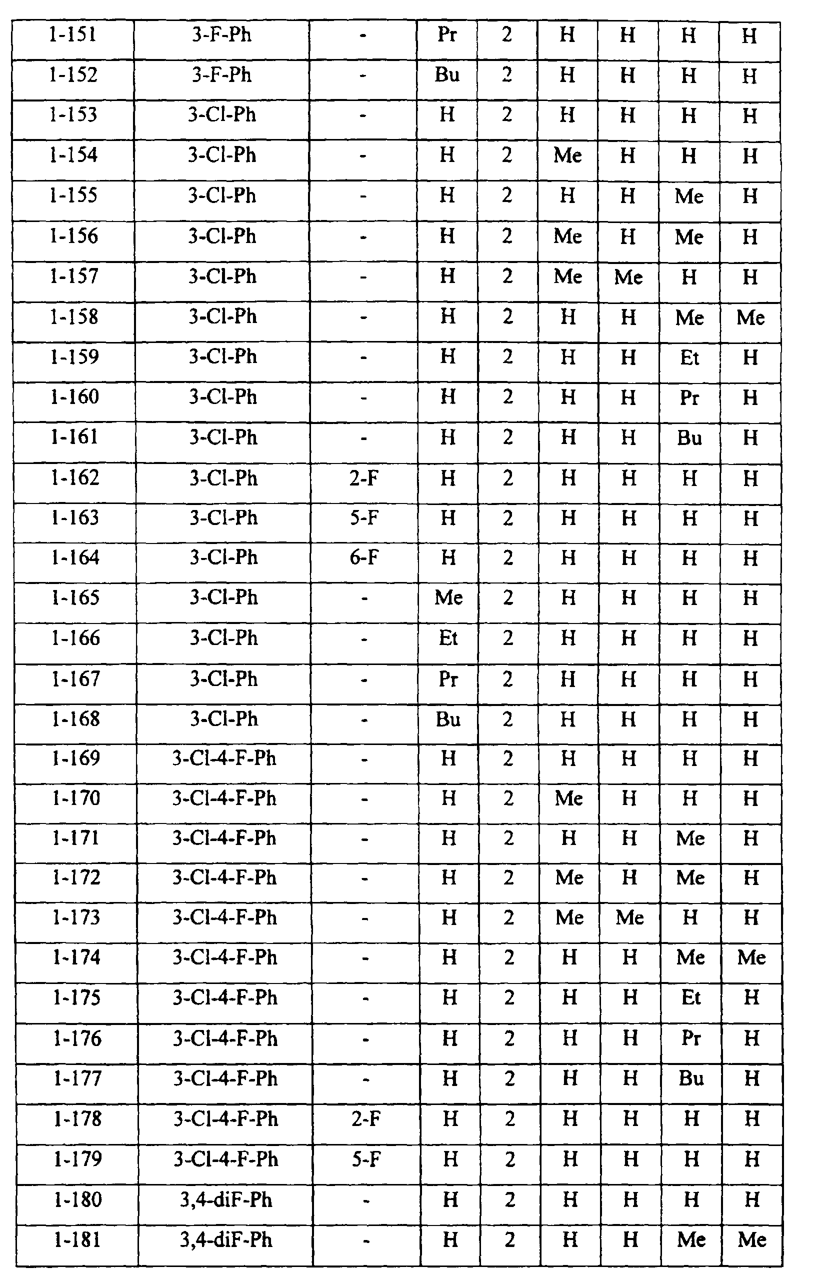

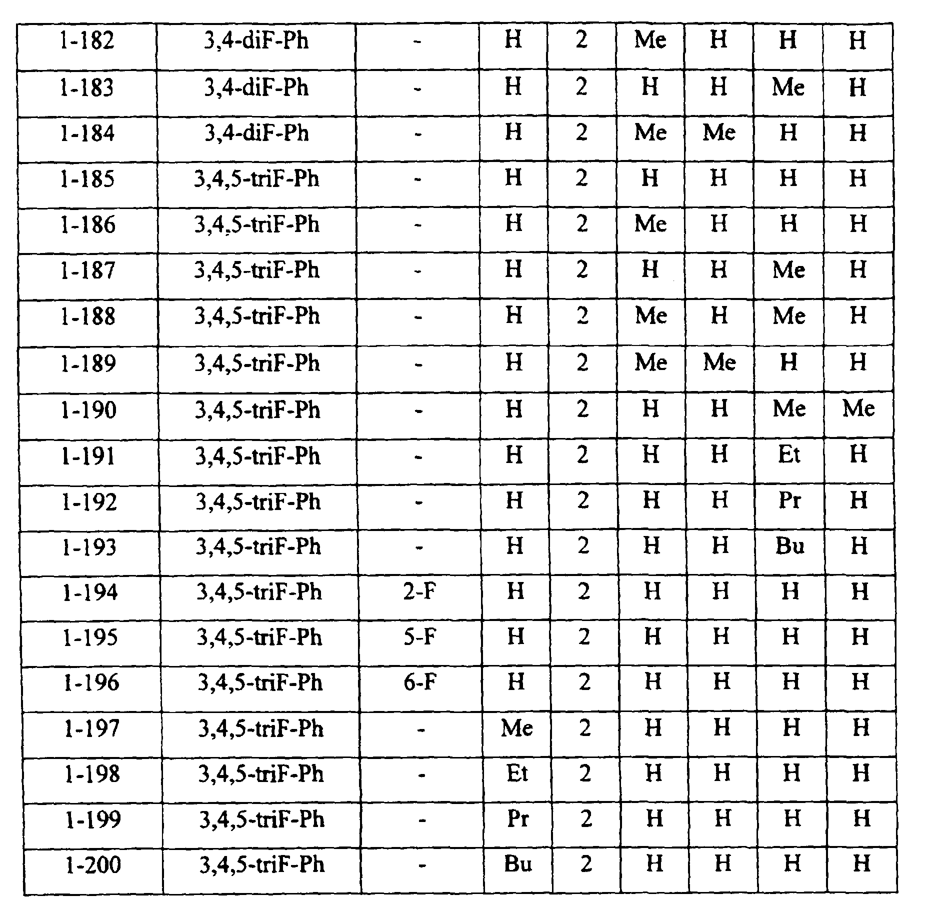

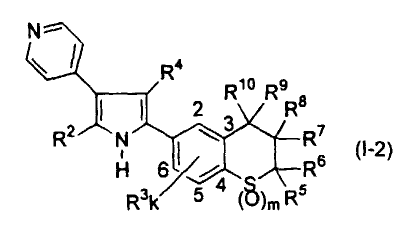

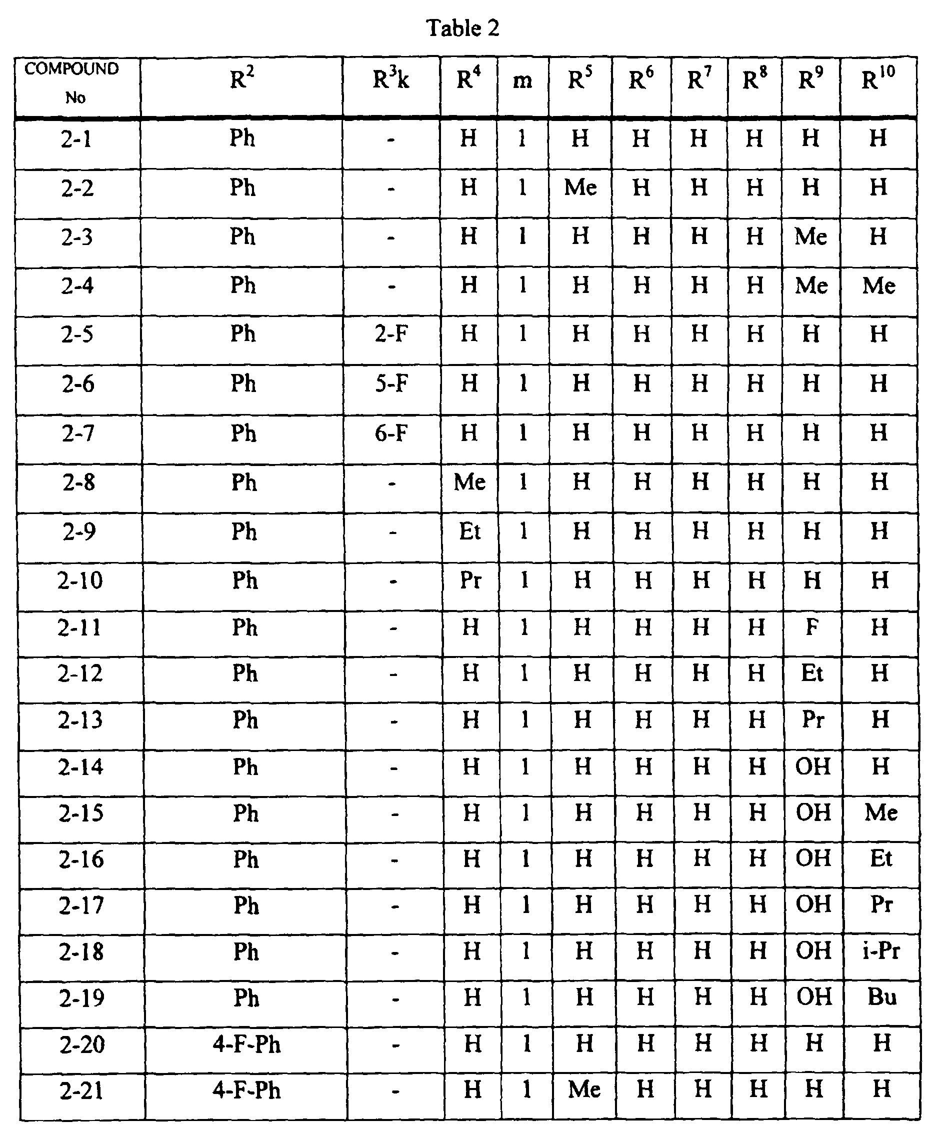

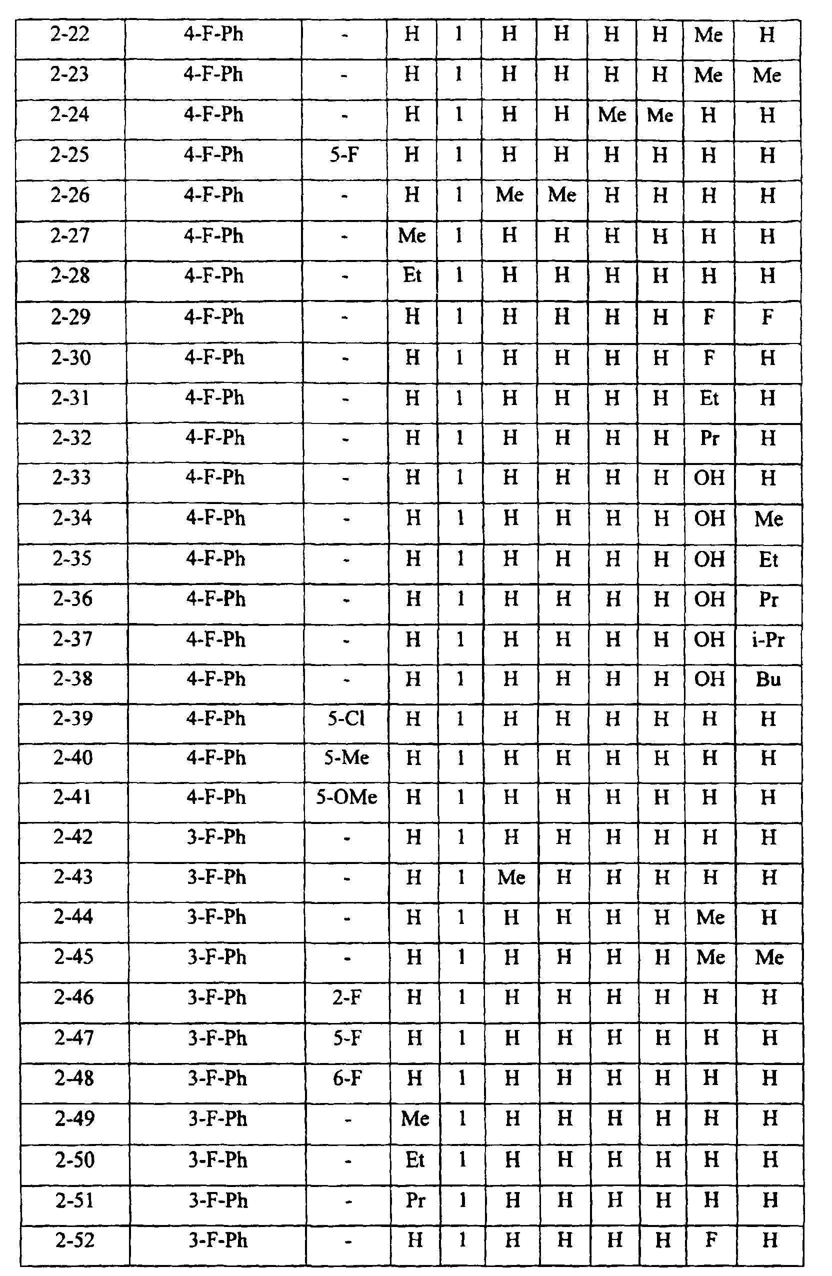

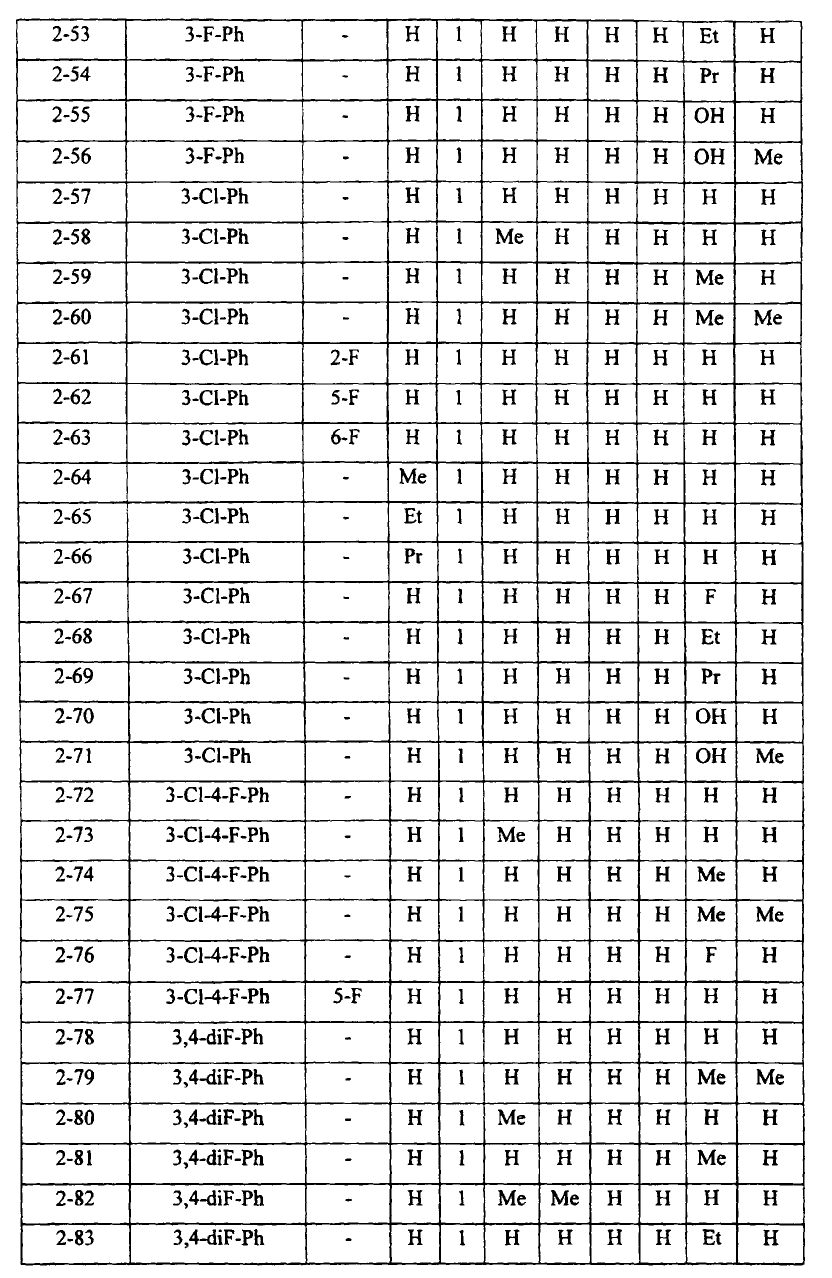

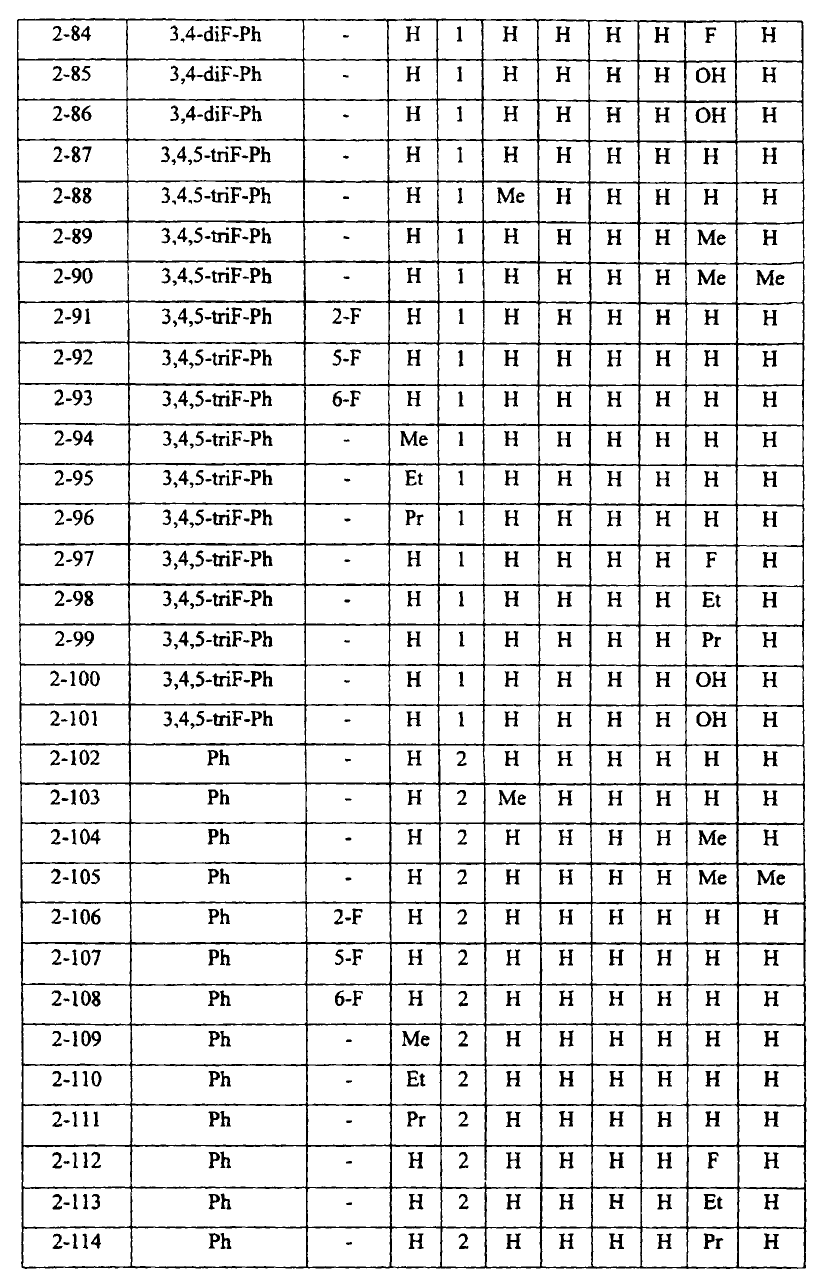

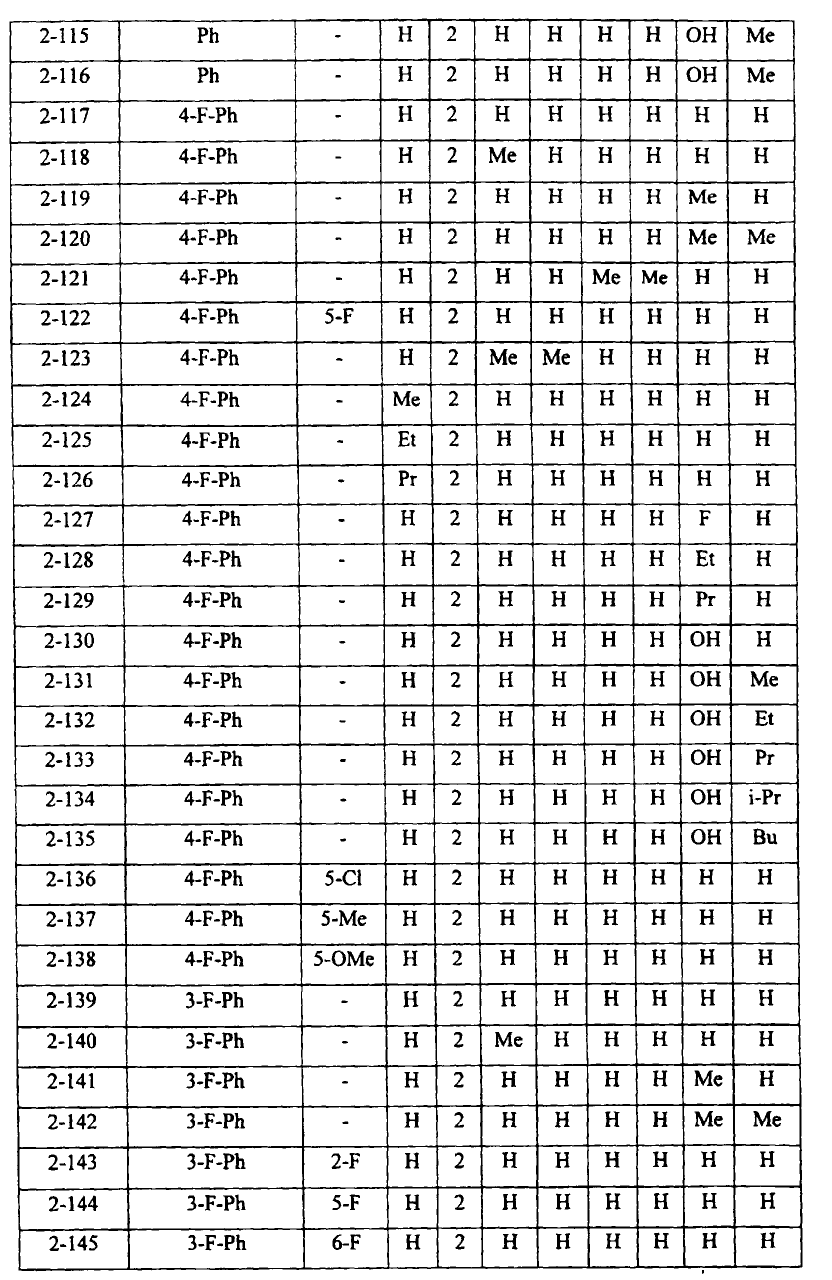

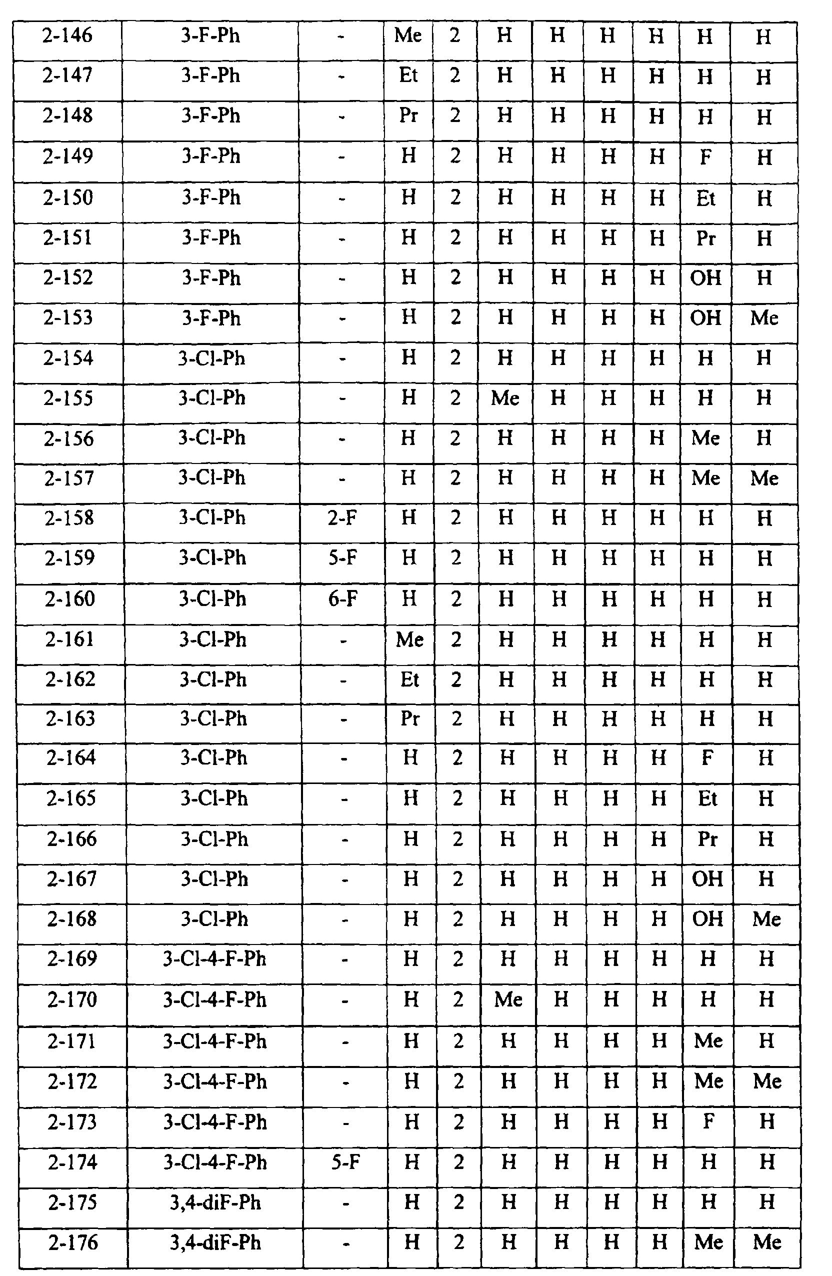

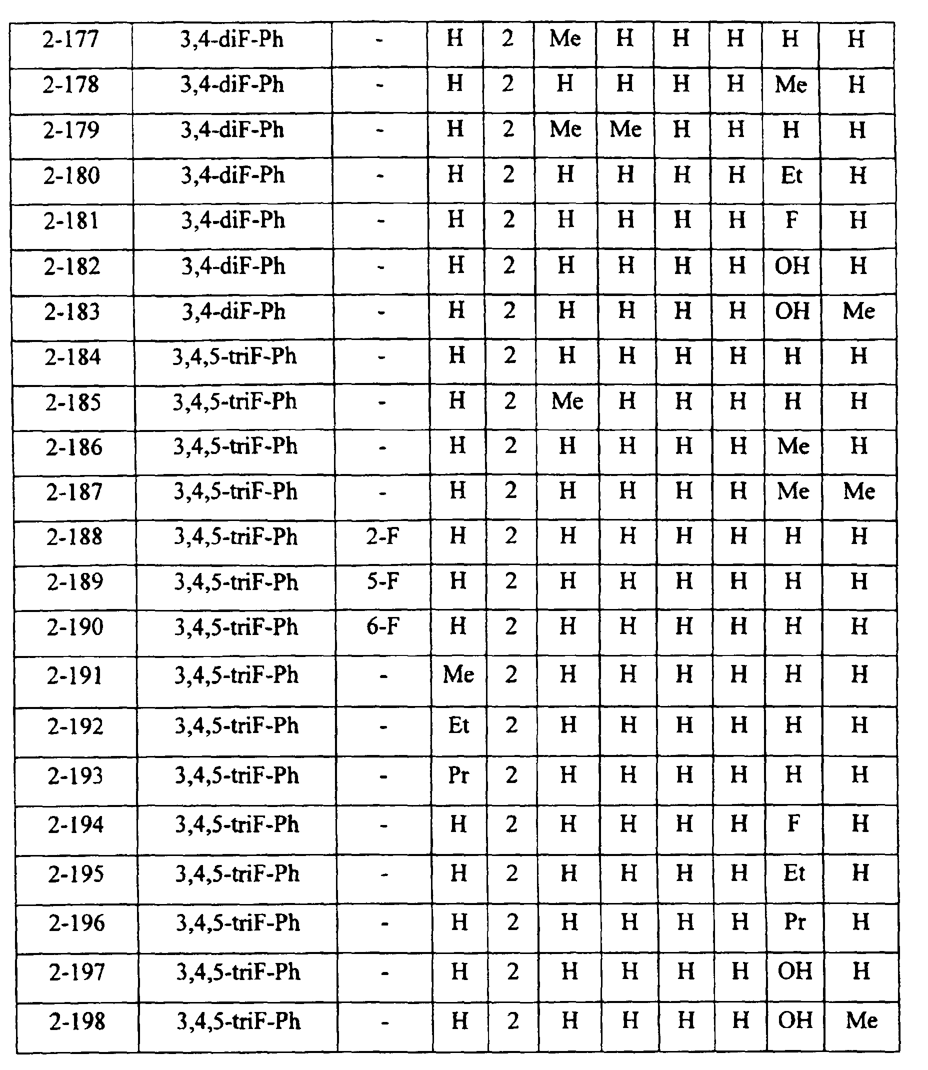

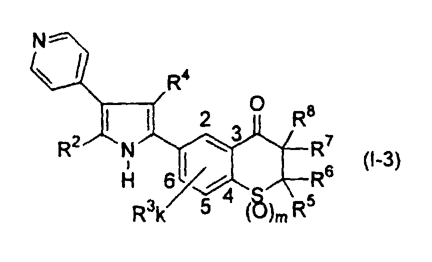

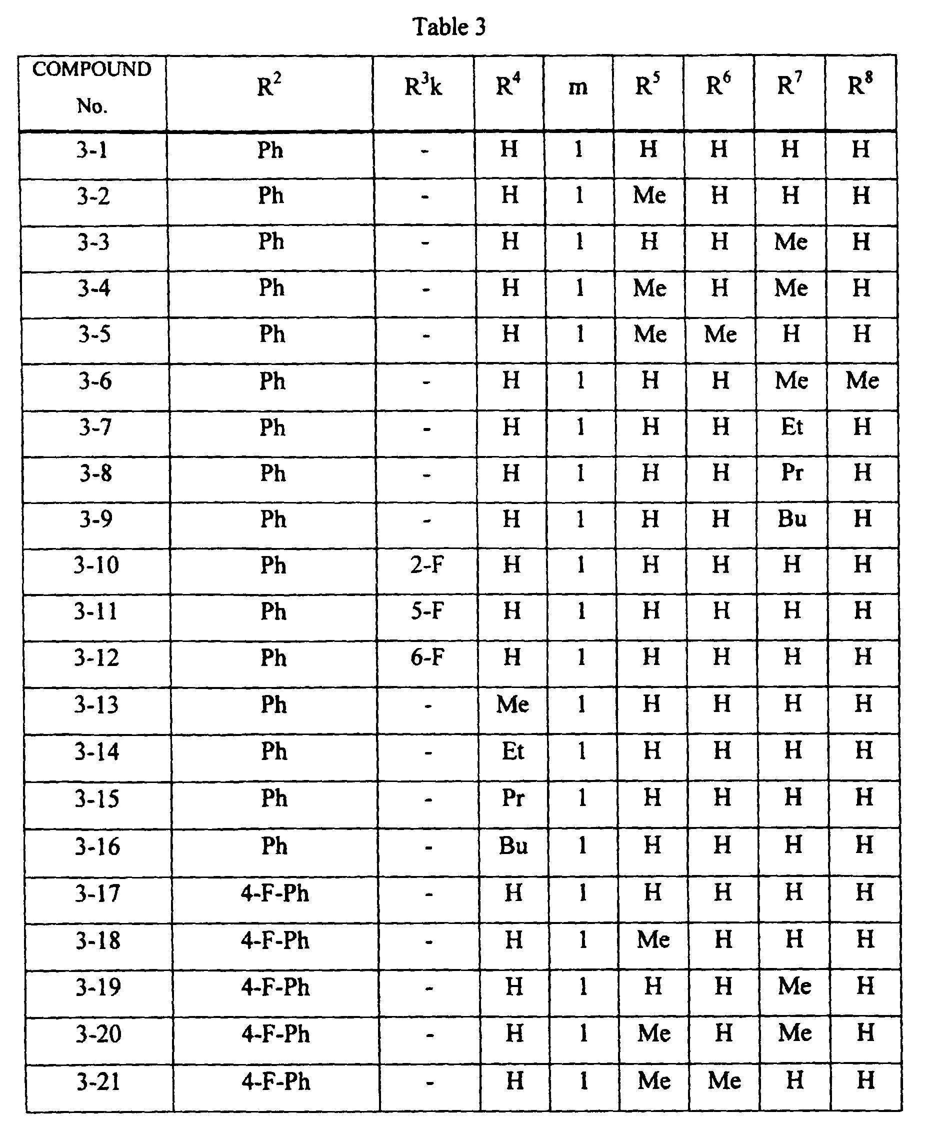

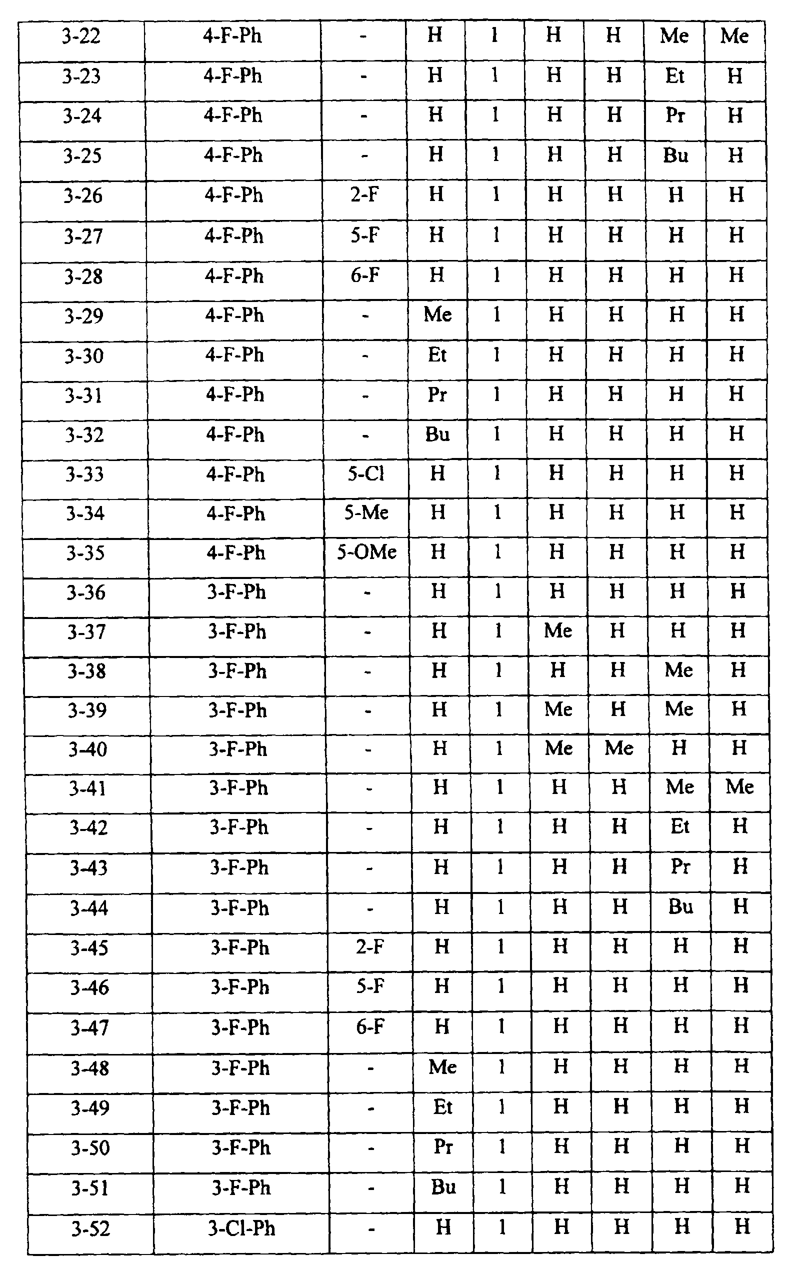

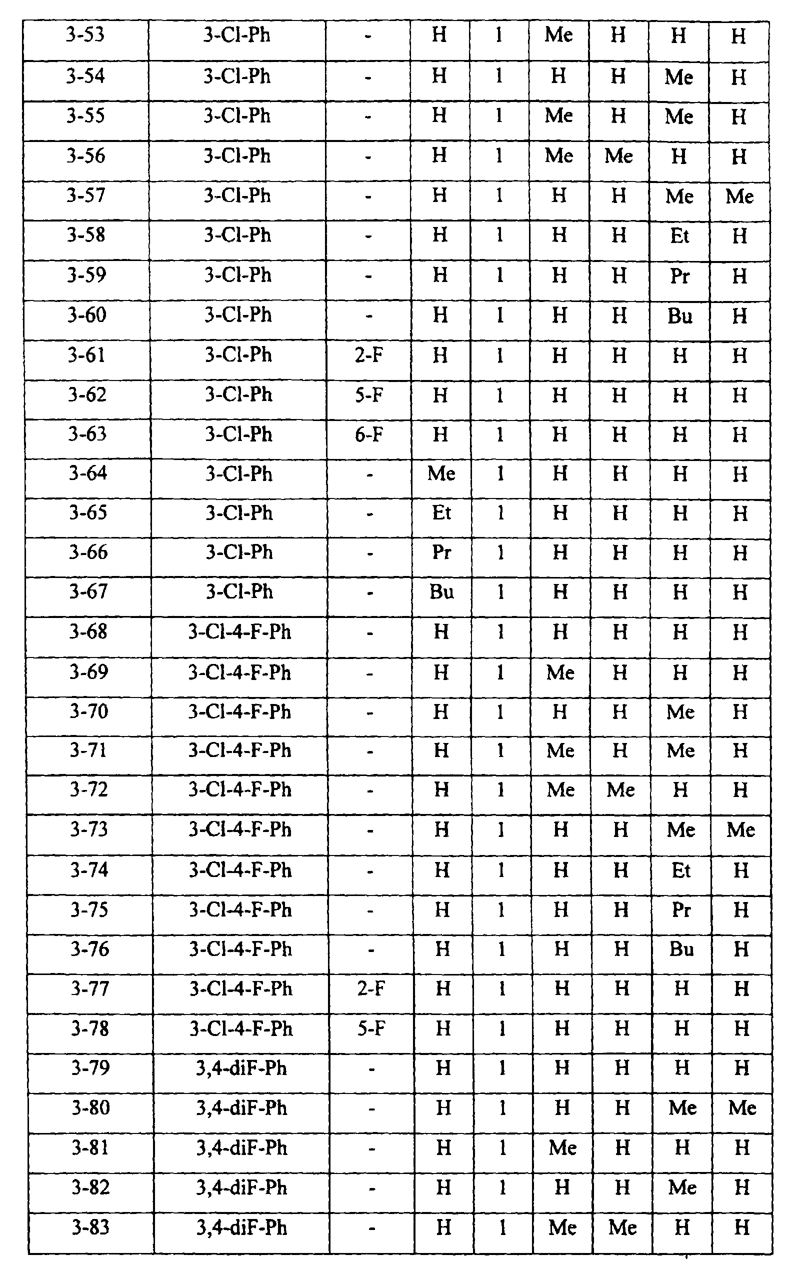

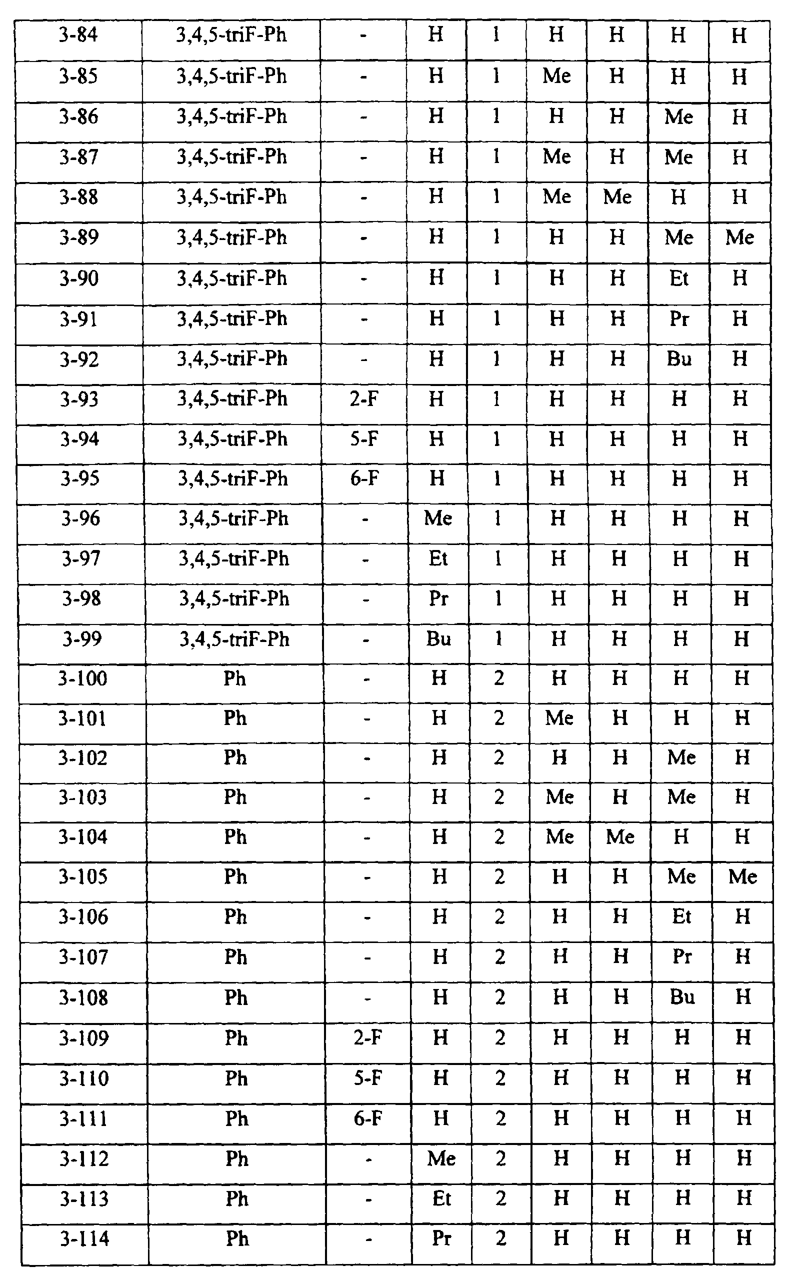

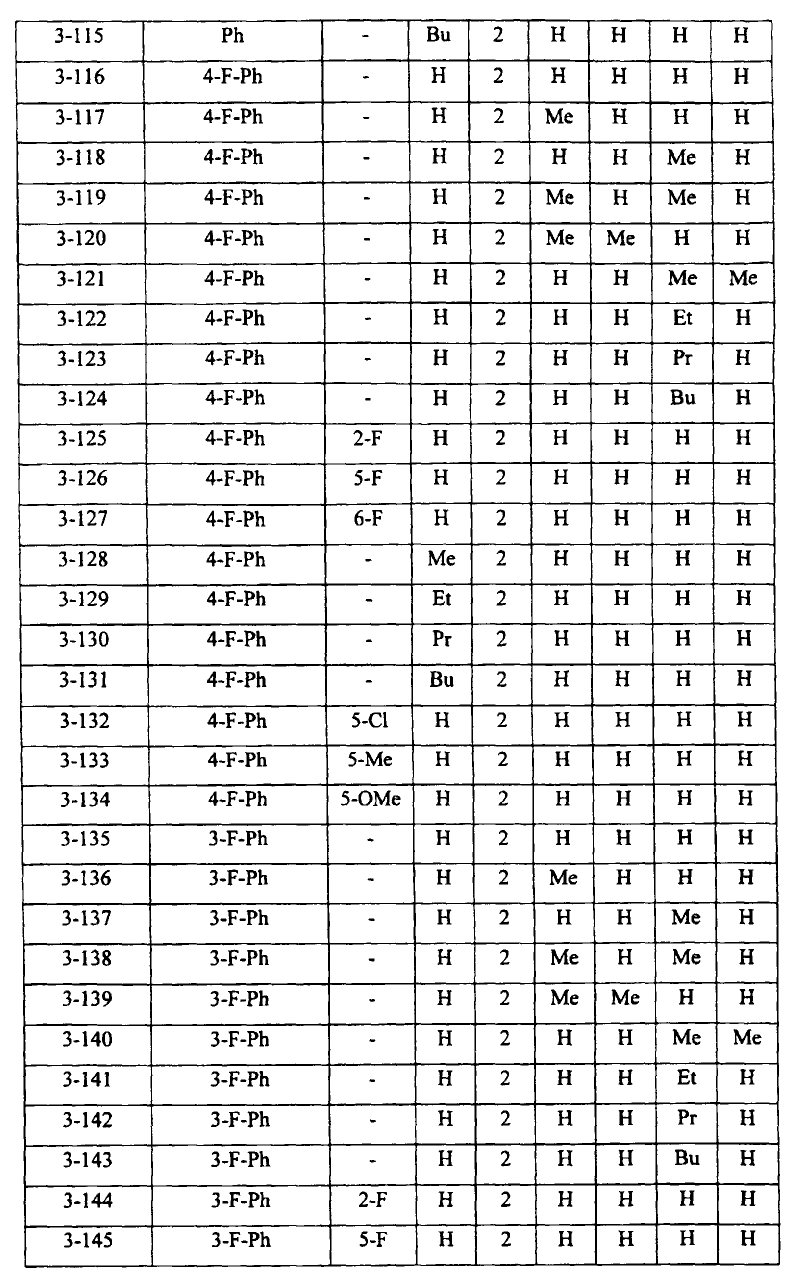

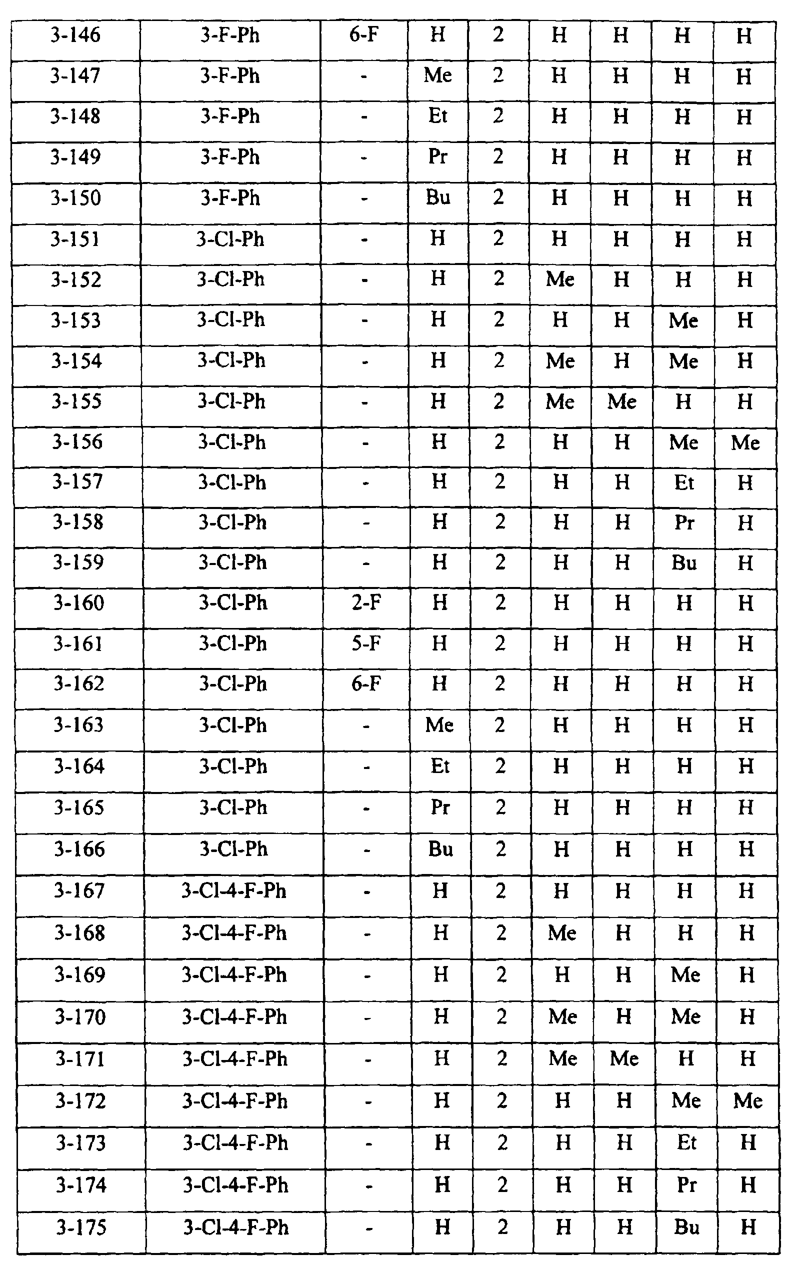

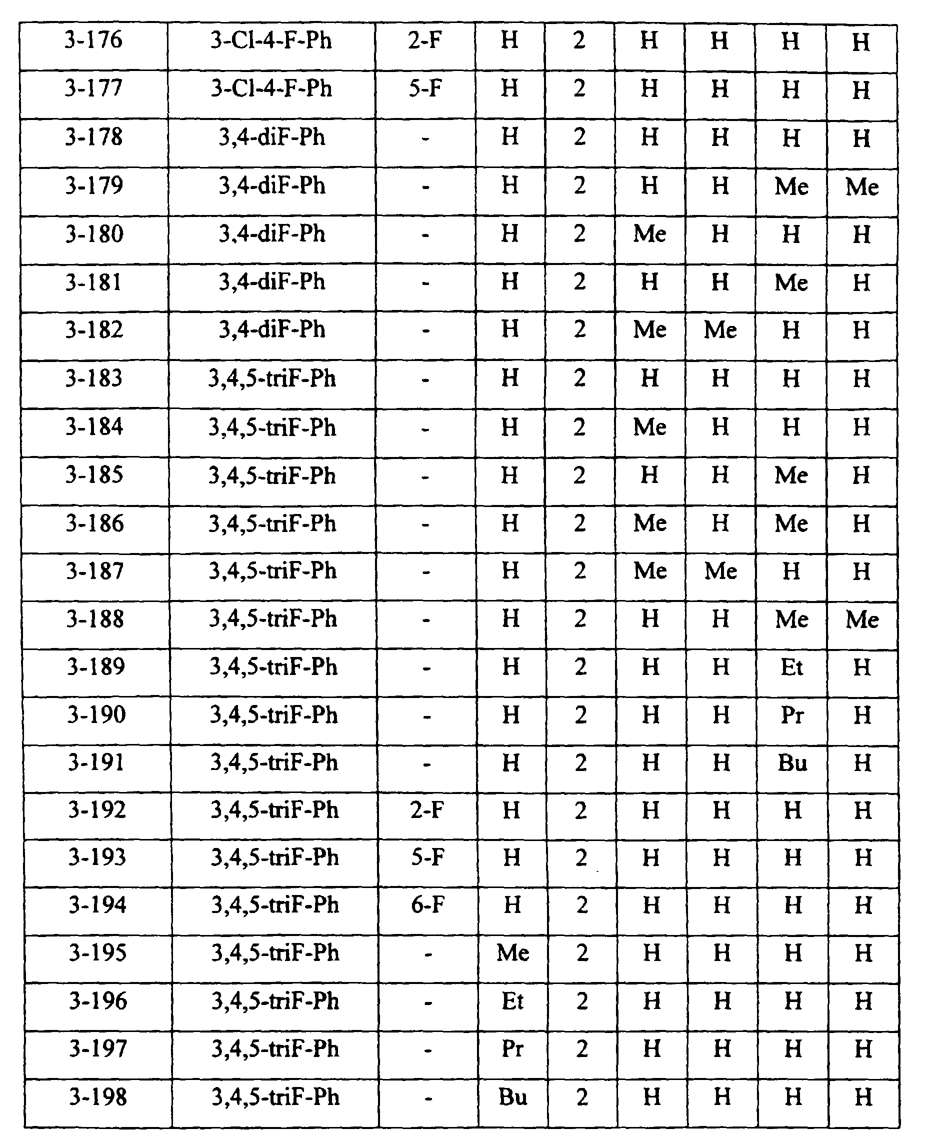

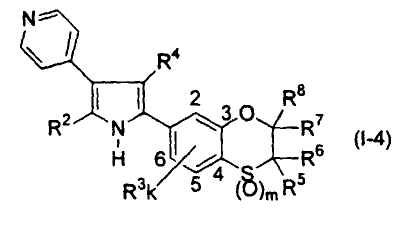

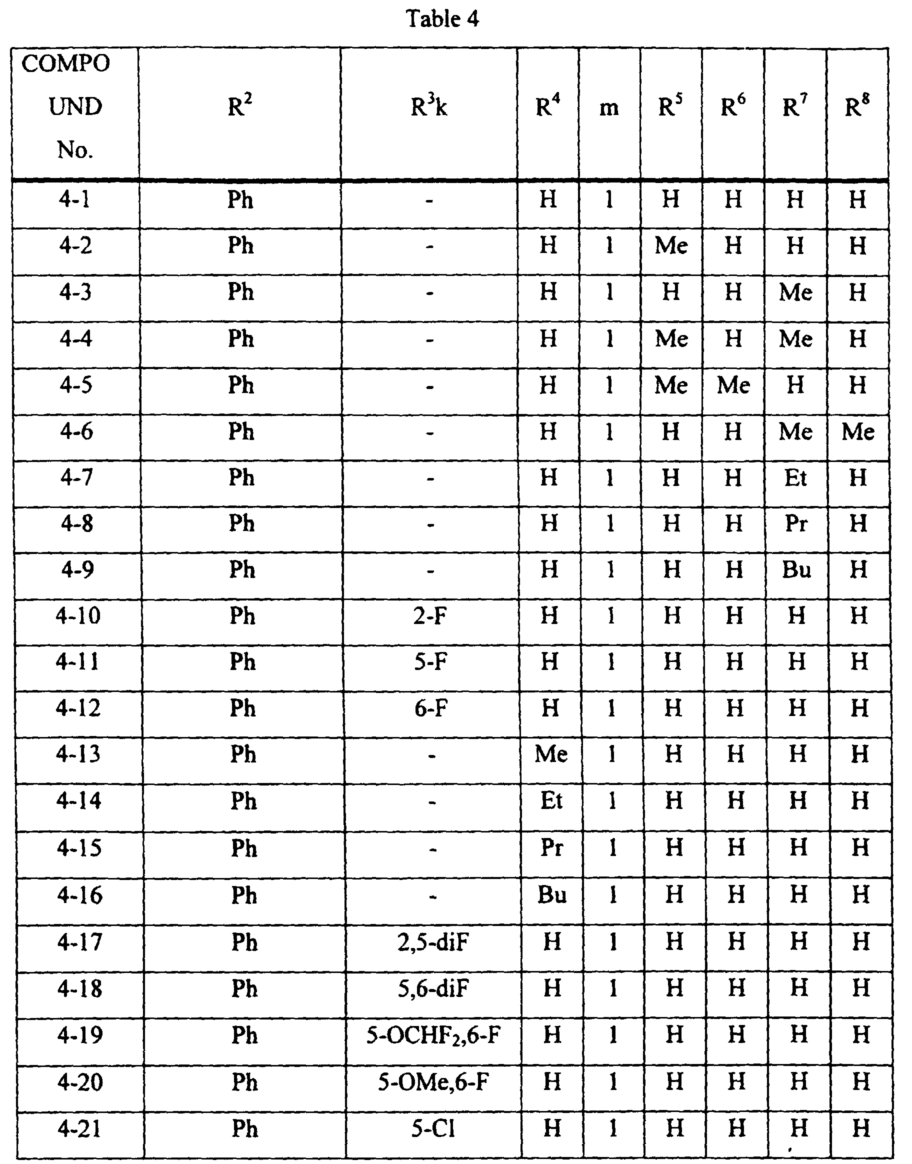

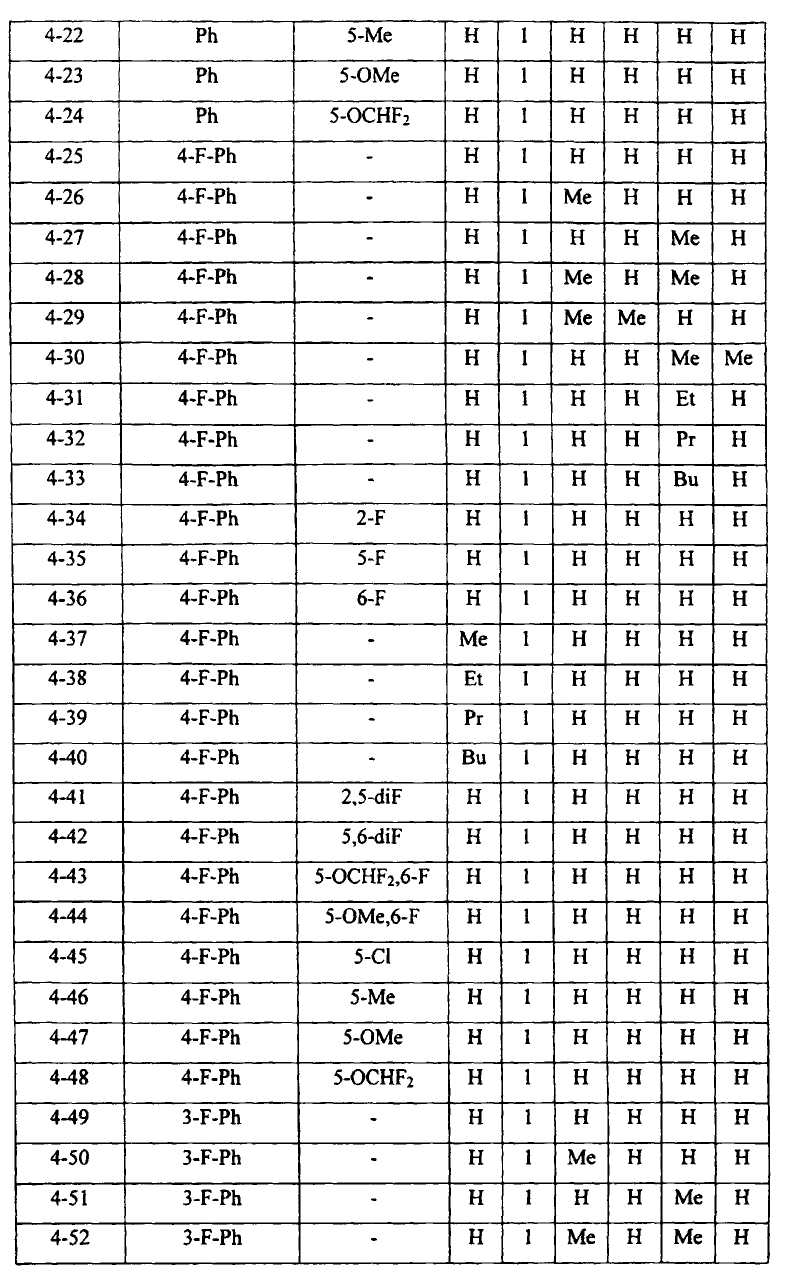

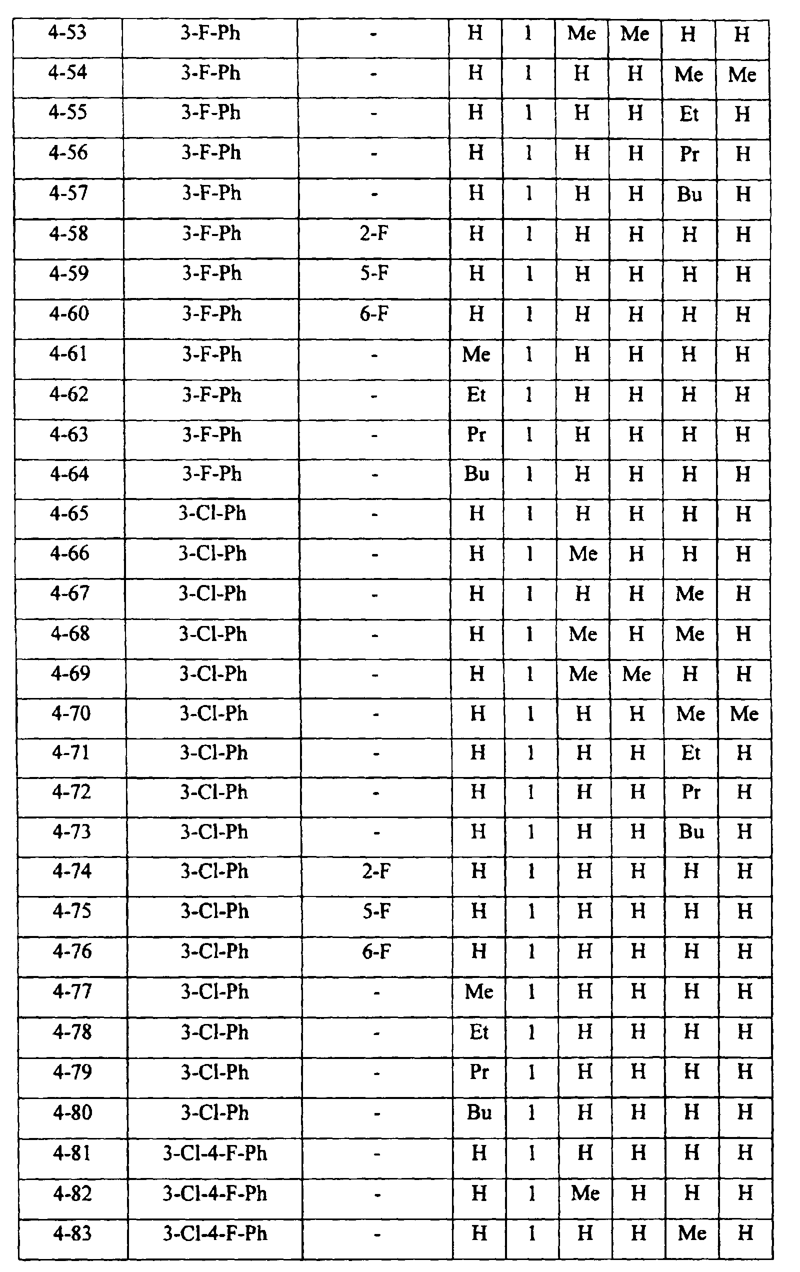

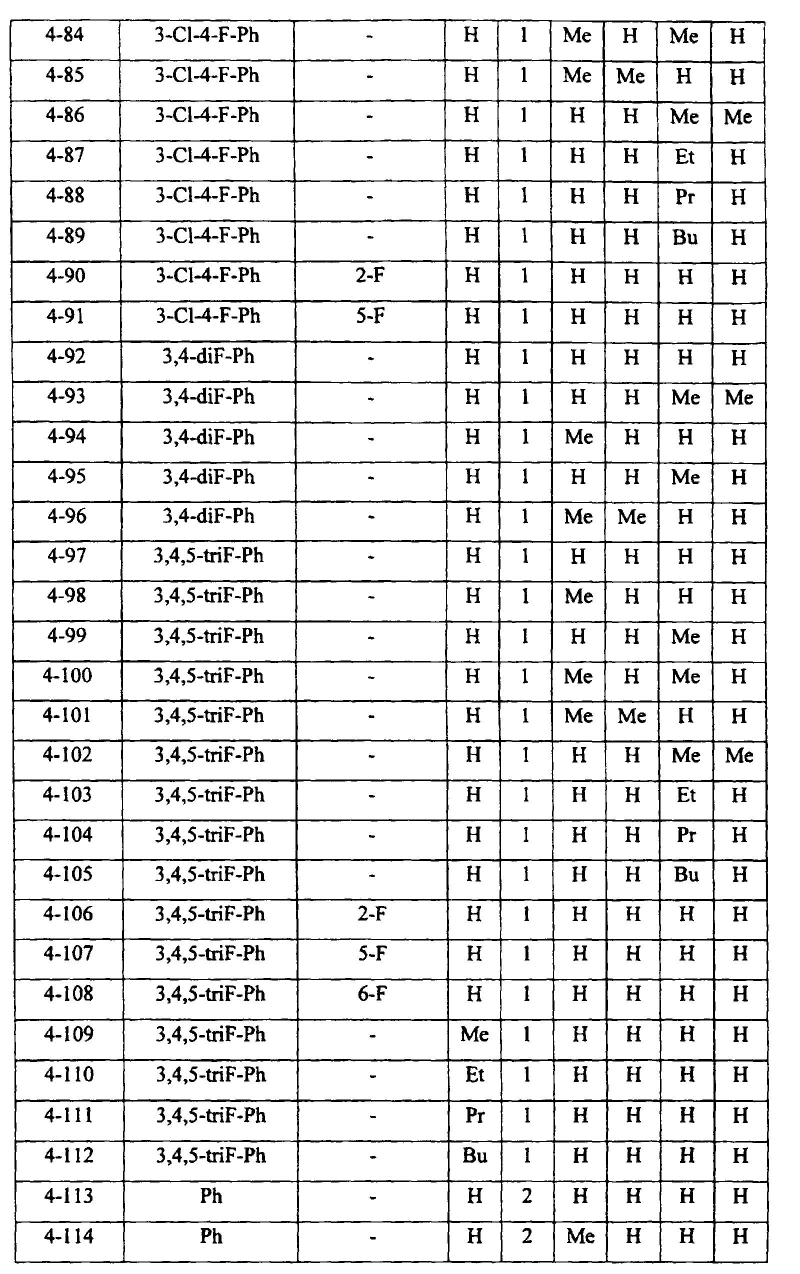

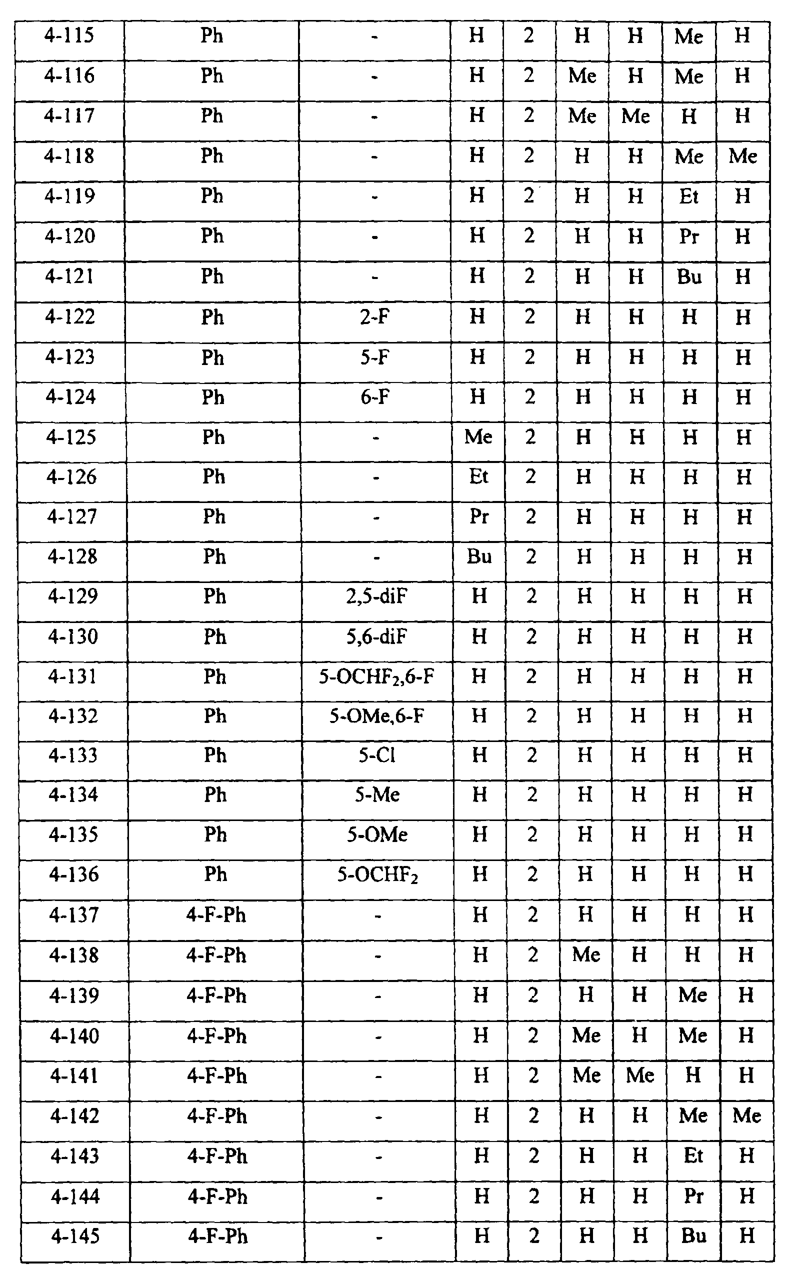

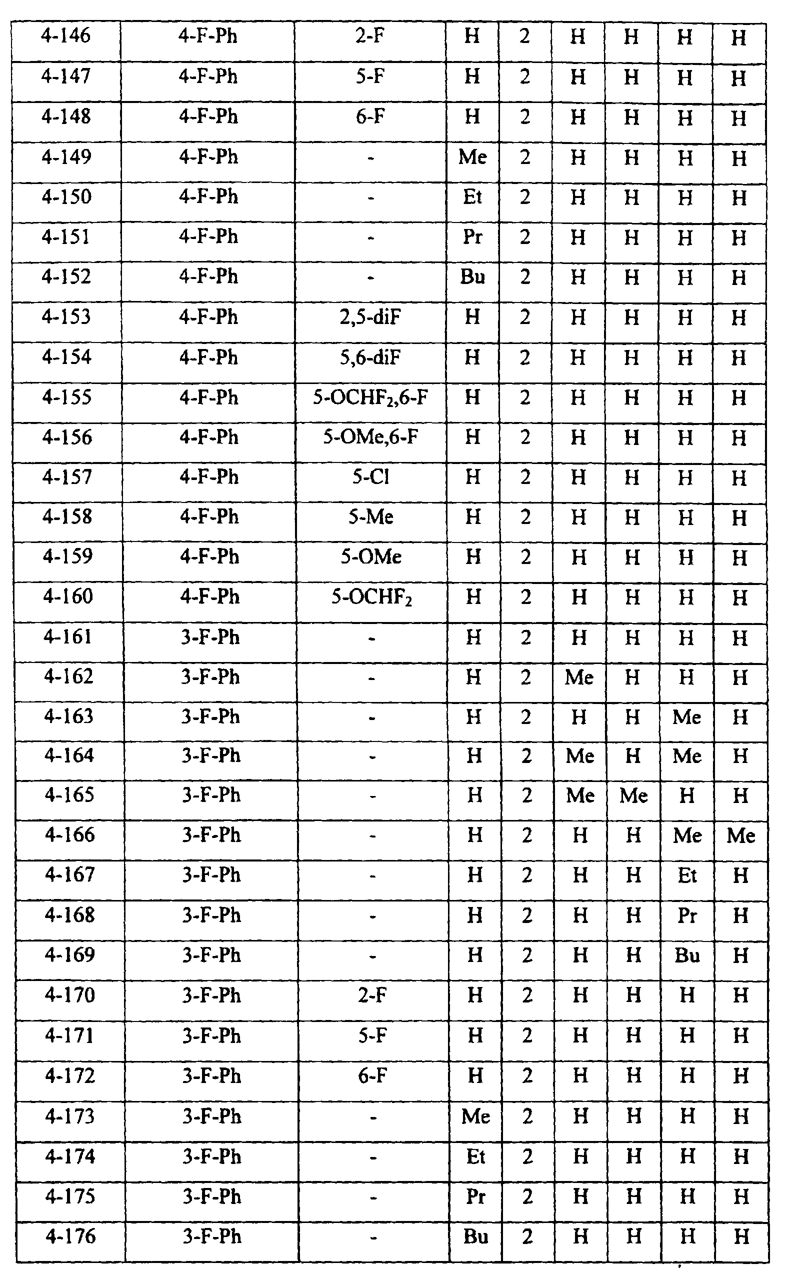

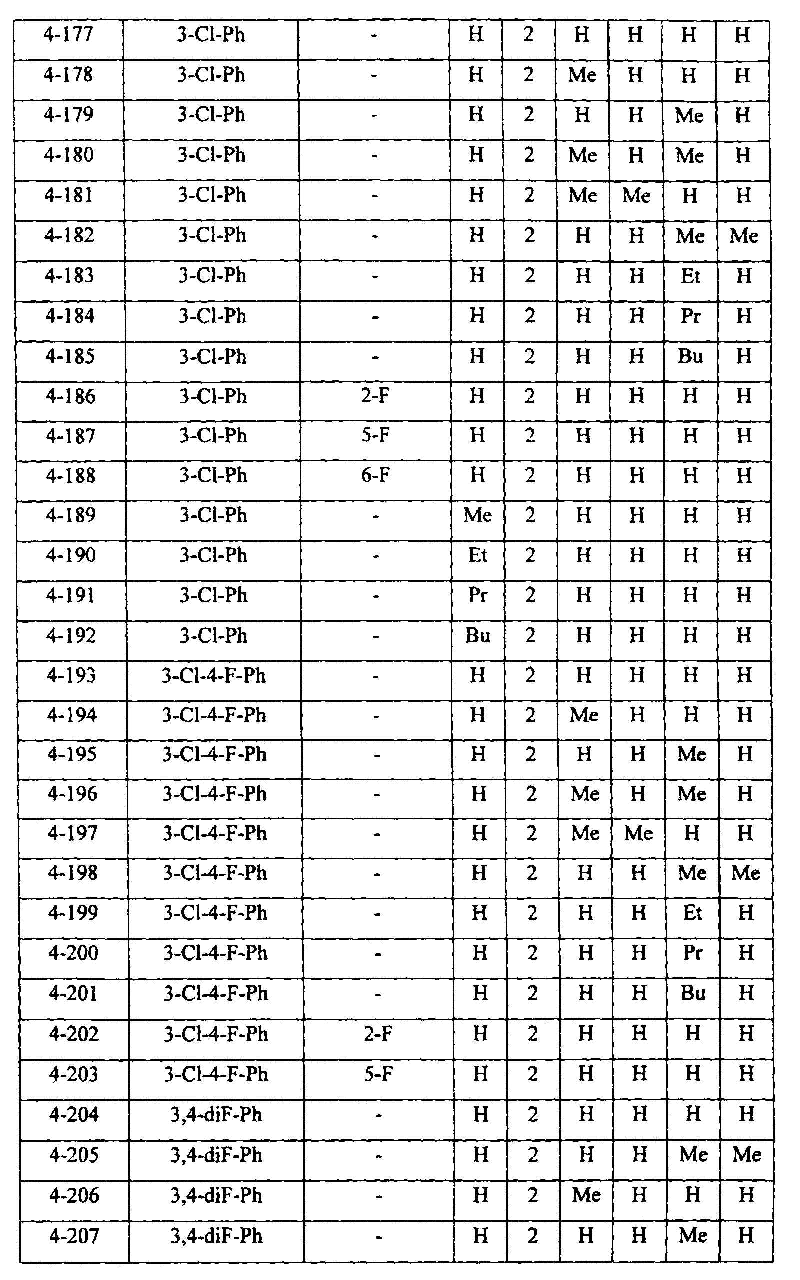

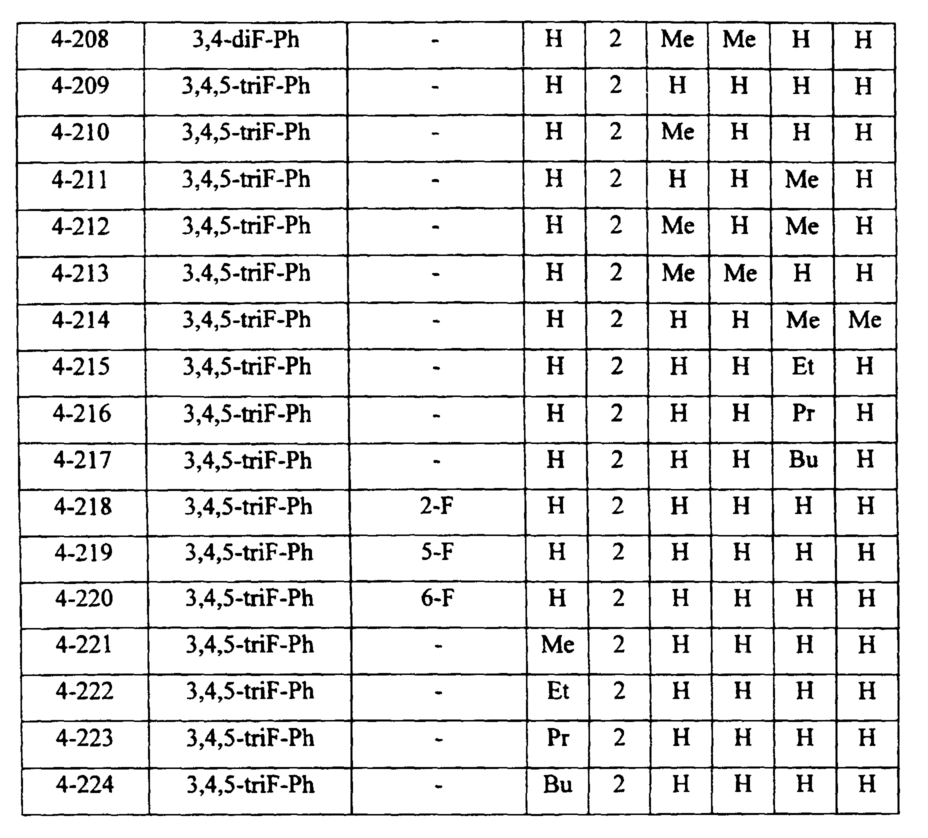

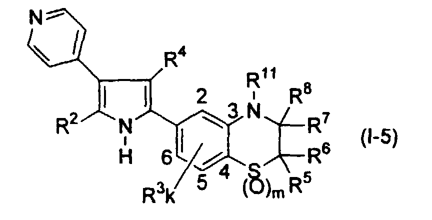

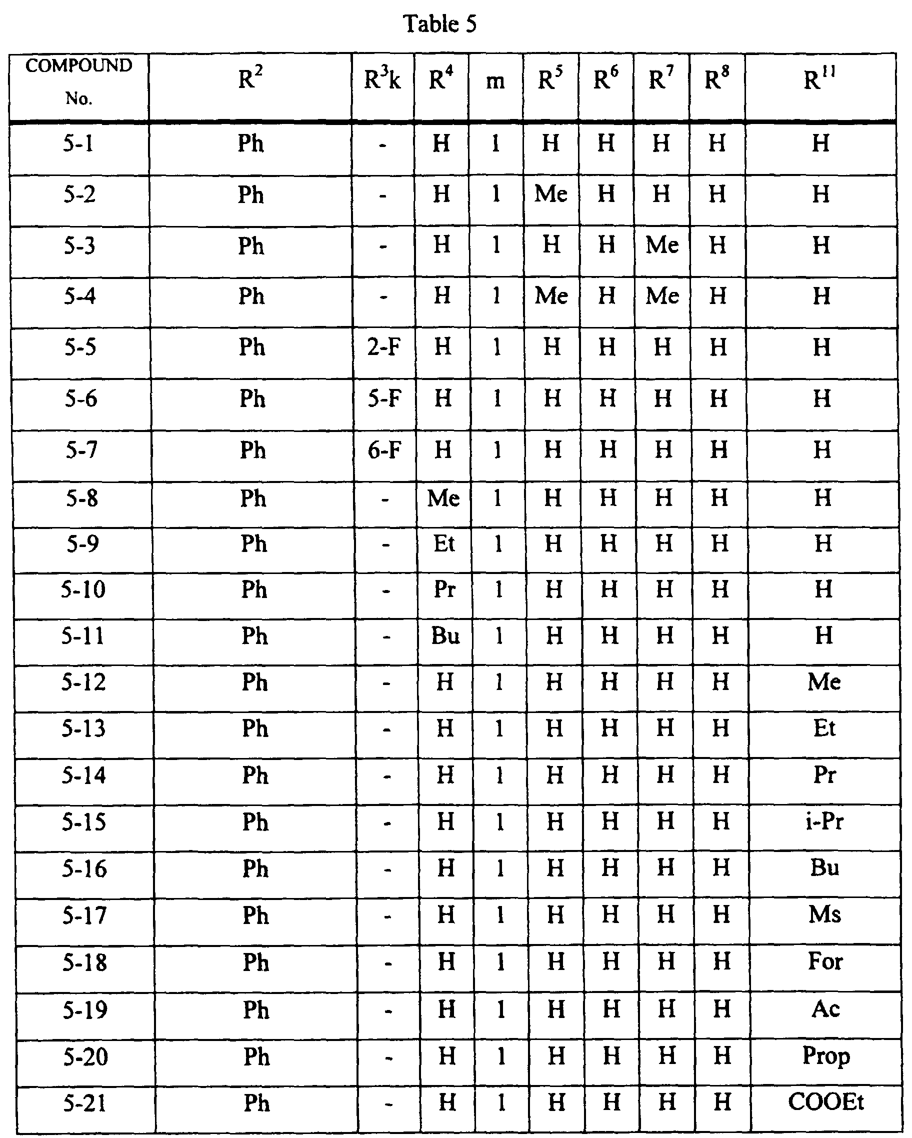

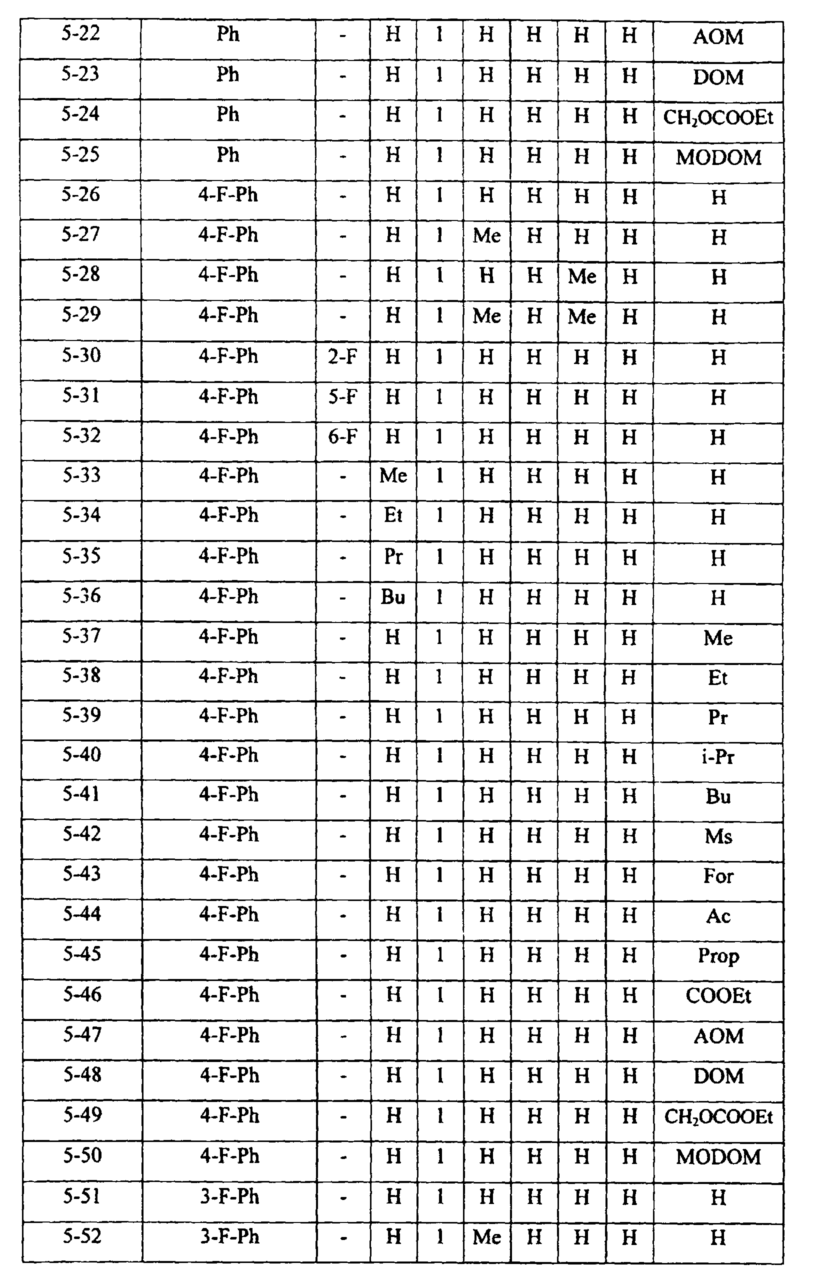

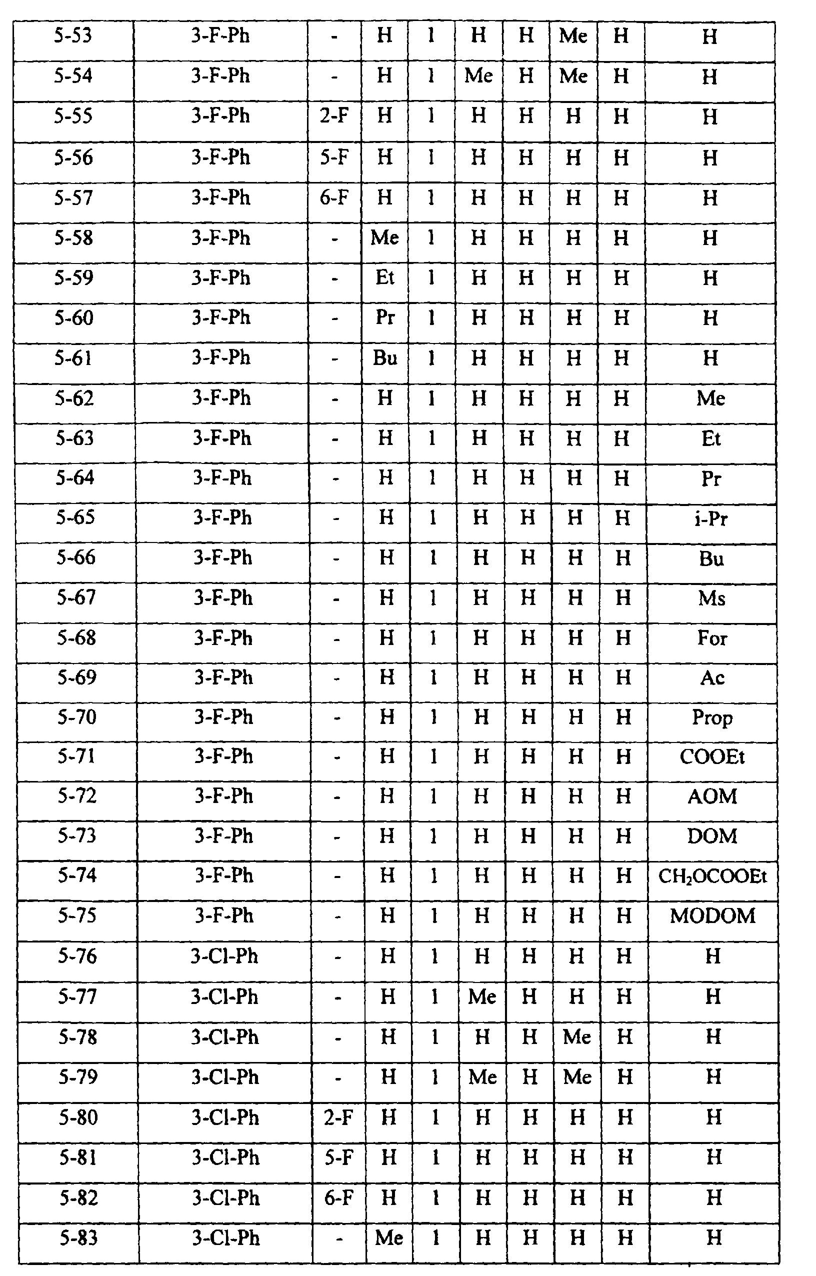

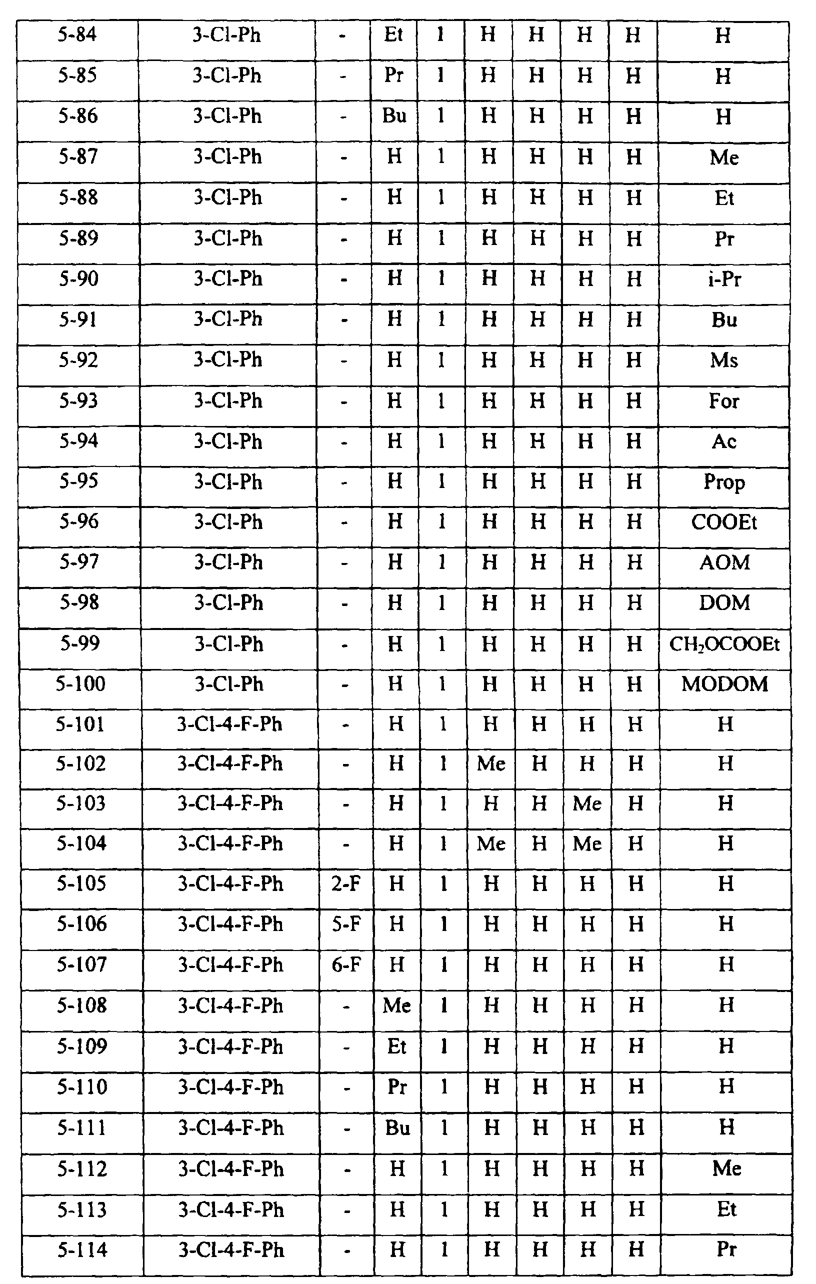

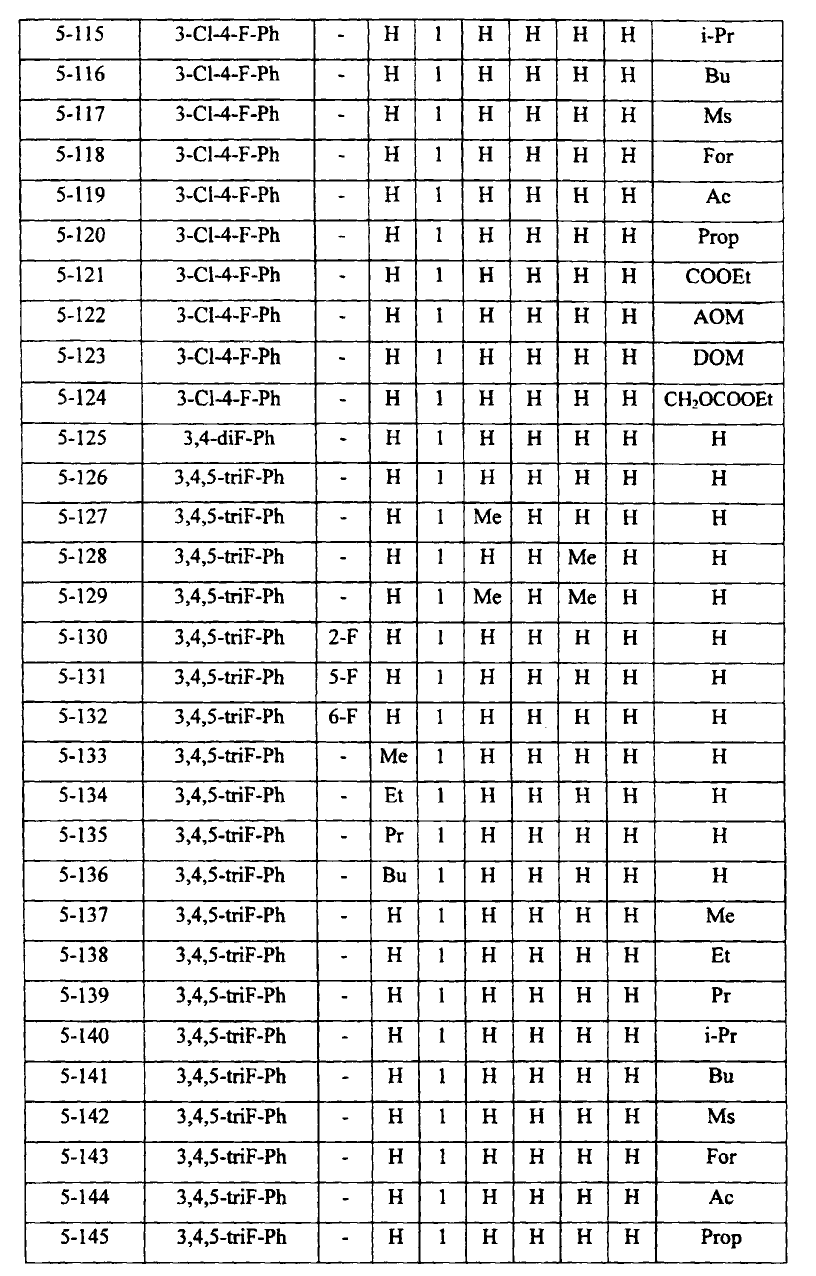

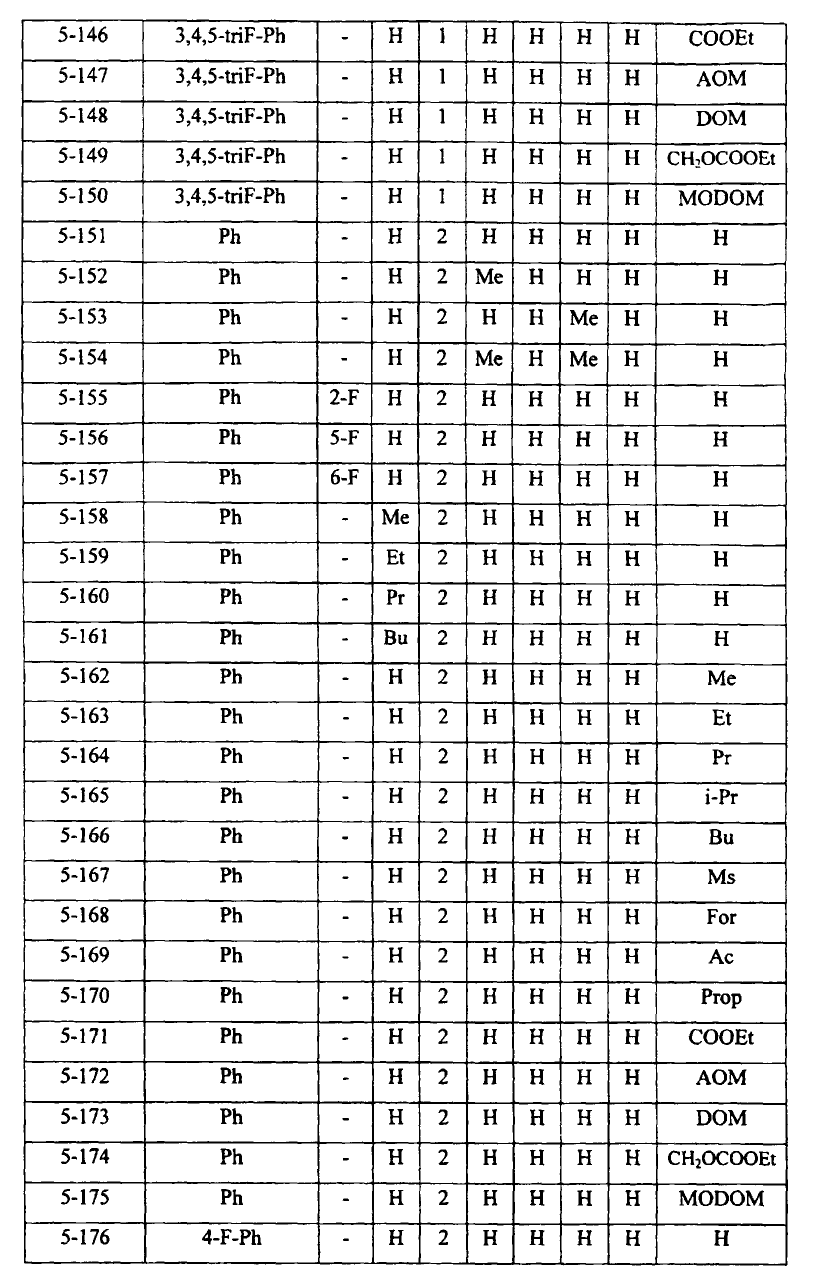

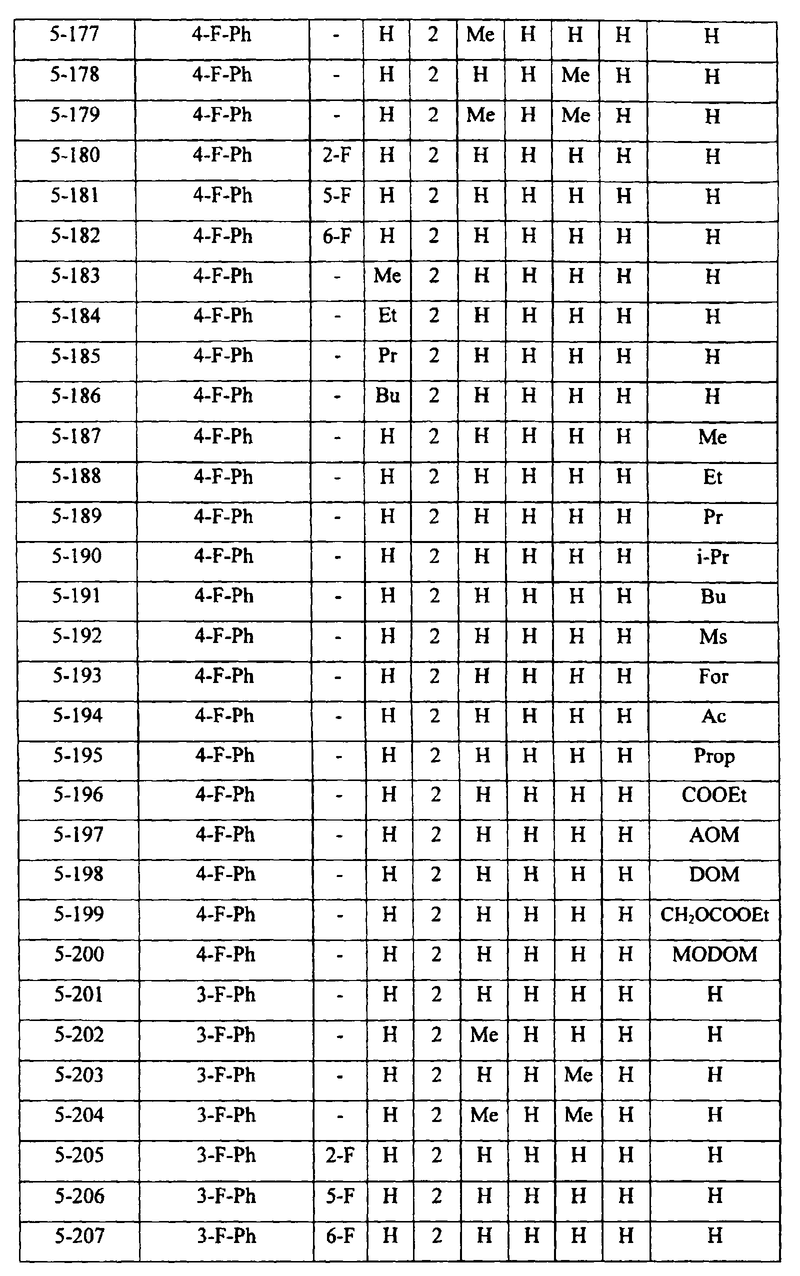

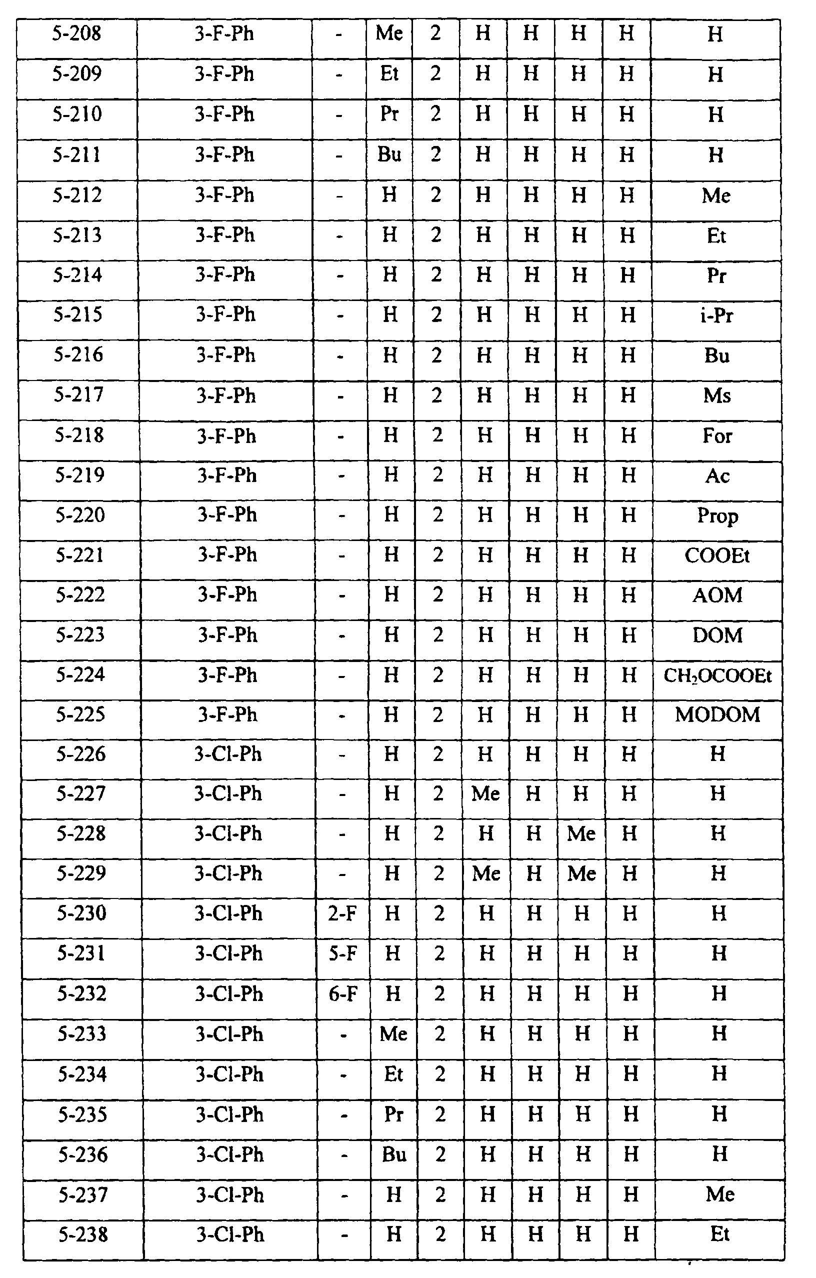

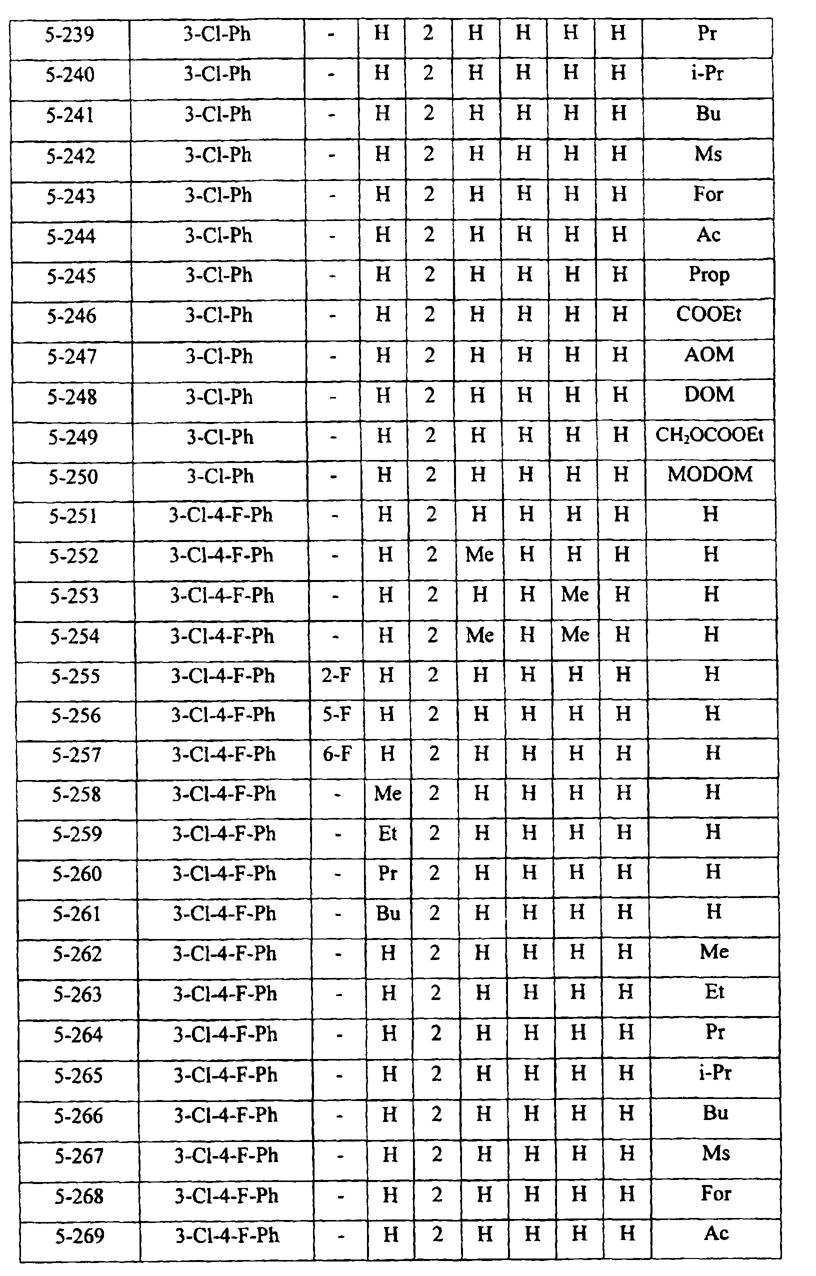

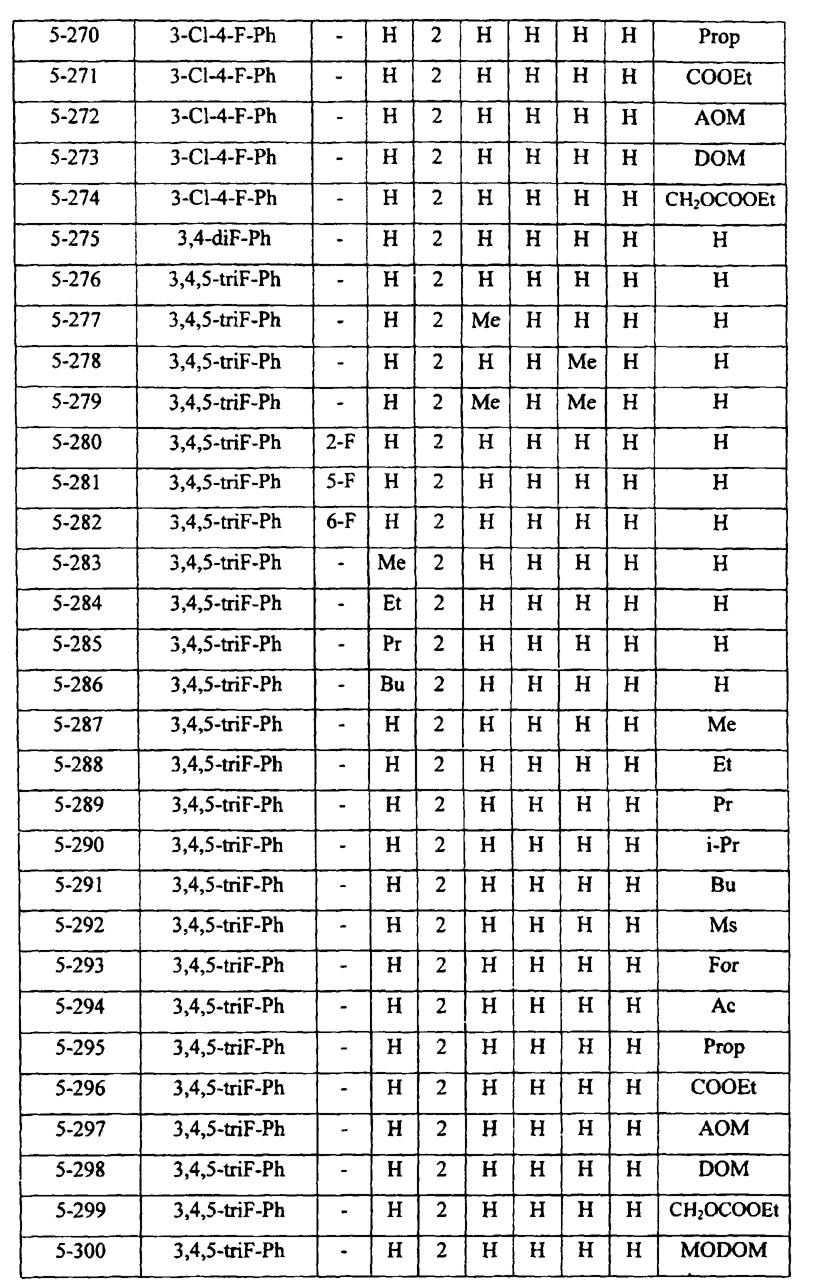

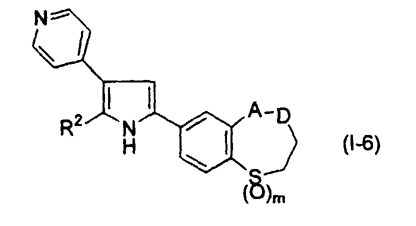

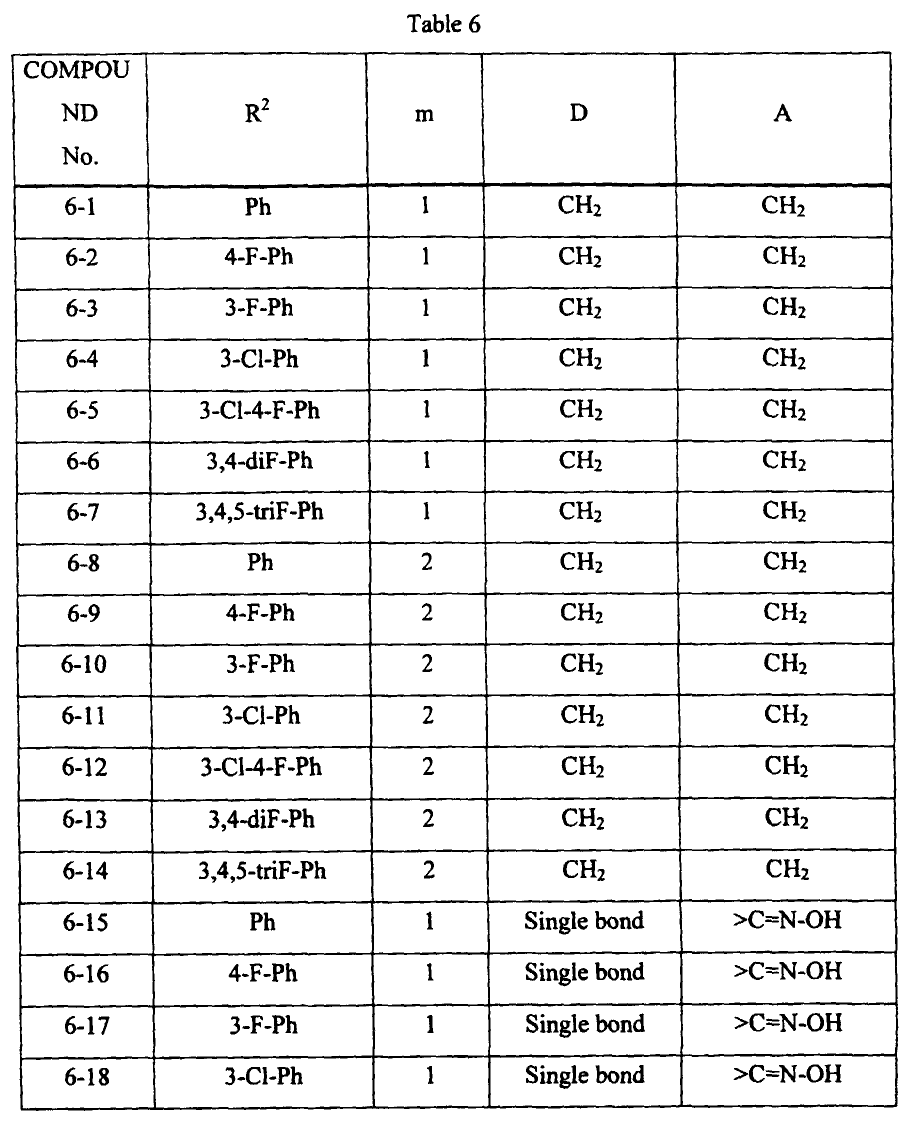

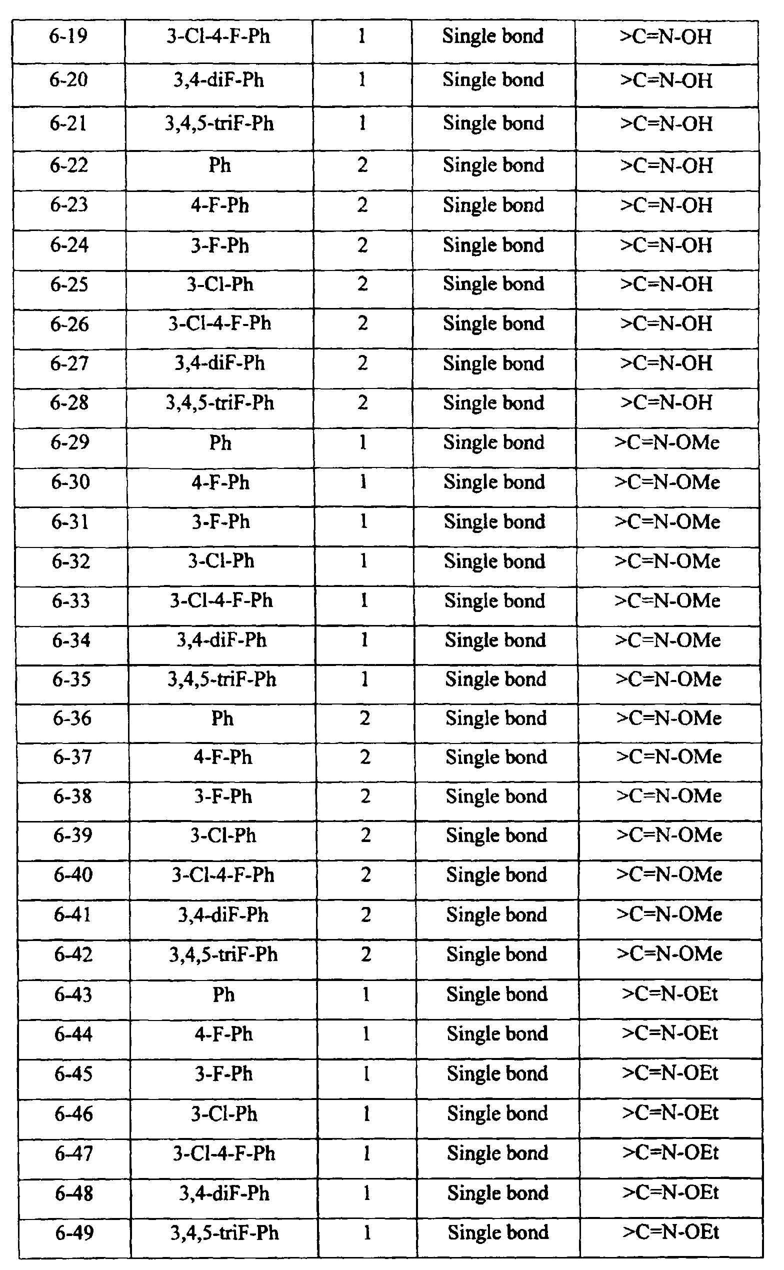

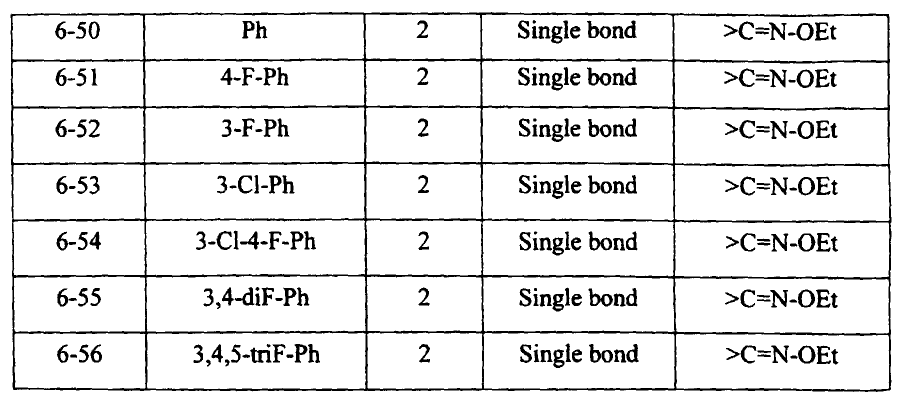

The following abbreviations are used in the tables. Ac : acetyl, AOM : acetoxymethyl, Bu : butyl, Et : ethyl For: formyl, Me : methyl, Ms : methylsulfonyl, Ph : phenyl MODOM : (5-methyl-2-oxo-1,3-dioxolene-4-yl)methyl POM : pivaloyloxymethyl, Pr : propyl, i-Pr : isopropyl Prop : propionyl

In column of R³k, "-" represents that k is 0.
In the tables preferred compounds are 1-1,1-6,1-17 to 1-19,1-22,1-37,1-42,1-53,1-58,1-69,1-74,1-80,1-81,1-85,1-90,1-101,1-106,1-117 to 1-119,1-122,1-137,1-142,1-153,1-158,1-169,1-174,1-180,1-181,1-185,1-190,2-1,2-4,2-14,2-20 to 2-24,2-26,2-30,2-33,2-42,2-45,2-55,2-57,2-60,2-70,2-72,2-75,2-78,2-85,2-87,2-90,2-100,2-102,2-105,2-115,2-117 to 2-121,2-123,2-130,2-139,2-142,2-152,2-154,2-157,2-167,2-169,2-172,2-175,2-176,2-182,2-184,2-187,2-197,3-1,3-17,3-36,3-52,3-68,3-79,3-84,3-100,3-116,3-135,3-151,3-167,3-183,4-1,4-25,4-49,4-65,4-81,4-90,4-92,4-97,4-113,4-137,4-161,4-177,4-193,4-204,4-209,5-1,5-12,5-26,5-37,5-42 to 5-51,5-62,5-76,5-87,5-101,5-112,5-125,5-126,5-137,5-151,5-162,5-176,5-187,5-192 to 5-201,5-212,5-226,5-237,5-251,5-262,5-275,5-276,5-287 and 6-1 to 6-14;
More preferred compounds are ,1-1,1-17,1-22,1-37,1-53,1-69,1-80,1-85,1-101,1-117,1-122,1-137,1-153,1-169,1-180,1-185,2-1,2-4,2-20,2-21,2-23,2-24,2-26,2-30,2-33,2-42,2-45,2-57,2-60,2-72,2-75,2-78,2-87,2-90,2-102,2-105,2-117,2-118,2-120,2-120,2-121,2-123,2-139,2-142,2-154,2-157,2-169,2-172,2-175,2-184,2-187,3-1,3-17,3-36,3-52,3-68,3-79,3-84,3-100,3-116,3-135,3-151,3-167,3-183,4-25,4-137,5-26,5-37,5-44,5-176,5-187,6-2,6-9,6-16 and 6-30;
The most preferred compounds are 1-1,1-17,1-37,1-53,1-69,1-80,1-85,1-101,1-117,1-137,1-153,1-169,1-180,1-185,2-1,2-20,2-21,2-23,2-24,2-26,2-30,2-42,2-57,2-72,2-78,2-87,2-102,2-117,2-118,2-120,2-121,2-123,2-139,2-154,2-169,2-175,2-184,3-17,3-116,6-2,6-9,6-16 and 6-30.

### [Mode for carrying out the Invention]

The compounds of formula (I) according to the present invention can be prepared by any one of the below-described methods A to G.

### [Method A: preparation of the compound (Ia) wherein R⁴ is a hydrogen atom]

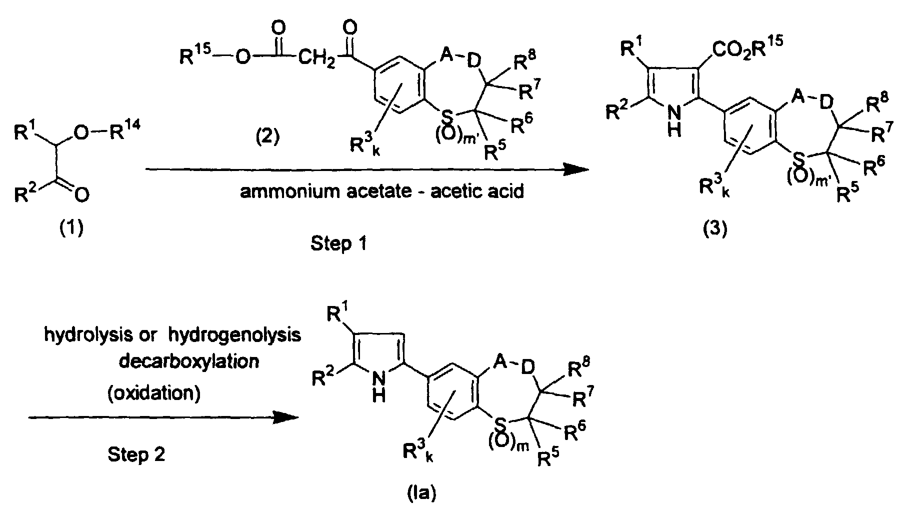

(wherein, A, D, R¹, R², R³, R⁵, R⁶, R⁷, R⁸, k and m have the same meanings as described above,
R¹⁴ represents a hydrogen atom or the above-described "silyl group",
R¹⁵ represents the above-described "lower alkyl group" or the above-described "aralkyl" group, and
m' is 0, 1 or 2).

Step 1 is a condensation of a ketoalcohol compound (1) with a benzoylacetate compound (2) in acetic acid in the presence of ammonium acetate, to give the corresponding pyrrolecarboxylester compound (3). This step is effected in accordance with the process described in the literature (D. Davidson, J. Org. Chem., 3, 361(1938)).

Step 2 is hydrolysis or hydrogenolysis of the pyrrolecarboxyl ester compound (3), followed by decarboxylation. When the pyrrolecarboxyl ester compound (3) wherein m' is 0, is used as a starting material, the sulfide group is oxidized, to give the compound (Ia) which is a compound (I) wherein R⁴ is a hydrogen.

Hydrolysis using an acid or alkali or hydrogenolysis employed ordinarily in organic synthetic chemistry is carried out in the former stage of the reaction and the subsequent decarboxylation is carried out using an acid or alkali or heating.

When the compound wherein m' stands for 0 is employed as a starting material for this step, a cyclic sulfide compound (Ia') is prepared by the above-described hydrolysis or hydrogenolysis and decarboxylation. The compound (Ia) can be prepared by oxidation of this sulfide group.

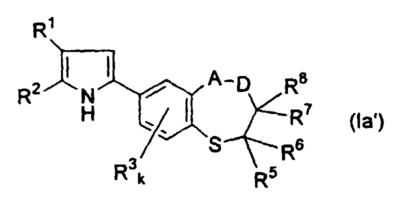

The oxidation of cyclic sulfide compound (Ia') is effected by an oxidizing agent (examples include peracids such as peracetic acid, perbenzoic acid and m-chloroperbenzoic acid; hydrogen peroxide; and alkali metal perhalogenate salts such as sodium metaperchlorate, sodium metaperiodate and potassium metaperiodate, of which the peracids and hydrogen peroxide, particularly, m-chloroperbenzoic acid are preferred) at -20°C to 150°C (preferably at 0 to 100°C) for 10 minutes to 10 hours (preferably for 30 minutes to 5 hours) in an inert solvent (examples include aliphatic hydrocarbons such as hexane, heptane and petroleum ether; aromatic hydrocarbons such as benzene, toluene and xylene; halogenated hydrocarbons such as methylene chloride, chloroform, carbon tetrachloride and dichloroethane; alcohols such as methanol, ethanol, propanol and butanol; esters such as ethyl acetate, propyl acetate, butyl acetate and ethyl propionate; carboxylic acids such as acetic acid and propionic acid; and water, and a mixed solvent thereof, of which the halogenated hydrocarbons (more preferably, methylene chloride, chloroform, dichloroethane and carboxylic acids, particularly acetic acid) are preferred).

When the compound (Ia) wherein m is 1 is prepared, the oxidizing agent is added in an amount of 0.6 to 1.4 equivalents (preferably, 0.8 to 1.2 equivalents), while when the compound (Ia) wherein m is 2 is prepared, the oxidizing agent is added in an amount of 1.5 to 3 equivalents (preferably, 1.8 to 2.5 equivalents).

### [Method B: preparation of compound (Ib), which is the compound (Ia) wherein A is an oxygen atom or -N(R¹¹)-]

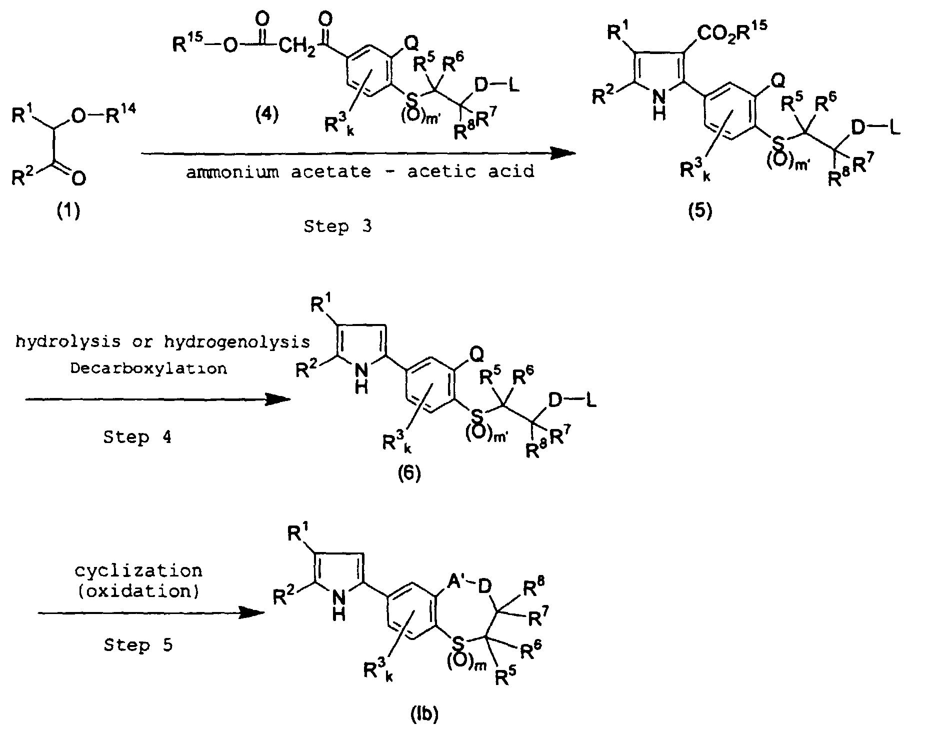

(wherein, D, R¹, R², R³, R⁵, R⁶, R⁷, R⁸, R¹¹, R¹², R¹³, k, m and m' have the same meanings as described above,
A' represents an oxygen atom or -N(R¹¹)-,
when A' represents an oxygen atom, Q represents a leaving group and L represents a hydroxyl group and
when A' represents -N(R¹¹)-, Q represents -NHR¹¹ and L represents a leaving group).

The "leaving group" in the definition of Q and L means a group, which usually will leave as a nucleophilic residue. Examples include halogen atoms such as fluorine, chlorine, bromine and iodine; trihalogenomethyloxy groups such as trichloromethyloxy; lower alkanesulfonyloxy groups such as methanesulfonyloxy and ethanesulfonyloxy; lower halogeno alkane sulfonyloxy groups such as trifluoromethanesulfonyloxy and pentafluoroethanesulfonyloxy, and arylsulfonyloxy groups such as benzenesulfonyloxy, p-toluenesulfonyloxy and p-nitrobenzenesulfonyloxy, of which halogen atoms are preferred.

Step 3 is condensation of a ketoalcohol compound (1) with a benzoylacetate compound (4) in acetic acid in the presence of ammonium acetate to prepare the corresponding pyrrolecarboxyl ester compound (5). This step is conducted in a similar manner to that described in the step 1.

Step 4 is hydrolyis or hydrogenolysis of the pyrrolecarboxyl ester compound (5), followed by decarboxylation to prepare the compound (6). This step is carried out in a similar manner to that described in the step 2.

Step 5 is cyclization of compound (6). When m' is 0, the sulfide group is oxidized, to give compound (Ib) of the present invention.
a) When the compound (6) has a hydroxyl group as L, the cyclization reaction can be carried out in accordance with the Mitsunobu reaction (D.L. Hughes, Org. React., 42, 335(1992)).
   There is no particular limitation on the nature of the reagent to be used for the Mitsunobu reaction provided that it is ordinarily used in the Mitsunobu reaction. Preferred are combinations of an azo compound, for example, a di(lower alkyl) azodicarboxylate such as diethyl azodicarboxylate or diisopropyl azodicarboxylate, or an azodicarbonyl compound such as 1,1'-(azodicarbonyl)dipiperidine, with a phosphine, for example, a triarylphosphine such as triphenylphosphine, or tri(lower alkyl)phosphine such as tri(n-butyl)phosphine, of which the combination of a di(lower alkyl) azodicarboxylate with a triarylphosphine is preferred and the combination of diethyl azodicarboxylate with triphenylphosphine is most preferred.
   As the solvent used for the reaction, there is no particular limitation on the nature of the solvent provided that it has no adverse effect on the reaction and can dissolve the starting material therein to some extent. Preferred examples include aromatic hydrocarbons such as benzene, toluene and xylene; halogenated hydrocarbons such as methylene chloride, chloroform, carbon tetrachloride, dichloromethane chlorobenzene and dichlorobenzene; esters such as ethyl formate, ethyl acetate, propyl acetate, butyl acetate and diethyl carbonate; ethers such as diethyl ether, diisopropyl ether, tetrahydrofuran, dioxane, dimethoxyethane and diethylene glycol dimethyl ether; nitriles such as acetonitrile and isobutylonitrile; amides such as formamide, N,N-dimethylformamide, N,N-dimethylacetamide, N-methyl-2-pyrrolidone and hexamethylphosphorous triamide; and sulfoxides such as dimethylsulfoxide and sulfolane, of which the aromatic hydrocarbons and ethers are preferred.
   The reaction temperature ranges from -20°C to 100°C, preferably 0°C to 50°C.
   Although the reaction time varies depending on the reaction temperature, starting material compounds, reaction reagent and nature of the solvent to be employed, it usually ranges from 10 minutes to 3 days, preferably from 30 minutes to 12 hours.
b) When the compound (6) has a leaving group as L, cyclization reaction is effected in a solvent in the presence or absence of a base.

Examples of the solvent to be used include alcohols such as methanol, ethanol, propanol and isopropanol, ethers such as diethyl ether, diisopropyl ether, tetrahydrofuran and dioxane; aprotic polar solvents such as dimethylformamide, dimethylacetamide and dimethylsulfoxide; nitriles such as acetonitrile; esters such as methyl acetate and ethyl acetate; aromatic hydrocarbons such as benzene, toluene and xylene; and aliphatic hydrocarbons such as pentane, hexane and heptane.

Examples of the base to be used include alkali metal alkoxides such as sodium methoxide, sodium ethoxide and potassium t-butoxide; alkali metal hydrides such as sodium hydride and lithium hydride; alkali metal hydroxides such as sodium hydroxide and potassium hydroxide; alkali metal carbonates such as sodium carbonate and potassium carbonate; and amines such as triethylamine, tributylamine, pyridine, picoline and 1,8-diazabicyclo[5.4.0]-7-undecene.

When the compound (5) wherein m' is 0 is used as the starting material of this step, a compound corresponding to the above-described cyclic sulfide compound (Ia') is prepared by the cyclization reaction in accordance with the above-described a) or b). The compound (Ib) can be prepared by oxidizing the sulfide group of this compound. This oxidation is effected in a similar manner to that described in step 2.

### [Method C: the preparation of the compound (Ic), wherein R⁴ is a lower alkyl group]

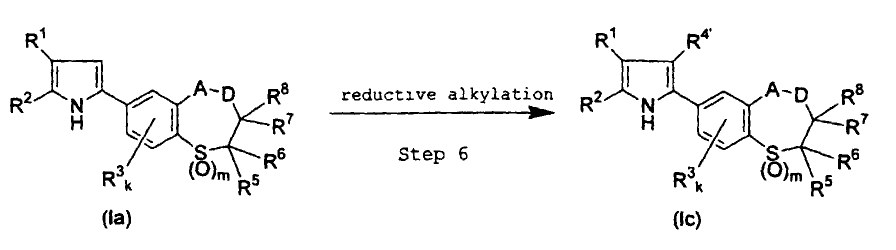

(wherein, D, A, R¹, R², R³, R⁵, R⁶, R⁷, R⁸, k and m have the same meanings as described above, and
R^{4'} represents the same lower alkyl group as that defined for R⁴ ).

Step 6 is reductive alkylation at the 4-position of the pyrrole ring of compound (Ia) or (Ib) according to the present invention, to give compound (Ic), which is the compound (I) wherein R⁴ a lower alkyl group. This step is carried out in a similar manner to that described in the literature (B.V. Gregorovich et al., Can. J. Chem., 46, 3291(1968)).

For the preparation of a cyclic sulfonyl compound of the formula (I) wherein m represents 2, the corresponding cyclic sulfoxide compound [compound of the formula (I) wherein m represents 1] is oxidized in a similar manner to that described in the step 2. While for the preparation of a cyclic sulfoxide compound of the formula (I) wherein m represents 1, the corresponding cyclic sulfide compound [compound of the formula (I) wherein m represents 0] is oxidized in a similar manner to that described in the step 2.

### [Method D: preparation of the compound (Id), wherein A represents -C(R⁹)(R¹⁰) (wherein, R⁹ represents a hydroxyl group and R¹⁰ has the same meaning as described above)]

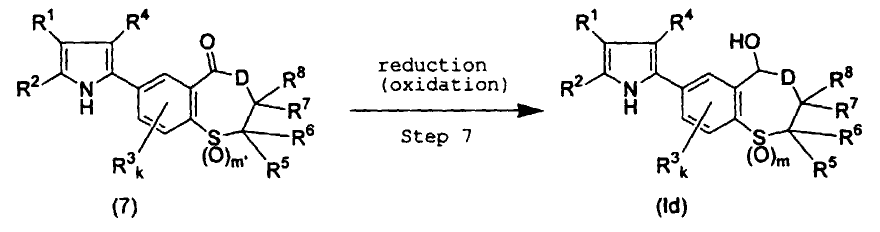

(wherein, R¹, R², R³, R⁴, R⁵, R⁶, R⁷, R⁸, k, m and m' have the same meanings as described above).

Step 7 is a reduction of the carbonyl group of compound (7) to prepare the compound (Id) of the present invention. Reduction using a hydride reagent, for example, alkali metal borohydride such as sodium borohydride or lithium borohydride; aluminum hydride compound such as lithium aluminum hydride or lithium triethoxide aluminum hydride; tellurium sodium hydride; or organoaluminum hydride type reducing agent such as diisobutylaluminum hydride or di(methoxyethoxy)aluminum sodium dihydride or catalytic reduction with hydrogen can be adopted. The reaction is carried our in accordance with the process specifically described in J. Dale, J. Chem. Soc., 910(1961) or F.B. Bordwell, et al., J. Org. Chem., 33, 3385(1968).

In this step, when the compound (7) wherein m' is 0, is employed as a starting material or when a sulfide compound corresponding to the compound (Id) is prepared by the above-described reduction, the compound (Id) is obtainable by an oxidation reaction in a similar manner to that described above in step 2.

### [Method E: preparation of the compound (Ie), wherein A represents -C(R⁹)(R¹⁰)- (wherein, R⁹ represents a halogen atom and R¹⁰ has the same meaning as described above)]

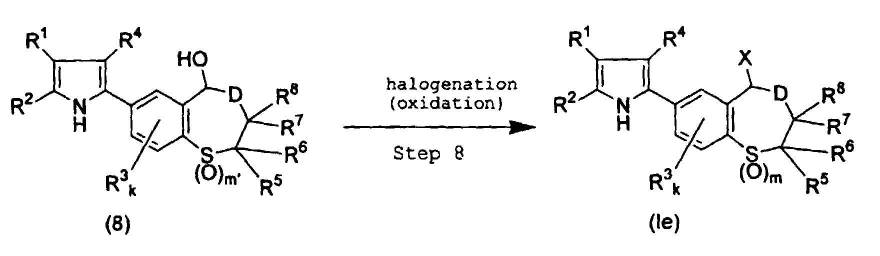

(wherein, D, R¹, R², R³, R⁴, R⁵, R⁶, R⁷, R⁸, k, m and m' have the same meanings as described above, and
X represents a fluorine, chlorine, bromine or iodine atom].

Step 8 is a substitution reaction of the hydroxyl group of compound (8) with a halogen atom, to give compound (Ie) of the present invention. In the above-described halogenation, fluorination using diethylaminosulfur trifluoride (DAST); chlorination using thionyl chloride, phosphorus trichloride, phosphorus pentachloride, phosphorus oxychloride or triphenylphosphine/carbon tetrachloride; bromination using hydrobromic acid, thionyl bromide, phosphorus tribromide or triphenylphosphine/carbon tetrabromide; or iodination using hydroiodic acid or phosphorus triiodide can be employed. The reaction is effected, for example, by the process described specifically in W.J. Middleton, J. Org. Chem., 40, 575(1957) or C.R. Noller & R. Dinsmore, Org. Synth., II, 358(1943).

When the compound (8) wherein m' stands for 0 is employed as the starting material in this step, the compound (Ie) can be obtained by oxidation in accordance with the process as described in step 2.

### [Method F: preparation of the compound (If), of which A is -C(R⁹)(R¹⁰)- (wherein R⁹ and R¹⁰ each represents a halogen atom)]

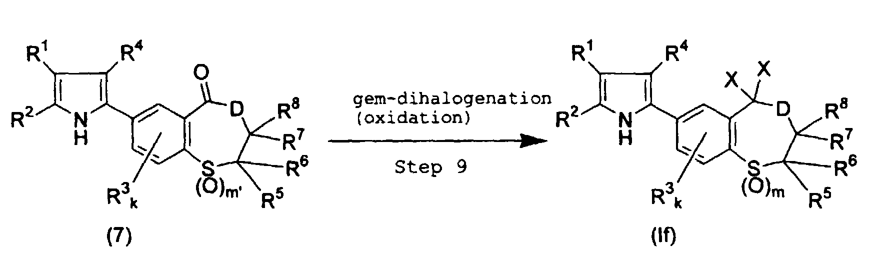

(wherein, D, R¹, R², R³, R⁴, R⁵, R⁶, R⁷, R⁸, X, k, m and m' have the same meanings as described above).

Step 9 is gem-dihalogenation of the carbonyl group of compound (7) to prepare the compound (If) of the present invention. In the gem-dihalogenation, for example, gem-difluorination using sulfur tetrafluoride or DAST, gem-dichlorination using phosphorus pentachloride or thionyl chloride/dimethylformamide; gem-dibromination using boron tribromide or gem-diiodination using trimethylsilicon iodide can be adopted in this step. The gem-dihalogenation is conducted in accordance with the process as described specifically in W.J. Middleton, J. Org. Chem., 40, 574(1975) or M.E. Jung, et al, J. Org. Chem., 43, 3698(1978).

When the compound (7) wherein m' is 0 is employed as a starting material in this step, the compound (If) can be obtained by oxidation in a similar process to that described in step 2.

### [Method G: preparation of the compound (Ig), of which A is C=NOR¹¹ (wherein, R¹¹ has the same meaning as described above)]

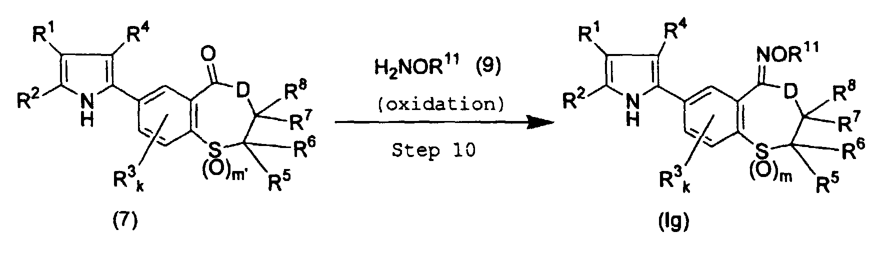

(wherein, D, R¹, R², R³, R⁴, R⁵, R⁶, R⁷, R⁸, R¹¹, k, m and m' have the same meanings as described above).

Step 10 is a condensation of compound (7) with a hydroxyamine derivative (9) to give compound (Ig) of the present invention. This step is carried out in a conventional manner (for example, the process described by E.W. Bousquet, Org. Synth., II, 3 13(1947)).

When the compound (7) wherein m' is 0 is employed as the starting material in this step, the compound (Ig) can be obtained by oxidation in a similar manner to that described in step 2.

The compounds to be used as the starting materials of the above-described methods A and B, that is, the compounds (1), (2) and (4) are each a known compound or a compound which can be prepared from a known compound according to known processes.

For example, the compound (2) can be prepared in accordance with the following process. 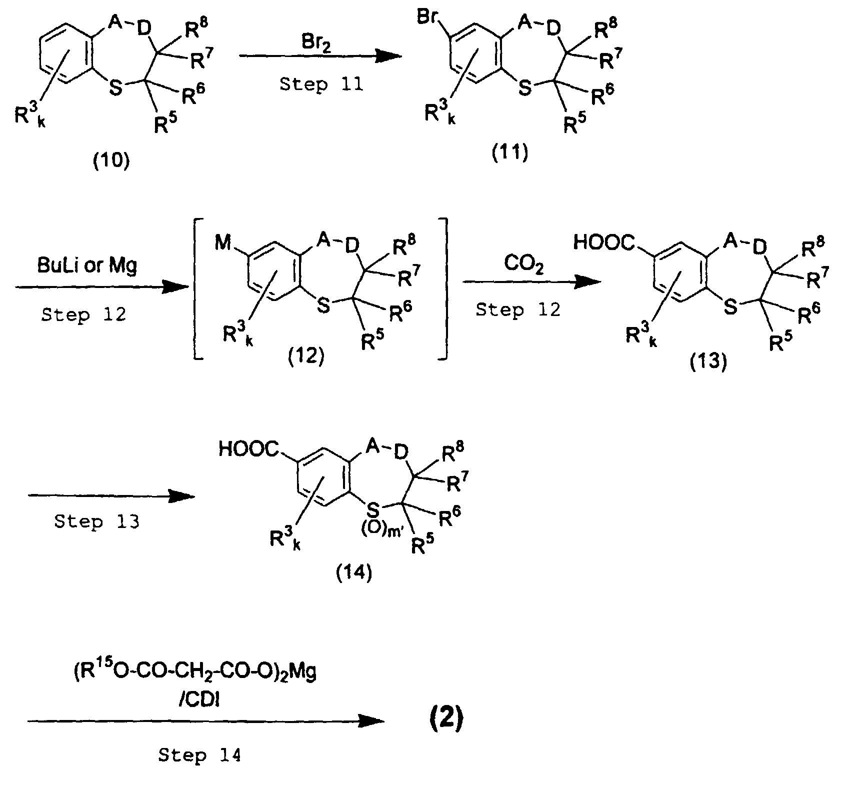 (wherein, A, D, R³, R⁵, R⁶, R⁷, R⁸, R¹⁵, k and m' have the same meanings as described above, and
M represents lithium or magnesium mono-bromide).

Step 11 is a reaction of compound (10) with bromine to introduce a bromine atom into the benzene ring, to give the brominated compound (11). This step is effected, for example, in accordance with the process as described by H. Becker, et al., "Organikum", VEB Deutscher Vorlag der Wissenschaften (1973), p189.

Step 12 is a reaction of the brominated compound (11) with butyl lithium or with magnesium to prepare the corresponding organic metal compound (12) and then a reaction of the resulting compound with carbon dioxide gas (CO₂), to give the carboxylic acid compound (13). This step is effected, for example, in accordance with the process as described in M.E. Volpin, J.S. Kolomnikov, Organometallic React., 5, 313(1975).

Step 13 is conversion of the sulfur atom of the carboxylic acid compound (13) into >S(O)_{m'} by oxidation if desired, to give compound (14). This step is carried out in a similar manner to that described in step 2.

Step 14 is a reaction of the carboxylic acid compound (14) with magnesium malonic acid mono-ester in the presence of 1,1'-carbonyl diimidazole (CDI) and an organic base, to give compound (2).

There is no particular limitation on the nature of the solvent to be used in this step provided that it has no adverse effect to the reaction and dissolves therein the starting substance to some extent. Preferred examples include aromatic hydrocarbons such as benzene, toluene and xylene; halogenated hydrocarbons such as methylene chloride, chloroform, carbon tetrachloride, dichloroethane, chlorobenzene and dichlorobenzene; esters such as ethyl formate, ethyl acetate, propyl acetate, butyl acetate and diethyl carbonate; ethers such as diethyl ether, diisopropyl ether, tetrahydrofuran, dioxane, dimethoxyethane and diethylene glycol dimethyl ether; nitriles such as acetonitrile and isobutyronitrile; amides such as formamide, N,N-dimethylformamide, N,N-dimethylacetamide, N-methyl-2-pyrrolidone and hexamethylphosphorous triamide; and sulfoxides such as dimethylsulfoxide and sulfolane, of which the aromatic hydrocarbons and ethers are preferred.

There is no particular limitation on the nature of the organic base to be used in this step, provided that it is used as a base in ordinary reactions. Examples include amines such as triethylamine, tributylamine, pyridine, picoline and 1,8-diazabicyclo[5.4.0]-7-undecene, of which triethylamine is preferred.

The reaction is carried out at a temperature range of from -20°C to 100°C, preferably from 0°C to 50°C.

Although the reaction time varies mainly depending on the reaction temperature, starting material compounds, reaction reagents and the nature of the solvent to be used, it usually ranges from 10 minutes to 3 days, preferably from 1 hour to 24 hours.

Reactions are carried out in similar manners to that described in the steps 11 to 14 using compound (15) instead of compound (10), to give compound (4). 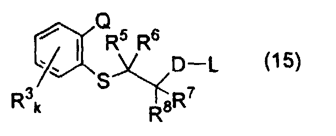 (wherein, D, Q, L, R³, R⁵, R⁶, R⁷, R⁸ and k have the same meanings as descried above)

After completion of each reaction described above, the desired compound is isolated from the reaction mixture in a conventional manner.

For example, it is obtained by neutralizing the reaction mixture as needed, removing the insoluble matters by filtration if any, adding organic solvents which are not miscible each other, such as water and ethyl acetate, washing with water or the like, separating the organic layer containing the desired compound, drying it over anhydrous magnesium sulfate or the like and then distilling off the solvent.

If necessary, the desired compound thus obtained can be isolated and purified by using a conventional method such as recrystallization or reprecipitation and chromatography in which a method ordinarily employed for the isolation and purification of an organic compound in combination as needed and eluting using a proper eluant. Examples of chromatography include adsorption column chromatography using a carrier such as silica gel, alumina or magnesium-silica gel type Florisil, chromatography using a synthetic adsorbent, for example, partition column chromatography using a carrier such as Sephadex LH-20 (product of Pharmacia), Amberlite XAD-11 (product of Rohm & Haas) or Diaion HP-20 (product of Mitsubishi Chemical), ion exchange chromatography or normal-phase·reverse-phase column chromatography (high-performance liquid chromatography) using a silica gel or alkylated silica gel.

Since the pyridylpyrrole derivatives of the present invention exhibit excellent inhibitory activity against the production of inflammatory cytokines, it is effective as a medicament (particularly, an agent for prevention or treatment of the diseases mediated by inflammatory cytokines). Examples of such a medicament include an analgesic, antiinflammatory drug and virucide, and an agent for prevention or treatment of chronic rheumatism, osteoarthritis, allergosis, asthma, sepsis, psoriasis, osteoporosis, autoimmune diseases (e.g. systemic lupus erythematosus, ulcerative colitis, Crohn's disease), diabetes, glomerular nephritis or arteriosclerosis, of which the analgesics, antiinflammatory drug and agent for prevention or treatment of chronic rheumatism, osteoarthritis, allergosis, sepsis, psoriasis, osteoporosis, ulcerative colitis, diabetes and arteriosclerosis are particularly preferred.

Examples of the administration route of the compound (I), or pharmacologically acceptable salt or derivative thereof according to the present invention include oral administration in the form of tablets, capsules, granules, powders or syrups and parental administration in the form of injections or suppositories. Such formulations can be prepared in a known manner by using additives such as an excipient, lubricant, binder, disintegrator, stabilizer, corrigent or diluent.

Examples of the excipient include organic excipients. e.g., sugar derivatives such as lactose, sucrose, dextrose, mannitol and sorbitol; starch derivatives such as corn starch, potato starch, α-starch, dextrin and carboxymethyl starch; cellulose derivatives such as crystalline cellulose, low-substituted hydroxypropylcellulose, hydroxypropylmethylcellulose, carboxymethylcellulose, calcium carboxymethylcellulose and sodium internally-crosslinked carboxymethylcellulose; gum arabic; dextran; and pullulan; and inorganic excipients, e.g., silicate derivatives such as soft silicic acid anhydride, synthetic aluminum silicate and magnesium aluminometasilicate; phosphates such as calcium phosphate; carbonates such as calcium carbonate; and sulfates such as calcium sulfate.

Examples of the lubricant include stearic acid; metal salts of stearic acid such as calcium stearate and magnesium stearate; talc; colloidal silica; waxes such as bee gum and spermaceti; boric acid; adipic acid; sulfates such as sodium sulfate; glycol; fumaric acid; sodium benzoate; DL-leucine; sodium salts of an aliphatic acid; lauryl sulfates such as sodium lauryl sulfate and magnesium lauryl sulfate; silicic acid derivatives such as silicic acid anhydride and silicic acid hydrate; and starch derivatives exemplified above as the excipient.

Examples of the binders include polyvinylpyrrolidone, Macrogol and compounds similar to those exemplified above as the excipient.

Examples of the disintegrator include compounds similar to those exemplified above as the excipient and chemically modified starch or cellulose derivatives such as sodium cross carmellose, sodium carboxymethyl starch and crosslinked polyvinylpyrrolidone.

Examples of the stabilizer include paraoxybenzoate esters such as methylparaben and propylparaben; alcohols such as chlorobutanol, benzyl alcohol and phenylethyl alcohol; benzalkonium chloride; phenol derivatives such as phenol and cresol; thimerosal; dehydroacetic acid; and sorbic acid.

Examples of the corrigent include ordinarily-employed sweeteners, acidifiers and flavors.

The dose of the compound (I) or pharmacologically acceptable salt or derivative thereof according to the present invention will vary depending on the condition, age of the patient, or administration route. Orally, it is administered to an adult in an amount of 0.1 mg (preferably 0.5 mg) a day as a lower limit and 2000 mg (preferably 500 mg) a day as an upper limit. It is desired to be administered in one to several portions depending on the condition of the patient. Intravenously, it is administered to an adult in an amount of 0.01 mg (preferably 0.05 mg) a day as a lower limit and 200 mg (preferably 50 mg) a day as an upper limit. It is desired to be administered in one to several portions per day depending on the condition of the patient.

### [Best Modes for Carrying Out the Invention]

The present invention will hereinafter be described more specifically by examples, formulation examples and test examples. However the present invention is not limited to these.

### [Example]

### [Example 1]

### 2-(4-Fluorophenyl)-5-(2,3-dihydro-4-oxo-1,4-benzooxathiin-7-yl)-3-(pyridin-4-yl)-1H-pyrrole (Exemplification compound No. 4-25)

### 1) 2-(t-Butyldimethylsilyloxy)-4'-fluoro-2-(pyridin-4-yl)acetophenone

A solution of n-butyllithium in hexane (74.78 ml of 1.68 M n-butyllithium/hexane solution, 0.125 mol) was added dropwise to a solution of diisopropylamine (12.13 g, 0.120 mol) in anhydrous tetrahydrofuran (130 ml) at -40°C under nitrogen atmosphere. To this mixture was added dropwise a solution of 4-(t-butyldimethylsilyloxymethyl)pyridine (25.51 g, 0.114 mol) in anhydrous tetrahydrofuran (25 ml). The mixture was stirred at -40°C for 1 hour. At the end of this time a solution of 4-fluoro-(N-methoxy-N-methyl)benzamide (20.92 g, 0.114 mol) in anhydrous tetrahydrofuran (45 ml) was added dropwise to the reaction mixture. This mixture was stirred at the same temperature for 2 hours, the cooling bath was removed and then the mixture warmed up to room temperature. To the reaction mixture, saturated aqueous ammonium chloride solution was added and then it was extracted with ethyl acetate. The organic layer was washed with water and dried over anhydrous magnesium sulfate and concentrated under reduced pressure. The residue was purified by chromatography on a silica gel column using hexane/ethyl acetate = 4/1 as the eluent to afford the desired compound (33.43 g, yield 85%) as an orange oil.
Nuclear magnetic resonance spectrum (270MHz, CDCl₃) δppm:
   8.60(2H,d,J=6Hz), 8.04(2H,dd,J=9Hz,5Hz), 7.46(2H,d,J=6Hz),
   7.04(2H,t,J=9Hz), 5.61(1H,s), 0.91(9H,s), 0.12(6H,s).

### 2) 3-Fluoro-4-(2-hydroxyethylthio)benzonitrile

A mixture of dimethylformamide (100 ml), 2-mercaptoethanol (2.52 ml, 35.95 mmol) and potassium butoxide (4.03g, 35.95 mmol) was stirred for 30 minutes with ice-cooling. At the end of this time to this mixture was added 3,4-difluorobenzonitrile (5.00 g, 35.95 mmol) and the mixture was stirred at 75°C for 2 hours. After cooling it to room temperature, water (200ml) was added to it and then the mixture was extracted with ethyl acetate. The organic layer was washed with water, dried over anhydrous magnesium sulfate and concentrated under reduced pressure to afford the desired compound (7.05 g, quantitative yield) as a pale brown oil.
Nuclear magnetic resonance spectrum (270MHz, CDCl₃) δppm:
   7.49-7.30(3H,m), 3 .85(2H,q,J=6Hz), 3.21(2H,t,J=6Hz),
   1.93(1H,br.t,J=6Hz).

### 3) 3-Fluoro-4-(2-hydroxyethylthio)benzoic acid

A mixture of 3-fluoro-4-(2-hydroxyethylthio)benzonitrile (1.00 g, 5.07 mmol), which was obtained in 2), acetic acid (10 ml), conentrated sulufuric acid (3 ml) and water (7 ml) was stirred at 130°C for 4 hours. After cooling it to room temperature the pH value of the reaction mixture was adjusted to about 3.0 by adding aqueous ammonia solution (28%) and it was extracted with ethyl acetate. The organic layer was washed with water, dried over anhydrous magnesium sulfate and concentrated under reduced pressure to afford the desired product (1.08 g, yield 99%) as a brown solid.
Nuclear magnetic resonance spectrum (270MHz, CDCl₃) δppm:
   7.88-7.82(1H,m), 7.78-7.71(1H,m), 7.50-7.42(1H,m),
   4.29(2H,t,J=7Hz), 3.25(2H,t,J=7Hz).

### 4) 4-(2-Acetoxyethylthio)-3-fluorobenzoic acid

Pyridine (10 ml) and acetic anhydride (8.62 ml, 91.41 mmol) were added to 3-fluoro-4-(2-hydroxyethylthio)benzoic acid (6.59 g, 30.47 mmol), which was obtained in 3). The mixture was stirred at 70°C for 1 hour. After cooling it to room temperature the reaction mixture was acidified with acetic acid and extracted with ethyl acetate. The organic layer was washed with water, dried over anhydrous magnesium sulfate and then concentrated under reduced pressure to afford the desired compound (7.69 g, yield 98%) as a brown oil.
Nuclear magnetic resonance spectrum (270MHz, CDCl₃) δppm:
   7.88-7.83(1H,m), 7.79-7.72(1H,m), 7.50-7.42(1H,m),
   4.29(2H,t,J=7Hz), 3.24(2H,t,J=7Hz), 2.04(3H,s).

### 5) Methyl [4-(2-acetoxyethylthio)-3-fluoro]benzoylacetate

1,1'-carbonyldiimidazole (9.05 g, 55.84 mmol) was added to a suspension of 4-(2-acetoxyethylthio)-3-fluorobenzoic acid (13.11 g, 50.76 mmol), which was obtained in 4), in anhydrous tetrahydrofuran (50 ml). The mixture was stirred at room temperature for 3 hours to give a homogeneous solution. At the same time, a mixture of potassium methyl malonate (11.89 g, 76.14 mmol), triethylamine (21.23 ml, 152.3 mmol), magnesium chloride (9.66 g, 101.5 mmol) and anhydrous tetrahydrofuran (100 ml) was stirred at room temperature for 3 hours. To this reaction mixture, the homogeneous solution obtained above was added dropwise at 0 - 5°C. After that this mixture was stirred at room temperature overnight. At the end of this time the reaction mixture was acidified with 1N hydrochloric acid and extracted with ethyl acetate. The organic layer was washed with water, dried over anhydrous magnesium sulfate and then concentrated under reduced presuure. The residue was purified by chromatography on a silica gel column using hexane/ethyl acetate = 2/1 as the eluant to afford the desired compound (13.83 g, yield 87%) as a colorless oil.
Nuclear magnetic resonance spectrum (270MHz, CDCl₃) δppm:
   7.72-7.40(3H,m), 4.28(2H,t,J=7Hz), 3.96(2H,s), 3.76(3H,s),
   3.25(2H,t,J=7Hz), 2.05(3H,s).

### 6) 5-[4-(2-Acetoxyethylthio)-3-fluorophenyl]-2-(4-fluorophenyl)-4-methoxycarbonyl-3-(pyridin-4-yl)-1H-pyrrole

A mixture of 2-(t-butyldimethylsilyloxy)-4'-fluoro-2-(pyridin-4-yl)acetophenone (15.30 g, 44.28 mmol), which was obtained in 1), methyl [4-(2-acetoxyethylthio)-3-fluoro]benzoylacetate (13.83 g, 44.28 mmol), which was obtained in 5), ammonium acetate (13.65 g, 177.0 mmol) and acetic acid (150 ml) was stirred at 130°C for 1 hour. After cooling to room temperature the reaction mixture was made basic with aqueous ammonia solution under ice-cooling and then extracted with ethyl acetate. The organic layer was washed with water, dried over anhydrous magnesium sulfate and concentrated under reduced pressure. The residue was purified by chromatography on a silica gel column using hexane/ethyl acetate = 2/5 as the eluant to afford the desired compound (5.71 g, yield 25%) as a pale yellow oil.
Nuclear magnetic resonance spectrum (270MHz, CDCl₃) δppm:
   8.88-8.82(1H,br.s), 8.53(2H,d,J=6Hz), 7.63-6.94(9H,m),
   4.26(2H,t,J=12Hz), 3.56(3H,s), 3.19(2H,t,J=7Hz),
   2.04(3H,s).

### 7) 2-(4-Fluorophenyl)-5-[3-fluoro-4-(2-hydroxyethylthio)phenyl]-3-(pyridin-4-yl)-1H-pyrrole

(a) A mixture of 5-[4-(2-acetoxyethylthio)-3-fluorophenyl]-2-(4-fluorophenyl)-4-methoxycarbonyl-3-(pyridin-4-yl)-1H-pyrrole (5.71 g, 11.24 mmol), which was obtained in 6), acetic acid (30 ml) and aqueous sulfuric acid solution (30% v/v, 30 ml) was stirred at 130°C for 15 hours. After cooling to room temperature the reaction mixture was made basic with aqueous ammonia solution (28%). The precipitate was collected by filtration to afford the O-acetyl derivative of the desired compound (4.96 g, yield 98%) as a pale yellow powder.
   Nuclear magnetic resonance spectrum (270MHz, CDCl₃) δppm:
      8.93-8.82(1H,br.s), 8.45(2H,d,J=6Hz), 7.65-7.50(2H,m),
      7.46(1 H,d,J=7Hz), 7.38(2H,dd,J=9Hz,5Hz), 7.22(2H,d,J=6Hz),
      7.09(2H,t,J=9Hz), 6.77(1H,d,J=3Hz), 4.24(2H,t,J=7Hz).
      3.14(2H,t,J=7Hz), 2.02(3H,s).
(b) A mixture of the O-acetyl derivative, methanol (50 ml) and aqueous sodium hydroxide solution (1N, 22 ml) was heated at reflux for 1 hour. The reaction mixture was concentrated under reduced pressure. To the residue, water was added then the mixture was extracted with ethyl acetate. The organic layer was washed with water, dried over anhydrous magnesium sulfate and then concentrated under reduced pressure to afford the desired compound (3.70 g, yield 82%) as a pale yellow powder.
   Nuclear magnetic resonance spectrum (270MHz, CDCl₃) δppm:
      11.45-11.29(1H,br.s), 8.44(2H,d,J=6Hz), 7.84-7.71(2H,m),
      7.58(1H,d,J=12Hz), 7.45(2H,dd,J=9Hz,5Hz),
      7.22(2H,d,J=6Hz), 7.09(2H,t,J=9Hz), 6.86(1H,d,J=3Hz),
      4.05-3.90(2H,m), 3.89-3.77(1H,m), 3.06-2.96(1H,m).

### 8) 2-(4-Fluorophenyl)-5-[3-fluoro-4-(2-hydroxyethylsulfinyl)phenyl]-3-(pyridin-4-yl)-1H-pyrrole

m-Chloroperbenzoic acid (70%, 1.56 g, 9.06 mmol) was added in small portions to a solution of 2-(4-fluorophenyl)-5-[3-fluoro-4-(2-hydroxyethylthio)phenyl]-3-(pyridin-4-yl)-1H-pyrrole (3.70 g, 9.06 mmol), which was obtained in 1-7), in tetrahydrofuran (70 ml) under ice-cooling with stirring. After the addition the mixture was further stirred at the same temperature for 30 minutes. To the reaction mixture. ethyl acetate and aqueous sodium thiosulfate solution (10%) were added, and then the mixture was shaken hard. The organic layer was washed with saturated aqueous sodium hydrogencarbonate solution and then with water, dried over anhydrous magnesium sulfate and then concentrated under reduced pressure. The residue was purified by chromatography on a silica gel column using ethyl acetate/methanol = 95/5 as the eluant to afford the desired compound (1.85 g, yield 48%) as a yellow powder.
Nuclear magnetic resonance spectrum (270MHz, CDCl₃) δppm:
   11.56-11.41(1H,br.s), 8.44(2H,d,J=6Hz), 7.83-7.73(2H,m),
   7.61(1H,d,J=12Hz), 7.46(2H,dd,J=9Hz,5Hz),
   7.22(2H,d,J=6Hz), 7.09(2H,t,J=9Hz), 6.87(1H,d,J=3Hz),
   4.684.57(1H,m), 4.19-4.05(1H,m), 4.02-3.90(1H,m),
   3.37-3.23(1H,m), 3.06-2.94(1H,m).

### 9) 2-(4-Fluorophenyl)-5-(2,3-dihydro-4-oxo-1,4-benzooxathiin-7-yl)-3-(pyridin-4-yl)-1H-pyrrole

Potassium t-butoxide (264 mg, 2.36 mol) and 18-crown-6 (catalytic amount) were added to a solution of 2-(4-fluorophenyl)-5-[3-fluoro-4-(2-hydroxyethylsulfinyl)phenyl]-3-(pyridin-4-yl)-1H-pyrrole
(1.00 g, 2.36 mmol) in dimethylformamide (30 ml). The mixture was stirred at 150°C for 9 hours. To the reaction mixture, water was added and the mixture was extracted with ethyl acetate. The organic layer was washed with water, dried over anhydrous magnesium sulfate and then concentrated under reduced pressure. The residue was purified by chromatography on a silica gel column using successively ethyl acetate - ethyl acetate/methanol = 97.5/2.5 - ethyl acetate/methanol = 95/5 as the eluant to afford the title compound (248 mg, yield 21%) as a pale brown powder.
Melting point: 224 - 232 °C
Nuclear magnetic resonance spectrum (270MHz, CDCl₃) δppm:
   8.94-8.83(1H,br.s), 8.48(2H,d,J=6Hz), 7.60(1H,d,J=8Hz),
   7.40(2H,dd,J=9Hz,5Hz), 7.28-7.19(3H,m), 7.17-7.06(3H,m),
   6.83(1H,d,J=3Hz), 4.82(1H,dt,J=12Hz,3Hz),
   4.59(1H,dt,J=12Hz,3Hz), 3.22-2.96(2H,m).

### [Example 2]

### 2-(4-Fluorophenyl)-5-(2,3-dihydro-4,4-dioxo-1,4-benzooxathiin-7-yl)-3-(pyridin-4-yl)-1H-pyrrole (Exemplification compound No. 4-137)

In a similar manner to that described in Example 1-8), the compound (110 mg, 0.27 mmol) of Example 1 was oxidized. The reaction mixture was purified by chromatography on a silica gel column using hexane/ethyl acetate = 1/3 as the eluant to afford the title compound (24 mg, yield 21%) as a pale yellow powder.
Melting point: 286 - 288 °C
Nuclear magnetic resonance spectrum (270MHz, CDCl₃) δppm:
   11.99-11.16(1H,br.s), 7.77(2H,d,J=6Hz), 7.52-7.38(3H,m),
   7.35(2H,d,J=10Hz), 7.21(2H,d,J=6Hz), 7.08(2H,t,J=9Hz),
   6.84(1H,d,J=3Hz), 4.92-4.81(2H,m), 3.60-3.49(2H,m).

### [Example 3]

### 2-(4-Fluorophenyl)-5-(2,3-dihydro-1-oxo-2H-1,4-benzothiazin-6-yl)-3-(pyridin-4-yl)-1H-pyrrole (Exemplification compound No. 5-26)

### 1) Methyl 4-fluoro-3-nitrobenzoate

A mixture of 4-fluoro-3-nitrobenzoic acid (26.52 g, 143 mmol), methanol (260 ml) and concentrated sulfuric acid (26 ml) was heated at reflux for 2 hours. After cooling to room temperature the reaction mixture was concentrated under reduced pressure. To the residue, water was added and the mixture was extracted with ethyl acetate. The organic layer was washed successively with water, saturated aqueous sodium hydrogencarbonate solution and water, dried over anhydrous magnesium sulfate and then concentrated under reduced pressure to afford the desired compound (28.77 g, quantitative yield) as a white powder.
Nuclear magnetic resonance spectrum (270MHz, CDCl₃) δppm:
   8.75(1H,dd,J=7Hz,2Hz), 8.37-8.18(1H,m),
   7.39(1H,dd,J=10Hz,9Hz), 3.98(3H,s).

### 2) Methyl 4-(2-hydroxyethylthio)-3-nitrobenzoate

Potassium butoxide (0.56 g, 5.02 mmol) and 2-mercaptoethanol (0.35 ml, 5.02 mnol) were added to a solution of methyl 4-fluoro-3-nitrobenzoate (1.00 g, 5.02 mmol), which was obtained in 1), in dimethylformamide (20 ml). The mixture was stirred under ice-cooling for 30 minutes. To the reaction mixture, water was added and the mixture was extracted with ethyl acetate. The organic layer was washed with water, dried over anhydrous magnesium sulfate and then concentrated under reduced pressure. The residue was purified by chromatography on a silica gel column using hexane/ethyl acetate = 1/1 as the eluant to afford the desired compound (1.42 g, quantitative yield) as a pale yellow powder.
Nuclear magnetic resonance spectrum (270MHz, CDCl₃) δppm: 8.83(1H,d,J=2Hz), 8.17(1H,dd,J=9Hz,2Hz), 7.56(1H,d,J=9Hz),
   4.03-3.92(5H,m), 3.27(2H,t,J=6Hz), 2.17-2.06(1H,br.s).

### 3) Methyl 3-amino-4-(2-hydroxyethylthio)benzoate

Zinc powder (11.92g, 364 mmol) was added in small portions to a solution of methyl 4-(2-hydroxyethylthio)-3-nitrobenzoate (13.81 g, 60.76 mmol), which was obtained in 2) in acetic acid (120 ml) with stirring. This reaction was an exothermic reaction but the reaction mixture was stirred for 30 minutes. After cooling to room temperature the reaction mixture was concentrated under reduced pressure. To the residue, saturated aqueous sodium hydrogencarbonate solution was added and the mixture was extracted with ethyl acetate. The organic layer was washed with water, dried over anhydrous magnesium sulfate and then concentrated under reduced pressure. The residue was purified by chromatography on a silica gel column using hexane/ethyl acetate = 1/1 as the eluant to afford the desired compound (9.58 g, yield 69%) as a pale yellow oil.
Nuclear magnetic resonance spectrum (270MHz, CDCl₃) δppm:
   7.46-7.33(3H,m), 4.56-4.36(2H,br.s), 3.89(3H,s),
   3.68(2H,q,J=6Hz), 2.99(2H,t,J=6Hz), 2.33(1H,br.t,J=6Hz).

### 4) Methyl 3-amino-4-(2-chloroethylthio)benzoate

Triphenylphosphine (30.12 g, 114.8 mmol) was added to a solution of methyl 3-amino-4-(2-hydroxyethylthio)benzoate (8.70 g, 38.28 mmol), which was obtained in 3), in a mixture of carbon tetrachloride (50 ml) and acetonitrile (50 ml). The mixture was heated at reflux for 1 hour. After cooling to room temperature the reaction mixture was concentrated under reduced pressure. The residue was purified by chromatography on a silica gel column using hexane/ethyl acetate = 4/1 as the eluant to afford the desired compound (4.73 g, yield 50%) as a white powder.
Nuclear magnetic resonance spectrum (270MHz, CDCl₃) δppm:
   7.46-7.32(3H,m), 4.87-4.54(2H,br.s), 3.91(3H,s),
   3 .58(2H,t,J=8Hz), 3.12(2H,t,J=8Hz).

### 5) Methyl 3,4-dihydro-2H-1,4-benzothiazine-6-carboxylate

Potassium carbonate (2.93 g, 21.18 mmol) and potassium iodide (4.79 g, 28.88 mmol) were added to a solution of methyl 3-amino-4-(2-chloroethylthio)benzoate (4.75 g, 19.25 mmol), which was obtained in 4), in dimethylformamide (50 ml). The mixture was stirred at 100°C for 1 hour. After cooling to room temperature the reaction mixture was concentrated under reduced pressure. To the residue, water was added and the mixture was extracted with ethyl acetate. The organic layer was washed with water, dried over anhydrous magnesium sulfate and then concentrated under reduced pressure to afford the desired compound (3.60 g, yield 88%) as a brown powder.
Nuclear magnetic resonance spectrum (270MHz, CDCl₃) δppm:
   7.27(1H,dd,J=8Hz,2Hz), 7.14(1H,d,J=2Hz), 7.03(1H,d,J=8Hz),
   4.18-4.03(1H,br.s), 3.86(3H,s), 3.69-3.59(2H,m), 3.14-3.05(2H,m).

### 6) 3,4-Dihydro-2H-1,4-benzothiazine-6-carboxylic acid

A mixture of methyl 3,4-dihydro-2H-1,4-benzothiazine-6-carboxylate (6.32 g, 30.20 mmol), which was obtained in 5), methanol (120 ml), water (60 ml) and aqueous sodium hydroxide solution (1N, 45 ml, 45.00 mmol) was heated at reflux for 1 hour. After cooling to room temperature the methanol of the reaction mixture was evaporated under reduced pressure. The residue was acidified to less than pH 2.0 with hydrochloric acid (2N) and extracted wit ethyl acetate. The organic layer was washed with water, dried over anhydrous magnesium sulfate and then concentrated under reduced pressure to afford the desired compound (6.14 g, quantitative yield) as a pale brown powder.
Nuclear magnetic resonance spectrum (270MHz, CDCl₃) δppm:
   7.29(1H,dd,J=8Hz,2Hz), 7.17(1H,d,J=2Hz), 7.02(1H,d,J=8Hz),
   3.67-3.60(2H,m), 3.13-3.05(2H,m).

### 7) 4-Acetyl-3,4-dihydro-2H-1,4-benzothiazine-6-carboxylic acid

A mixture of 3,4-dihydro-2H-1,4-benzothiazine-6-carboxylic acid (5.69 g, 29.14 mmol), which was obtained in 6), pyridine (7.07 ml, 87.42 mmol), 4-dimethylaminopyridine (0.36 g, 2.91 mmol) and acetic anhydride (5.50 ml, 58.28 mmol) was stirred at 70°C for 1 hour. After cooling to room temperature, water was added to the reaction mixture. The mixture was acidified to less than pH 2.0 with hydrochloric acid (2N) and extracted with ethyl acetate. The organic layer was washed with water, dried over anhydrous magnesium sulfate and then concentrated under reduced pressure to afford the desired compound (7.39 g, quantitative yield) as a brown powder.
Nuclear magnetic resonance spectrum (270MHz, CDCl₃) δppm:
   7.93-7.78(2H,m), 7.33(1H,d,J=8Hz), 4.02(2H,br.t,J=6Hz),
   3.27(2H,t,J=6Hz), 2.23(3H,s).

### 8) Methyl (4-acetyl-3,4-dihydro-2H-1,4-benzothiazine-6-carbonyl)acetate

In a similar manner to that described in Example 1-5), a reaction was carried out using 4-acetyl-3,4-dihydro-2H-1,4-benzothiazine-6-carboxylic acid, which was obtained in 7), instead of 4-(2-acetoxyethylthio)-3-fluorobenzoic acid to afford the desired compound (yield 64%) as a pale yellow oil.
Nuclear magnetic resonance spectrum (270MHz, CDCl₃) δppm:
   7.80-7.70(1H,br.s), 7.67(1H,dd,J=8Hz,2Hz),
   7.32(1H,d,J=8Hz),4.02(1H,br.t,J=6Hz),
   3.96(2H,s), 3.76(3H,s), 3.26(2H,t,J=6Hz),
   2.20(3H,s).

### 9) 5-(4-Acetyl-3,4-dihydro-2H-1,4-benzothiazin-6-yl)-2-(4-fluorophenyl)-4-methoxycarbonyl-3-(pyridin-4-yl)-1H-pyrrole

In a similar manner to that described in Example 1-6), a reaction was carried out using methyl (4-acetyl-3,4-dihydro-2H-1,4-benzothiazine-6-carbonyl)acetate, which was obtained in 8) instead of ethyl [4-(2-acetoxyethylthio)-3-fluoro]benzoylacetate to afford the desired compound (yield 18%) as a pale brown amorphous solid.
Nuclear magnetic resonance spectrum (270MHz, CDCl₃) δppm:
   8.97-8.74(1H,br.s), 8.51(2H,d,J=6Hz), 7.57-7.43(1H,br.s),
   7.40-6.92(8H,m), 3.99(2H,br.t,J=6Hz), 3.53(3H,s),
   3.26(2H,t,J=6Hz), 2.26(3H,s).

### 10) 2-(4-Fluorophenyl)-5-(3,4-dihydro-2H-1,4-benzothiazin-6-yl)-3-(pyridin-4-yl)-1H-pyrrole

In a similar manner to that described in Example 1-7), hydrolysis and decarboxylation were carried out using 5-(4-acetyl-3,4-dihydro-2H-1,4-benzothiazin-6-yl)-2-(4-fluorophenyl)-4-methoxycarbonyl-3-(pyridin-4-yl)-1H-pyrrole, which was obtained in 9), to afford the desired compound (yield 17%) as a brown powder.
Nuclear magnetic resonance spectrum (270MHz, CDCl₃) δppm:
   10.38-10.21(1H,br.s), 8.42(2H,d,J=6Hz),
   7.42(2H,dd,J=9Hz,5Hz), 7.22(2H,d,J=6Hz), 7.13-6.90(4H,m),
   6.82(1H,d,J=2Hz), 6.62(1H,d,J=3Hz), 4.46-4.20(1H,br.s),
   3.71-3.60(2H,m), 3.12-3.02(2H,m).

### 11) 2-(4-Fluorophenyl)-5-(3,4-dihydro-1-oxo-2H-1,4-benzothiazin-6-yl)-3-(pyridin-4-yl)-1H-pyrrole

In a similar manner to that described in Example 1-8), oxidation was carried out using 2-(4-fluorophenyl)-5-(3,4-dihydro-2H-1,4-benzothiazin-6-yl)-3-(4-pyridin-4-yl)-1H-pyrrole, which was obtained in 10), to give the title compound (yield 38%) as a slightly green powder.
Melting point: 265 - 267 °C
Nuclear magnetic resonance spectrum (270MHz, CDCl₃) δppm:
   10.20-10.12(1H,br.s), 8.40(2H,d,J=6Hz), 7.43(1H,d,J=8Hz),
   7.41(2H,dd,J=9Hz,6Hz), 7.23(2H,d,J=6Hz), 7.09(2H,t,J=9Hz),
   6.93(1H,dd,J=8Hz,2Hz), 6.84(1H,d,J=2Hz), 6.73(1H,s),
   3.89(1H,dt,J=14Hz,2Hz), 3.51(1H,dt,J=14Hz,4Hz), 3.14-
   3.09(1H,m), 2.70(1H,dt,J=14Hz,4Hz), 2.21-2.09(1H,br.s).

### [Example 4]

### 2-(4-Fluorophenyl)-5-(3,4-dihydro-1,1-dioxo-2H-1,4-benzothiazin-6-yl)-3-(pyridin-4-yl)-1H-pyrrole (Exemplification compound No. 5-176)

In a similar manner to that described in Example 2, an oxidation was carried out using the compound obtained in Example 3 to give the title compound (yield 62%) as a yellow powder.
Melting point: 283 - 286 °C
Nuclear magnetic resonance spectrum (270MHz, DMSO-d₆) δppm:
   11.87-11.71(1H,br.s), 8.41(2H,d,J=6Hz), 7.52(1H,d,J=8Hz),
   7.46(2H,dd,J=9Hz,5Hz), 7.28(2H,t,J=9Hz), 7.21(2H,d,t=6Hz),
   6.98-6.86(2H,m), 6.91(1H,d,J=3Hz), 3.81-3.69(2H,m), 3.44-
   3.32(2H,m).

### [Example 5]

### 2-(4-Fluorophenyl)-5-(3,4-dihydro-4-methyl-1-oxo-2H-1,4-benzothiazin-6-yl)-3-(pyridin-4-yl)-1H-pyrrole (Exemplification compound No. 5-37)

### 1) Methyl 3,4-dihydro-4-methyl-2H-1,4-benzothiazine-6-carboxylate

A mixture of methyl 3,4-dihydro-2H-1,4-benzothiazine-6-carboxylate (3.00 g, 14.34 mmol), which was obtained in Example 3-5), formic acid (88%, 1.98 ml, 43.02 mmol) and formalin (37%, 2.15 g, 28.68 mmol) was stirred at 70°C for 1 hour. After cooling to room temperature the reaction mixture was made basic with saturated aqueous sodium hydrogencarbonate and extracted with ethyl acetate. The organic layer was washed with water, dried over anhydrous magnesium sulfate and then concentrated under reduced pressure. The residue was purified by chromtography on a silica gel column using hexane/ethyl acetate = 4/1 as the eluant to give the desired compound (1.83 g yield, 50%) as a pale brown powder.
Nuclear magnetic resonance spectrum (270MHz, CDCl₃) δppm:
   7.34-7.28(2H,m), 7.08(1H,d,J=8Hz), 3.88(3H,s), 3.59-
   3.53(2H,m), 3.17-3.09(2H,m), 3.00(3H,s).

### 2) 3,4-Dihydro-4-methyl-2H-1,4-benzothiazine-6-carboxylic acid

In a similar manner to that described in Example 3-6), hydrolysis was carried out using methyl 3,4-dihydro-4-methyl-2H-1,4-benzothiazine-6-carboxylate obtained in 1) to afford the desired compound (yield 95%) as a white powder.
Nuclear magnetic resonance spectrum (270MHz, CDCl₃) δppm:
   7.41-7.34(2H,m), 7.11(1H,d,J=8Hz), 3.61-3.54(2H,m),
   3.18-3.12(2H,m), 3.01(3H,s).

### 3) Methyl (3,4-dihydro-4-methyl-2H-1,4-benzothiazine-6-carbonyl)acetate

In a similar manner to that described in Example 1-5), benzoylation was carried out using 3,4-dihydro-4-methyl-2H-1,4-benzothiazine-6-carboxylic acid to afford the desired compound (yield 98%) as a pale yellow oil.
Nuclear magnetic resonance spectrum (270MHz, CDCl₃) δppm:
   7.27-7.07(3H,m), 3.95(2H,s), 3.74(3H,s), 3.61-3.54(2H,m),
   3.18-3.09(2H,m), 3.01(3H,s).

### 4) 2-(4-Fluorophenyl)-5-(3,4-dihydro-4-methyl-2H-1,4-benzothiazin-6-yl)-4-methoxycarbonyl-3-(pyridin-4-yl)-1H-pyrrole

In a similar manner to that described in Example 1-6), cyclization was carried out using methyl (3,4-dihydro-4-methyl-2H-1,4-benzothiazine-6-carbonyl)acetate, which was obtained in 3), instead of ethyl [4-(2-acetoxyethylthio)-3-fluoro]benzoylacetate to afford the desired compound (yield 37%) as an orange powder.
Nuclear magnetic resonance spectrum (270MHz, CDCl₃) δppm:
   8.63-8.50(1H,br.s), 8.51(2H,d,J=6Hz), 7.23-6.80(9H,m),
   3.64-3.56(2H,m), 3.57(3H,s), 3.17-3.08(2H,m), 3.01(3H,s).

### 5) 2-(4-Fluorophenyl)-5-(3,4-dihydro-4-methyl-2H-1,4-benzothiazin-6-yl)-3-(pyridin-4-yl)-1H-pyrrole

In a similar manner to that described in Example 1-7a), hydrolysis and decarboxylation were carried out using 2-(4-fluorophenyl)-5-(3,4-dihydro-4-methyl-2H-1,4-benzothiazin-6-yl)-4-methoxycarbonyl-3-(pyridin-4-yl)-1H-pyrrole to afford the desired compound (yield 15%) as a yellow powder.
Nuclear magnetic resonance spectrum (270MHz, CDCl₃) δppm:
   8.57-8.45(1H,br.s), 8.45(2H,d,J=6Hz),
   7.38(2H,dd,J=9Hz,5Hz),7.24(2H,d,J=6Hz), 7.16-7.04(3H,m),
   6.85-6.77(2H,m), 6.67(1H,d,J=3Hz), 3.66-3.57(2H,m), 3.15-3.07(2H,m), 3.04(3H,s).

### 6) 2-(4-Fluorophenyl)-5-(3,4-dihydro-4-methyl-1-oxo-2H-1,4-benzothiazin-6-yl)-3-(pyridin-4-yl)-1H-pyrrole

In a similar manner to that described in Example 1-8), oxidation was carried out using 2-(4-fluorophenyl)-5-(3,4-dihydro-4-methyl-2H-1,4-benzothiazin-6-yl)-3-(pyridin-4-yl)-1H-pyrrole, which was obtained in 5), to afford the title compound (yield 57%) as a pale yellow powder.
Melting point: 238 - 240 °C
Nuclear magnetic resonance spectrum (270MHz, CDCl₃) δppm:
   10.99-10.84(1H,br.s), 8.44(2H,d,J=6Hz), 7.53(1H,d,J=8Hz),
   7.46(2H,dd,J=9Hz,5Hz), 7.23(2H,d,J=6Hz), 7.16-7.03(4H,m).
   6.81(1H,d,J=3Hz), 4.22-4.06(1H,m), 3.47(1H,dt,J=13Hz,4Hz),
   3.16-3.05(1H,m), 2.79(1H,dt,J=13Hz,4Hz).

### [Example 6]

### 2-(4-Fluorophenyl)-5-(3,4-dihydro-4-methyl-1,1-dioxo-2H-1,4-benzothiazin-6-yl)-3-(pyridin-4-yl)-1H-pyrrole (Exemplification compound No. 5-187)

In a similar manner to that described in Example 2, oxidation was carried out using the compound obtained in Example 5 to give the title compound (yield 33%) as pale yellow powder.
Melting point: 323 - 325 °C
Nuclear magnetic resonance spectrum (270MHz, CDCl₃) δppm:
   10.89-10.72(1H,br.s), 8.45(2H,d,J=6Hz), 7.76(1H,d,J=8Hz),
   7.45(2H,dd,J=9Hz,5Hz), 7.22(2H,d,J=6Hz),
   7.16(1H,dd,J=8Hz,2Hz), 7.09(2H,t,J=9Hz), 7.03(1H,d,J=2Hz), 6.82(1H,d,J=3Hz),
   4.99-4.91(2H,m), 3.42-3.33(2H,m),
   3.16(3H,s).

### [Example 7]

### 5-(4-Acetyl-3,4-dihydro-1-oxo-2H-1,4-benzothiazin-6-yl)-2-(4-fluorophenyl)-3-(pyridin-4-yl)-1H-pyrrole (Exemplification compound No. 5-44)

### 1) 5-(4-Acetyl-3,4-dihydro-2H-1,4-benzothiazin-6-yl)-2-(4-fluorophenyl)-3-(pyridin-4-yl)-1H-pyrrole

In a similar manner to that described in Example 3-7), acetylation was carried out using 2-(4-fluorophenyl)-5-(3,4-dihydro-2H-1,4-benzothiazin-6-yl)-3-(pyridin-4-yl)-1H-pyrrole, which was obtained in Example 3-10), to afford the desired compound (yield 88%) as a pale brown powder.
Nuclear magnetic resonance spectrum (270MHz, CDCl₃) δppm:
   8.87-8.63(1H,br.s), 7.44-7.21(7H,m), 7.09(2H,t,J=9Hz),
   6.71(1H,d,J=3Hz), 4.00(2H,br.t,J=6Hz), 3.25(2H,t,J=6Hz),
   2.24(3H,s).

### 2) 5-(4-Acetyl-3,4-dihydro-1-oxo-2H-1,4-benzothiazin-6-yl)-2-(4-fluorophenyl)-3-(pyridin-4-yl)-1H-pyrrole

In a similar manner to that described in Example 1-8), oxidation was carried out using 5-(4-acetyl-3,4-dihydro-2H-1,4-benzothiazin-6-yl)-2-(4-fluorophenyl)-3-(pyridin-4-yl)-1H-pyrrole, which was obtained in 1), to give the title compound (yield 70%) as a pale yellow powder.
Melting point: 251 - 253 °C
Nuclear magnetic resonance spectrum (270MHz, CDCl₃) δppm:
   11.35-11.13(1H,br.s), 8.45(2H,d,J=6Hz), 7.85-7.73(3H,m),
   7.47(2H,dd,J=9Hz,5Hz), 7.21(2H,d,J=6Hz), 7.10(2H,t,J=9Hz),
   6.88(1H,d,J=3Hz), 4.64-4.49(1H,m), 3.92-3.75(1H,m),
   3.96-3.81(1H,m), 3.22-3.09(1H,m), 2.35(3H,s).

### [Example 8]

### 2-(4-Fluorophenyl)-5-(1-oxothiochroman-6-yl)-3-(pyridin-4-yl)-1H-pyrrole (Exemplification compound No. 2-20)

### 1) 3-(4-Bromophenylthio)propionic acid

Sodium carbonate (18.50 g, 175 mmol) was added to a mixture of water (100 ml) and 3-bromopropionic acid (48.50 g, 317mmol) in small portions at room temperature with stirring. The mixture was further stirred for 30 minutes and then 4-bromobenzenethiol (50.00 g, 264 mmol) and aqueous sodium hydroxide solution (100 ml containing sodium hydroxide 12.70 g, 317 mmol) were added dropwise to the above reaction mixture. The mixture was stirred at 100°C for 2 hours. After cooling to room temperature the pH of the reaction mixture was adjusted to pH 7 using concentrated hydrochloric acid and extracted with diethyl ether. The pH of the aqueous layer was adjusted to 3 using concentrated hydrochloric acid and extracted with diethyl ether. The organic layer was washed with water, dried over anhydrous magnesium sulfate and concentrated under reduced pressure to afford the desired compound (68.45 g, yield 99%) as a white powder.
Nuclear magnetic resonance spectrum (270MHz, CDCl₃) δppm;
   11.50-10.20(1H,br.s), 7.43(2H,d,J=9Hz), 7.24(2H,d,J=9Hz),
   3.15(2H,t,J=7Hz), 2.67(2H,t,J=7Hz).

### 2) 6-Bromo-4-oxothiochroman

Concentrated sulfuric acid (135.2 g, 131 mmol) was added in small portions to a suspension of 3-(4-bromophenylthio)propionic acid (68.45 g, 262 mmol), which was obtained in 1), in dichloromethane (100 ml). The mixture was heated at reflux for 6 hours. After cooling to room temperature the reaction micture was partitioned between diethyl ether and water. The organic layer was washed successively with saturated aqueous sodium hydrogencarbonate solution and water, dried over anhydrous magnesium sulfate and then concentrated under reduced pressure to afford the desired compound (54.63 g, yield 86%) as an orange powder.
Nuclear magnetic resonance spectrum (270MHz, CDCl₃) δppm:
   8.23(1H,d,J=2Hz), 7.48(1H,dd,J=9Hz,2Hz), 7.16(1H,d,J=9Hz),
   3.25(1H,d,J=9Hz), 3.23(1H,d,J=7Hz), 2.99(1H,d,J=7Hz),
   2.97(1H,d,J=8Hz).

### 3) 6-Bromo-4-hydroxythiochroman

Sodium borohydride (934 mg, 24.7 mmol) was added to a solution of 6-bromo-4-oxothiochroman (20.00 g, 82.3 mmol), which was obtained in 2) in tetrahydrofuran (100 ml) in small portions under ice-cooling with stirring. The mixture was stirred at the same temperature for 30 minutes. At the end of this time methanol (20 ml) was added at the same temperature to the reaction mixture and the mixture was further stirred for 30 minutes. To the reaction mixture was added a saturated aqueous ammonium chloride solution and the mixture was concentrated under reduced pressure. To the residue was added saturated aqueous sodium hydrogencarbonate solution and extracted with ethyl acetate. The organic layer was washed with water, dried over anhydrous magnesium sulfate and concentrated under reduced pressure to afford the desired compound (20.16 g, quantitative yield) as a pale yellow powder.
Nuclear magnetic resonance spectrum (270MHz, CDCl₃) δppm:
   7.50(1H,d,J=2Hz), 7.27(1H,dd,J=8Hz,2Hz), 7.01(1H,d,J=8Hz),
   4.77(1H,dd,J=8Hz,5Hz), 3.28(1H,ddd,J=8Hz,8Hz,3Hz),
   2.90(1H,ddd,J=13Hz,6Hz,3Hz), 2.37-2.27(1H,m),
   2.07(1H,ddd,J=11Hz,9Hz,3Hz), 1.81-1.71(1H,br.s).

### 4) 6-Bromo-3-thiochromene

p-Toluensulfonic acid mono-hydrate (0.80 g) was added to a solution of 6-bromo-4-hydroxythiochroman (20.00g, 81.59 mmol), which was obtained in 3) in benzene (200 ml). The mixture was heated at reflux for 1 hour. After cooling to room temperature saturated aqueous sodium hydrogencarbonate solution was added to the reaction mixture. The organic layer was separated, washed with water, dried over anhydrous magnesium sulfate and concentrated under reduced pressure. The residue was purified by chromatography on a silica gel column using hexane/ethyl acetate = 50/1 as the eluant to afford the desired compound (9.13 g, yield 49%) as a pale yellow powder.
Nuclear magnetic resonance spectrum (270MHz, CDCl₃) δppm:
   7.20(1H,dd,J=8Hz,2Hz), 7.17(1H,t,J=2Hz), 7.06(1H,d,J=8Hz),
   6.41(1H,d,J=10Hz), 5.99(1H,dt,J=10Hz,5Hz),
   3.45(2H,dd,J=6Hz,2Hz).

### 5) 6-Bromothiochroman

Palladium on carbon (10%, 350 mg) was added to a solution of 6-bromo-3-thiochromene (7.10 g, 31.26 mmol) in ethyl acetate (30 ml). The mixture was stirred under hydrogen atmosphere at room temperature for 2 hours. The reaction mixture was filtered and the filtrate was concentrated under reduced pressure to afford the desired compound (7.00 g, yield 98%) as a pale brown powder.
Nuclear magnetic resonance spectrum (270MHz, CDCl₃) δppm:
   7.17-7.15(2H,m), 6.95(1H,d,J=8Hz), 3.03-2.99(2H,m),
   2.78(2H,dd,J=6Hz,4Hz), 2.16-2.04(2H,m).

### 6) Thiochroman-6-carboxylic acid

n-Butyllithium in hexane (1.59M solution, 20 ml, 31.80 mmol) was added dropwise at -78°C under nitrogen atmosphere to a solution of 6-bromothiochromane (7.00 g, 30.55 mmol) in tetrahydrofuran (30 ml). The mixture was stirred at the same temperature for 1 hour. The reaction mixture was poured into finely divided dry ice and stirred for 3 hours. The reaction mixture was concentrated under reduced pressure. To the residue, saturated aqueous sodium hydrogencarbonate solution was added and mixture was extracted with diethyl ether. The pH of the aqueous layer was adjusted to 2 using aqueous sulfuric acid solution (20%) and extracted with ethyl acetate. The organic layer was washed with water, dried over anhydrous magnesium sulfate and concentrated under reduced pressure. The residue was washed with a large amount of water to afford the desired compound (4.80 g, yield 81%) as a pale brown powder.
Nuclear magnetic resonance spectrum (270MHz, CDCl₃) δppm:
   7.79-7.76(2H,m), 7.18(1H,d,J=9Hz), 3.12-3.08(2H,m),
   2.70(2H,dd,J=6Hz,5Hz), 2.21-2.12(2H,m).

### 7) p-Nitrobenzyl (thiochroman-6-carbonyl)acetate (a mixture of tautomers of enol form)

In a similar manner to that described in Example 1-5), a reaction was carried out using thiochroman-6-carboxylic acid, which was obtained in 6), and magnesium mono-(p-nitrobenzyl) malonate instead of 4-(2-acetoxyethylthio)-3-fluorobenzoic acid and potassium mono-methyl malonate to afford the desired compound (quantitative yield) as a pale brown powder.
Nuclear magnetic resonance spectrum (270MHz, CDCl₃) δppm:
   12.33(0.1H,s), 8.20(2H,d,J=9Hz), 7.58-7.52(2H,m),
   7.48(2H,d,J=9Hz), 7.15(1H,d,J=9Hz), 5.70(0.1H,s),
   5.33(0.2H,s), 5.29(1.8H,s), 4.02(1.8H,s), 3.10-3.06(2H,m),
   2.84(2H,dd,J=6Hz,5Hz), 2.18-2.09(2H,m).

### 8) 2-(4-Fluorophenyl)-4-(p-nitrobenzyloxycarbonyl)-3-(pyridin-4-yl)-5-(thiochroman-6-yl)-1H-pyrrole

In a similar manner to that described in Example 1-6), a reaction was carried out using p-nitrobenzyl (thiochroman-6-carbonyl)acetate obtained in 7) instead of ethyl [4-(2-acetoxyethylthio)-3-fluoro]benzoylacetate to afford the desired compound (yield 20%) as a yellow powder.
Nuclear magnetic resonance spectrum (270MHz, CDCl₃) δppm:
   8.67(2H,d,J=5Hz), 8.58-8.55(1H,br.s), 8.23(2H,d,J=9Hz),
   7.41-7.36(5H,m), 7.30-7.24(3H,m), 7.17-7.09(3H,m),
   5.26(2H,s), 3.24-3.20(2H,m), 2.96-2.91(2H,m), 2.34-2.22(2H,m).

### 9) 2-(4-Fluorophenyl)-3-(pyridin-4-yl)-5-(thiochroman-6-yl)-1H-pyrrole

Palladium on carbon (10%, 290 mg) was added to a solution of 2-(4-fluoropbenyl)-4-(p-nitrobenzyloxycarbonyl)-3-(pyridin-4-yl)-5-(thiochroman-6-yl)-1H-pyrrole (2.90 g, 5.13 mmol) in tetrahydrofuran (15 ml). The mixture was stirred at 50°C under a hydrogen atomosphere for 2 hours. The reaction mixture was filtered and the filtrate was concentrated under reduced pressure to afford free carboxylic acid. A solution of the carboxylic acid in ethylene glycol diethyl ether (30 ml) was heated at reflux for 2 hours. After cooling to room temperature, water was added to the reaction mixture, and the resulting precipitate was collected by filtration and washed with a large amount of water. This was purified by chromatography on a silica gel column using hexane/ethyl acetate = 3/1 as the eluant to afford the desired compound (1.10 g, yield 56%) as a pale yellow powder.
Nuclear magnetic resonance spectrum (270MHz, CDCl₃) δppm:
   8.46(2H,d,J=6Hz), 7.38(2H,ddd,J=9Hz,5Hz,2Hz), 7.25-7.22(3H,m), 7.21(1H,d,J=2Hz), 7.13(1H,d,J=8Hz),
   7.08(2H,t,J=8Hz), 6.67(1H,d,J=3Hz), 3.10-3.04(2H,m),
   2.87(2H,dd,J=7Hz,6Hz), 2.20-2.13(2H,m).

### 10) 2-(4-Fluorophenyl)-5-(1-oxothiochroman-6-yl)-3-(pyridin-4-yl)-1H-pyrrole

In a similar manner to that described in Example 1-8), oxidation was carried out using 2-(4-fluorophenyl)-3-(pyridin-4-yl)-5-(thiochroman-6-yl)-1H-pyrrole, which was obtained in 9), to give the title compound (yield 67%) as a pale yellow powder.
Melting point: 254 - 261 °C
Nuclear magnetic resonance spectrum (270MHz, CDCl₃) δppm:
   9.43(1H,br.s), 8.47(2H,d,J=5Hz), 7.63(1H,d,J=8Hz),
   7.48(1H,dd,J=9Hz,2Hz), 7.42(2H,ddd,J=9Hz,5Hz,2Hz),
   7.39(1H,d,J=2Hz), 7.24(2H,d,J=5Hz), 7.10(2H,t,J=9Hz),
   6.79(1H,d,J=3Hz), 3.18-2.80(4H,m), 2.54-2.47(1H,m),
   2.09-2.01(1H,m).

### [Example 9]

### 5-(1,1-Dioxothiochroman-6-yl)-2-(4-fluorophenyl)-3-(pyridin-4-yl)-1H-pyrrole (exemplification compound No. 2-117)

In a similar manner to that described in Example 2, a reaction was carried out using the compound obtained in Example 8 to give the title compound (yield 49%).
Melting point: 304 - 311 °C
Nuclear magnetic resonance spectrum (270MHz, CDCl₃) δppm:
   11.23-11.15(1H,br.s), 8.45(2H,d,J=5Hz), 7.88(1H,d,J=8Hz),
   7.77(1H,dd,J=8Hz,2Hz), 7.60(1H,d,J=2Hz),
   7.50(2H,ddd,J=8Hz,5Hz,2Hz), 7.22(2H,d,J=6Hz),
   7.09(2H,t,J=8Hz), 6.84(1H,d,J=3Hz), 3.41-3.37(2H,m),
   3.07(2H,dd,J=7Hz,6Hz), 2.58-2.51(2H,m).

### [Example 10]

### 2-(4-Fluorophenyl)-5-(2,3-dihydro-1,1-dioxobenzo[b]thiophen-5-yl)-3-(pyridin-4-yl)-1H-pyrrole

### (Exemplification compound No. 1-117)

### 1) 1,1-dioxobenzo[b]thiophene-5-carboxylic acid

An aqueous solution of hydrogen peroxide (30%, 9.9 ml, 87.2 mmol) was added to a solution of benzo[b]thiophene-5-carboxylic acid (2.59 g, 14.5 mmol) in acetic acid (60 ml). The mixture was stirred at 120°C for 1 hour. After cooling to room temperature the reaction mixture was poured into a mixture of ice and water and neutralized using potassium carbonate. The pH of this mixture was adjusted to 2 using concentrated hydrochloric acid. The mixture was extracted with ethyl acetate. The organic layer was washed with aqueous sodium thiosulfate solution (10%) and with water, dried over anhydrous magnesium sulfate and then concentrated under reduced pressure to afford the desired compound (2.30 g, yield 90%) as a white powder.
Nuclear magnetic resonance spectrum (270MHz, CD₃OD) δppm:
   8.20(1H,d,J=8Hz), 8.08(1H,s), 7.79(1H,d,J=8Hz),
   7.49(1H,d,J=6Hz), 7.03(1H,d,J=7Hz).

### 2) 2,3-Dihydro-1,1-dioxobenzo[b]thiophene-5-carboxylic acid

Acetic acid (2 ml) and palladium on carbon (10%) were added to a solution of 1,1-dioxobenzo[b]thiophene-5-carboxylic acid (2.30 g, 12.9 mmol), which was obtained in 1), in tetrahydrofuran (20 ml). The mixture was stirred under hydrogen atmosphere at 50°C for 2 hours. The reaction mixture was filtered and the filtrate was concentrated under reduced pressure. The residue was washed with a small amount of diethyl ether to afford the desired compound (1.53 g, yield 56%) as a white powder.
Nuclear magnetic resonance spectrum (270MHz, CD₃OD) δppm:
   8.12-8.10(2H,m), 7.78-7.75(1H,m), 3.60-3.29(4H,m).

### 3) Methyl (2,3-dihydro-1,1-dioxobenzo[b]thiophene-5-carbonyl)acetate (a mixture of tautomers of enol form)

In a similar manner to that described in Example 1-5), a reaction was carried out using 2,3-dihydro-1,1-dioxobenzo[b]thiophene-5-carboxylic acid, which was obtained in 2), instead of 4-(2-acethoxyethylthio)-3-fluorobenzoic acid to afford the desired compound (yield 93%) as a white powder.
Nuclear magnetic resonance spectrum (270MHz, CDCl₃) δppm:
   12.50(0.4H,s), 8.02-7.78(3H,m), 5.74(0.4H,s), 4.04(1.2H,s),
   3.83(1.2H,s), 3.76(1.8H,s), 3.56-3.43(4H,m).

### 4) 2-(4-Fluorophenyl)-5-(2,3-dihydro-1,1-dioxobenzo[b]thiophen-5-yl)-4-methoxycarbony1-3-(pyridin-4-yl)-1H-pyrrole

In a similar manner to that described in Example 1-6), a reaction was carried out using methyl (2,3-dihydro-1,1-dioxobenzo[b]thiophene-5-carbonyl)acetate, which was obtained in 3), instead of ethyl [4-(2-acethoxyethylthio)-3-fluoro]benzoylacetate to afford the desired compound (yield 22%) as a white powder.
Nuclear magnetic resonance spectrum (270MHz, CDCl₃) δppm:
   9.52(1H,br.s), 8.49(2H,d,J=4Hz), 7.68-7.51(3H,m),
   7.27-6.96(6H,m), 3.54-3.40(7H,m).

### 5) 2-(4-Fluorophenyl)-5-(2,3-dihydro-1,1-dioxobenzo[bjthiophen-5-yl)-3-(pyridin-4-yl)-1H-pyrrole

In a similar manner to that described in Example 1-7a), hydrolysis and decarboxylation was carried out using 2-(4-fluorophenyl)-5-(2,3-dihydro-1,1-dioxobenzo[b]thiophen-5-yl)-4-methoxycarbonyl-3-(pyridin-4-yl)-1H-pyrrole obtained in 4) to afford the title compound (yield 96%) as a pale yellow powder.
Melting point: 283 - 287 °C
Nuclear magnetic resonance spectrum (270MHz, CDCl₃) δppm:
   8.70-8.69(1H,m), 8.50-8.47(2H,m), 7.76(1H,d,J=8Hz),
   7.62(1H,d,J=8Hz), 7.52(1H,s), 7.42-7.37(2H,m),
   7.35-7.22(2H,m), 7.11(2H,t,J=9Hz), 6.88(1H,d,J=3Hz),
   3.60-3.40(4H,m).

### [Example 11]

### 2-(4-Fluorophenyl)-5-(2,3-dihydro-1-oxobenzo[b]thiophen-5-yl)-3-(pyridin-4-yl)-1H-pyrrole

### (Exemplification compound No. 1-17)

### 1) 5-(2,3-Dihydrobenzo[b]thiophen-5-yl)-2-(4-fluorophenyl)-3-(pyridin-4-yl)-1H-pyrrole

A solution of the compound (1.74 g, 4.3 mmol) of Example 10 in anhydrous tetrahydrofuran (20 ml) was added dropwise to a suspension of lithium aluminum hydride (1.47 g, 38.7 mmol) in anhydrous tetrahydrofuran (100 ml). After the addition, the mixture was heated at reflux for 12 hours. After cooling to room temperature, the lithium aluminum hydride was quenched by addition of a small amount of water under ice-cooling. Ethanol (100 ml) was added to the mixture. The resulting mixture was filtered and the filtrate was concentrated under reduced pressure. The residue was purified by chromatography on a silica gel column using hexane/ethyl acetate = 2/1 as the eluant to afford the title compound (0.32 g, yield 20%) as a white powder.
Nuclear magnetic resonance spectrum (270MHz, CDCl₃) δppm:
   8.85(1H,br.s), 8.41(2H,d,J=5Hz), 7.40-7.22(7H,m),
   7.07(2H,t,J=9Hz), 6.67(1 H,d,J=3Hz), 3.44-3.28(4H,m).

### 2) 2-(4-Fluorophenyl)-5-(2,3-dihydro-1-oxobenzo[b]thiophen-5-yl)-3-(pyridin-4-yl)-1H-pyrrole

In a similar manner to that described in Example 1-8), oxidation was carried out using 5-(2,3-dihydrobenzo[b]thiophen-5-yl)-2-(4-fluorophenyl)-3-(pyridin-4-yl)-1H-pyrrole, which was obtained in 1), to afford the title compound (yield 51%) as a yellow powder.
Melting point: 235 - 240 °C
Nuclear magnetic resonance spectrum (270MHz, CD₃OD) δppm:
   8.34(2H,d,J=6Hz), 7.92-7.82(3H,m), 7.49-7.44(2H,m), 7.35-7.33(2H,m), 7.20-7.14(2H,m), 7.05(1H,s), 4.81(1H,br.s),
   3.57-3.28(4H,m).

### [Example 12]

### 5-(1,4-dioxothiochroman-6-yl)-2-(4-fluorophenyl)-3-(pyridin-4-yl)-1H-pyrrole

### (Exemplification compound No. 3-17)

### 1) 6-Bromo-4-oxothiochroman ethylene ketal

Under ice-cooling ethylenedioxybis(trimethylsilane) (13.4 g, 64.78 mmol) and trimethylsilyl triflate (catalytic amount) were added to a solution of 6-bromo-4-oxothiochroman (10.50 g, 43.18 mmol), which was obtained in 8-2), in anhydrous tetrahydrofuran (100 ml). The mixture was stirred at room temperature for 4 hours. Saturated aqueous sodium hydrogencarbonate solution was added to the reaction mixture and the mixture was extracted with ethyl acetate. The organic layer was washed with water, dried over anhydrous magnesium sulfate and then concentrated under reduced pressure. The residue was purified by chromatography on a silica gel column using hexane/ethyl acetate = 20/1 as the eluant to afford the desired compound (8.55 g, yield 69%) as a red powder.
Nuclear magnetic resonance spectrum (270MHz, CDCl₃) δppm:
   7.63(1H,d,J=2Hz), 7.27(1H,dd,J=8Hz,2Hz), 6.98(1H,d,J=8Hz),
   4.26-4.08(4H,m), 3.20-3.15(2H,m), 2.23-2.18(2H,m).

### 2) 6-Carboxy-4-oxothiochroman ethylene ketal

In a similar manner to that described in Example 8-6), a reaction was carried out using 6-bromo-4-oxothiochroman ethylene ketal, which was obtained in 1), instead of 6-bromothiochroman to afford the desired compound (yield 80%) as a pale brown powder.
Nuclear magnetic resonance spectrum (270MHz, CDCl₃) δppm:
   8.24(1H,d,J=2Hz), 7.86(1H,dd,J=8Hz,2Hz), 7.20(1H,d,J=8Hz),
   4.29-4.20(2H,m), 4.19-4.10(2H,m), 3.26-3.22(2H,m),
   2.28-2.23(2H,m).

### 3) Benzyl (4,4-ethylenedioxythiochroman-6-carbonyl)acetate (a mixture of tautomers of enol form)

In a similar manner to that described in Example 1-5), a reaction was carried out using 6-carboxy-4-oxothiochroman ethylene ketal, which was obtained in 2), instead of 4-(2-acetoxyethylthio)-3-fluorobenzoic acid and using magnesium mono-benzyl malonate instead of potassium mono-methyl malonate to afford the desired compound (quantitative yield) as a brown oil.
Nuclear magnetic resonance spectrum (270MHz, CDCl₃) δppm:
   12.47(0.1H,s), 8.10(1H,d,J=2Hz), 7.70(1H,dd,J=8Hz,2Hz),
   7.32(5H,s), 7.18(1H,d,J=8Hz), 5.68(0.1H,s), 5.24(0.2H,s),
   5.18(1.8H,s), 4.21-4.05(4H,m), 3.99(1.8H,s),
   3.26-3.20(2H,m), 2.25-2.20(2H,m).

### 4) 4-Benzyloxycarbonyl-2-(4-fluorophenyl)-5-(4-oxothiochroman-6-yl)-3-(pyridin-4-yl)-1H-pyrrole

In a similar manner to that described in Example 1-6), a reaction was carried out using benzyl (4,4-ethylenedioxythiochroman-6-carbonyl)acetate, which was obtained in 3) instead of ethyl [4-(2-acetoxyethylthio)-3-fluoro]benzoylacetate to afford the desired compound (yield 18%) as an amorphous yellowish brown solid.
Nuclear magnetic resonance spectrum (270MHz, CDCl₃) δppm:
   8.93(1H,br.s), 8.43(2H,d,J=6Hz), 8.27(1H,d,J=2Hz),
   7.68(1H,dd,J=8Hz,2Hz), 7.24-7.11(11H,m), 6.98(1H,d,J=8Hz),
   5.02(2H,s), 3 .25(2H,dd,J=7Hz,6Hz), 3.01-2.94(2H,m).

### 5) 4-Benzyloxycarbonyl-5-(4,4-ethylenedioxythiochroman-6-yl)-2-(4-fluorophenyl)-3-(pyridin-4-yl)-1H-pyrrole

In a similar manner to that described in 1), a reaction was carried out using 4-benzyloxycarbonyl-2-(4-fluorophenyl)-5-(4-oxothiochroman-6-yl)-3-(pyridin-4-yl)-1H-pyrrole, which was obtained in 4), instead of 6-bromo-4-oxothiochroman to afford the desired compound (quantitative yield) as an amorphous brown solid.
Nuclear magnetic resonance spectrum (270MHz, CDCl₃) δppm:
   8.65(1H,br.s), 8.43(2H,d,J=6Hz), 7.77(1H,d,J=2Hz),
   7.38(1H,dd,J=8Hz,2Hz), 7.25-7.10(9H,m), 6.97(2H,t,J=8Hz),
   6.94(1H,d,J=8Hz), 4.25-4.04(4H,m), 3.26-3.21(2H,m),
   2.36-2.23(2H,m).

### 6) 2-(4-Fluorophenyl)-5-(4-oxothiochroman-6-yl)-3-(pyridin-4-yl)-1H-pyrrole

Potassium hydroxide (19.80 g, 300 mmol) was added to a solution of 4-benzyloxycarbonyl-5-(4,4-ethylenedioxythiochroman-6-yl)-2-(4-fluorophenyl)-3-(pyridin-4-yl)-1H-pyrrole (2.0 g, 3.45 mmol) in ethanol (90%(v/v), 50 ml). The mixture was heated at reflux for 70 hours. The reaction mixture was concentrated under reduced pressure. Water was added to the residue and the mixture was extracted with ethyl acetate. The organic layer was washed with water and then concentrated under reduced pressure. To a solution of this residue in acetone (50 ml) was added hydrochloric acid (5N, 20 ml). This mixture was stirred at 50°C for 2 hours. After cooling to room temperature, the pH of the reaction mixture was adjusted to 8 using saturated aqueous sodium hydrogencarbonate solution and this was extracted with ethyl acetate. The organic layer was washed with water, dried over anhydrous magnesium sulfate and then concentrated under reduced pressure. The residue was purified by chromatography on a silica gel column using hexane/ethyl acetate = 3/2 as the eluant to afford the desired compound (280 mg, yield 20%) as a yellow powder.
Nuclear magnetic resonance spectrum (270MHz, CDCl₃) δppm:
   8.66(1H,br.s), 8.47(2H,d,J=6Hz), 8.25(1H,d,J=2Hz),
   7.63(1H,dd,J=8Hz,2Hz), 7.39(2H,dd,J=6Hz,3Hz),
   7.23(2H,d,J=6Hz), 7.10(2H,t,J=8Hz), 6.79(1H,d,J=3Hz),
   3.28(2H,dd,J=7Hz,6Hz), 3.00(2H,dd,J=7Hz,6Hz).

### 7) 5-(1,4-Dioxothiochroman-6-yl)-2-(4-fluorophenyl)-3-(pyridin-4-yl)-1H-pyrrole

In a similar manner to that described in Example 1-8), oxidation was carried out using 2-(4-fluorophenyl)-5-(4-oxothiochroman-6-yl)-3-(pyridin-4-yl)-1H-pyrrole, which was obtained in 6) to afford the title compound (yield 79%) as a yellow powder.
Melting point: 237 - 240 °C
Nuclear magnetic resonance spectrum (270MHz, DMSO-d₆) δppm:
   11.54(1H,br.s), 8.57(1H,d,J=2Hz), 8.45(2H,d,J=6Hz),
   8.10(1H,dd,J=8Hz,2Hz), 7.84(1H,d,J=8Hz),
   7.47(2H,dd,J=9Hz,5Hz), 7.22(2H,d,J=6Hz), 7.09(2H,t,J=9Hz),
   6.94(1H,d,J=3Hz), 3.65-3.57(1H,m), 3.55-3.41(2H,m),
   3.00-2.90(1H,m).

### [Example 13]

### 2-(4-Fluorophenyl)-5-(4-hydroxy-1-oxothiochroman-6-yl)-3-(pyridin-4-yl)-1H-pyrrole (Exemplification compound No. 2-33)

In a similar manner to that described in Example 8-3), reduction was carried out using the compound of Example 12 to give the title compound (yield 89%) as a pale yellow powder.
Melting point: 243 - 248 °C
Nuclear magnetic resonance spectrum (270MHz, DMSO-d₆) δppm:
   11.28(1H,br.s), 8.44(2H,d,J=6Hz), 8.12(1H,d,J=2Hz),
   7.81(1H,dd,J=8Hz,2Hz), 7.72(1H,d,J=8Hz),
   7.47(2H,dd,J=9Hz,5Hz), 7.23(2H,d,J=6Hz), 7.09(2H,t,J=9Hz),
   6.88(1H,d,J=3Hz), 5.16(1H,br.d,J=7Hz), 4.81-4.75(1H,m),
   3.34-3.17(2H,m), 3.15-3.09(2H,m).

### [Example 14]

### 5-(4,4-Dimethyl-1-oxothiochroman-6-yl)-2-(4-fluorophenyl)-3-(pyridin-4-yl)-1H-pyrrole (Exemplification compound No. 2-23)

### 1) 4-(4-Bromophenylthio)-2-methyl-2-butene

A solution of sodium hydroxide (2.68 g, 67.1 mmol) in water (80 ml) was added dropwise to a solution of 4-bromobenzenethiol (12.69 g, 67.10 mmol) and 4-bromo-2-methyl-2-butene (10.00 g, 67.10 mmol) in methanol (120 ml) under ice-cooling. The mixture was stirred at room temperature overnight. Water (300 ml) was added to the reaction mixture and this was extracted with dichloromethane. The organic layer was washed with water, dried over anhydrous magnesium sulfate and then concentrated under reduced pressure. The residue was purified by chromatography on a silica gel column using hexane as the eluant to afford the desired compound (15.06 g, yield 87%) as a colorless oil.
Nuclear magnetic resonance spectrum (270MHz, CDCl,) δppm:
   7.38(2H,d,J=9Hz), 7.19(2H,d,J=9Hz), 5.31-5.24(1H,m),
   3.51(2H,d,J=8Hz), 1.71(3H,s), 1.59(3H,s).

### 2) 6-Bromo-4,4-dimethylthiochroman

Polyphosphoric acid (20.00 g) was added to a solution of 4-(4-bromophenylthio)-2-methyl-2-butene (15.06 g, 58.55 mmol), which was obtained in 1), in toluene (36 ml). The mixture was stirred at 100°C overnight. Ethyl acetate and water were added to the reaction mixture. This mixture was stirred vigorously, and the organic layer was separated, washed successively with aqueous sodium hydroxide solution (1N) and with water, dried over anhydrous magnesium sulfate and ten concentrated under reduced pressure. The residue was purified by chromatography on a silica gel column using hexane as the eluant to afford the desired compound (9.55 g, yield 63%) as a white powder.
Nuclear magnetic resonance spectrum (270MHz, CDCl₃) δppm:
   7.45(1H,d,J=2Hz), 7.13(1H,dd,J=8Hz,2Hz), 6.95(1H,d,J=8Hz),
   3.04-2.99(2H,m), 1.95-1.91(2H,m), 1.31(6H,s).

### 3) 4,4-Dimethylthiochroman-6-carboxylic acid

In a similar manner to that described in Example 8-6), a reaction was carried out using 6-bromo-4,4-dimethylthiochroman, which was obtained in 2), instead of 6-bromothiochroman to afford the desired compound (yield 77%) as a white powder.
Nuclear magnetic resonance spectrum (270MHz, CDCl₃) δppm:
   8.10(1H,d,J=2Hz), 7.74(1H,dd,J=8Hz,2Hz), 7.17(1H,d,J=8Hz),
   3.10-3.05(2H,m), 2.00-1.95(2H,m), 1.37(6H,s).

### 4) p-Nitrobenzyl (4,4-dimethylthiochroman-6-carbonyl)acetate

In a similar manner to that described in Example 1-5), a reaction was carried out using 4,4-dimethylthiochroman-6-carboxylic acid, which was obtained in 3), and magnesium mono-(p-nitrobenzyl) malonate instead of 4-(2-acetoxyethylthio)-3-fluorobenzoic acid and potassium mono-methyl malonate to afford the desired compound (yield 97%) as a pale yellow powder.
Nuclear magnetic resonance spectrum (270MHz, CDCl₃) δppm:
   8.19(2H,d,J=8Hz), 7.96(1H,d,J=2Hz), 7.53(1H,dd,J=8Hz,2Hz),
   7.47(2H,d,J=8Hz), 7.16(1H,d,J=8Hz), 5.29(2H,s), 4.04(2H,s),
   3.09-3.05(2H,m), 1.98-1.93(2H,m), 1.33(6H,s).

### 5) 5-(4,4-Dimethylthiochroman-6-yl)-2-(4-fluorophenyl)-4-(p-nitrobenzyloxycarbonyl)-3-(pyridin-4-yl)-1H-pyrrole

In a similar manner to that described in Example 1-6), a reaction was carried out using p-nitrobenzyl (4,4-dimethylthiochroman-6-carbonyl)acetate, which was obtained in 4) instead of ethyl [4-(2-acetoxyethylthio)-3-fluoro]benzoylacetate to afford the desired compound (yield 19%) as a pale yellow powder.
Nuclear magnetic resonance spectrum (270MHz, CDCl₃) δppm:
   8.80(1H,br.s), 8.47(2H,d,J=6Hz), 8.06(2H,d,J=8Hz).
   7.58(1H,d,J=2Hz), 7.23(1H,dd,J=8Hz,2Hz), 7.22(2H,d,J=6Hz),
   7.14(2H,dd,J=9Hz,5Hz), 7.14(1H,d,J=8Hz), 6.98(2H,t,J=9Hz),
   6.92(2H,d,J=8Hz), 5.09(2H,s), 3.07-3.03(2H,m),
   1.95-1.91(2H,m), 1.28(6H,s).

### 6) 5-(4,4-Dimethylthiochroman-6-yl)-2-(4-fluorophenyl)-3-(pyridin-4-yl)-1H-pyrrole

In a similar manner to that described in Example 8-9), a reaction was carried out using 5-(4,4-dimethylthiochroman-6-yl)-2-(4-fluorophenyl)-4-(p-nitrobenzyloxycarbonyl)-3-(pyridin-4-yl)-1H-pyrrole, which was obtained in 5) instead of 2-(4-fluorophenyl)-4-(p-nitrobenzyloxycarbonyl)-3-(pyridin-4-yl)-5-(thiochroman-6-yl)-1H-pyrrole to afford the desired compound (yield 70%) as a pale yellow powder.
Nuclear magnetic resonance spectrum (270MHz, CDCl₃) δppm:
   8.47(1H,br.s), 8.45(2H,d,J=6Hz), 7.52(1H,d,J=2Hz),
   7.39(2H,dd,J=9Hz,5Hz), 7.24(2H,d,J=6Hz),
   7.22(1H,dd,J=8Hz,2Hz), 7.13(1H,d,J=8Hz), 7.09(2H,t,J=9Hz),
   6.67(1H,d,J=3Hz), 3.09-3.05(2H,m), 2.02-1.98(2H,m),
   1.39(6H,s).

### 7) 5-(4,4-Dimethyl-1-oxothiochroman-6-yl)-2-(4-fluorophenyl)-3-(pyridin-4-yl)-1H-pyrrole

In a similar manner to that described in Example 1-8), oxidation was carried out using 5-(4,4-dimethylthiochroman-6-yl)-2-(4-fluorophenyl)-3-(pyridin-4-yl)-1H-pyrrole, which was obtained in 6) to afford the title compound (yield 88%) as a pale yellow powder.
Melting point: 248 - 250 °C
Nuclear magnetic resonance spectrum (270MHz, CDCl₃) δppm:
   9.70(1H,br.s), 8.47(2H,d,J=6Hz), 7.58(1H,d,J=8Hz),
   7.56(1H,d,J=2Hz), 7.46(1H,dd,J=8Hz,2Hz),
   7.43(2H,dd,J=9Hz,5Hz), 7.25(2H,d,J=6Hz),
   7.10(2H,t,J=9Hz), 6.78(1H,d,J=3Hz), 3.09-2.94(2H,m),
   2.48-2.37(1H,m), 1.82-1.72(1H,m), 1.39(3H,s), 1.31(3H,s).

### [Example 15]

### 5-(4,4-Dimethyl-1,1-dioxothiochroman-6-yl)-2-(4-fluorophenyl)-3-(pyridin-4-yl)-1H-pyrrole (Exemplification compound No. 2-120)

In a similar manner to that described in Example 2, oxidation was carried out using the compound of Example 14 to afford the title compound (yield 70%) as a pale yellow powder.
Melting point: 285 - 287 °C
Nuclear magnetic resonance spectrum (270MHz, CDCl₃-DMSO-d₆) δppm:
   11.3(1H,br.s), 8.44(2H,d,J=5Hz), 7.86(1H,d,J=8Hz),
   7.81(1H,d,J=1Hz), 7.73( H,dd,J=8Hz,1Hz),
   7.47(2H,dd,J=9Hz,5Hz), 7.22(2H,d,J=5Hz), 7.10(2H,t,J=9Hz),
   6.86(1H,d,J=2Hz), 3.43-3.38(2H,m), 2.45-2.40(2H,m),
   1.49(6H,s).

### [Example 16]

### 2-(3,4-Difluorophenyl)-5-(1-oxothiocbroman-6-yl)-3-(pyridin-4-yl)-1H-pyrrole (Exemplification compound No. 2-78)

### 1) 2-(t-Butyldimethylsilyloxy)-3',4'-difluoro-2-(pyridin-4-yl)acetophenone

In a similar manner to that described in Example 1-1), a reaction was carried out using 3,4-difluoro-(N-methoxy-N-methyl)benzamide instead of 4-fluoro-(N-methoxy-N-methyl)benzamide to afford the desired compound (yield 72%) as a pale brown oil.
Nuclear magnetic resonance spectrum (270MHz, CDCl₃) δppm:
   8.61(2H,d,J=6Hz), 7.93-7.82(2H,m), 7.44(2H,d,J=6Hz),
   7.18-7.09(1H,m), 5.59(1H,s), 0.92(9H,s), 0.13(6H,s).

### 2) 2-(3,4-Difluorophenyl)-4-(p-nitrobenzyloxycarbonyl)-3-(pyridin-4-yl)-5-(thiochroman-6-yl)-1H-pyrrole

In a similar manner to that described in Example 1-6), a reaction was carried out using 2-(t-butyldimethylsilyloxy)-3',4'-difluoro-2-(pyridin-4-yl)acetophenone, which was obtained in 1), instead of 2-(t-butyldimethylsilyloxy)-4'-fluoro-2-(pyridin-4-yl)acetophenone, and using p-nitrobenzyl (thiochroman-6-carbonyl)acetate, which was obtained in Example 8-7), instead of ethyl [4-(2-acetoxyethylthio)-3-fluoro]benzoylacetate to afford the desired compound (yield 16%) as an amorphous yellow solid.
Nuclear magnetic resonance spectrum (270MHz, CDCl₃) δppm:
   8.88(1H,br.s), 8.55(2H,d,J=6Hz), 8.11(2H,d,J=9Hz),
   7.41-7.21(5H,m), 7.25(2H,d,J=6Hz), 7.00(2H,d,J=9Hz),
   6.92-6.87(1H,m), 4.85(2H,s), 3.11-3.07(2H,m),
   2.82-2.77(2H,m), 2.15-2.09(2H,m).

### 3) 2-(3,4-Difluorophenyl)-3-(pyridin-4-yl)-5-(thiochroman-6-yl)-1H-pyrrole

In a similar manner to that described in Example 8-9), a reaction was carried out using 2-(3,4-difluorophenyl)-4-(p-nitrobenzyloxycarbonyl)-3-(pyridin-4-yl)-5-(thiochroman-6-yl)-1H-pyrrole, which was obtained in 2), instead of 2-(4-fluorophenyl)-4-(p-nitrobenzyloxycarbonyl)-3-(pyridin-4-yl)-5-(thiochroman-6-yl)-1H-pyrrole to afford the desired compound (yield 45%) as a yellowish brown powder.
Nuclear magnetic resonance spectrum (270MHz, CDCl₃) δppm:
   8.51(1H,br.s), 8.48(2H,d,J=6Hz), 7.38-7.08(8H,m),
   6.65(1H,d,J=3Hz), 3.09-3.04(2H,m), 2.89-2.84(2H,m),
   2.20-2.11(2H,m).

### 4) 2-(3,4-Difluorophenyl)-5-(1-oxothiochroman-6-yl)-3-(pyridin-4-yl)-1H-pyrrole

In a similar manner to that described in Example 1-8), oxidation was carried out using 2-(3,4-difluorophenyl)-3-(pyridin-4-yl)-5-(thiochroman-6-yl)-1H-pyrrole, which was obtained in 3) to afford the title compound (yield 68%) as a yellow powder.
Melting point: 222 - 224 °C
Nuclear magnetic resonance spectrum (270MHz, CDCl₃-DMSO-d₆) δppm:
   10.30(1H,br.s), 8.49(2H,d,J=6Hz), 7.50-7.10(8H,m),
   6.74(1H,d,J=3Hz), 3.16-2.71(4H,m), 2.55-2.39(1H,m),
   2.07-1.97(1H,m).

### [Example 17]

### 2-(3,4-Difluorophenyl)-5-(1,1-dioxothiochroman-6-yl)-3-(pyridin-4-yl)-1H-pyrrole

### (Exemplification compound No. 2-175)

In a similar manner to that described in Example 2, an oxidation was carried out using the compound of Example 16 to give the title compound (yield 56%) as a pale yellow powder.
Melting point: 293 - 294°C
Nuclear magnetic resonance spectrum (270MHz, CDCl₃) δppm:
   11.2(1H,br.s), 8.48(2H,d,J=6Hz), 7.90(1H,d,J=8Hz),
   7.77(1H,dd,J=8Hz,1Hz), 7.60(1H,d,J=1Hz), 7.40-7.33(1H,m),
   7.23(2H,d,J=6Hz), 7.18-7.13(2H,m), 6.81(1H,d,J=3Hz),
   3.42-3.37(2H,m), 3.10-3.05(2H,m), 2.56-2.51(2H,m).

### [Example 18]

### 2-(3,4-Difluorophenyl)-5-(2,3-dihydro-1-oxobenzo[b]thiophen-5-yl)-3-(pyridin-4-yl)-1H-pyrrole

### (Exemplification compound No. 1-80)

### 1) 5-Bromo-2,3-dihydrobenzo[b]thiophene

Iron powder (0.11 g) was added to a solution of 2,3-dihydrobenzo[b]thiophene (4.04 g, 30.1 mmol) in dichloromethane (40 ml). Bromine (1.5 ml. 29.8 ml) was added dropwise to the mixture with stirring under ice-cooling. This mixture was stirred at the same temperature for 30 minutes. Saturated aqeous sodium hydrogencarbonate solution was added to the reaction mixture and this was extracted with dichloromethane. The organic layer was washed with water, dried over anhydrous magnesium sulfate and then concentrated under reduced pressure. The residue was purified by chromatography on a silica gel column using hexane as the eluant to afford the desired compound (3.28 g, yield 51%) as a yellow oil.
Nuclear magnetic resonance spectrum (270MHz, CDCl₃) δppm:
   7.29-7.20(2H,m), 7.05(1H,d,J=8Hz), 3.36-3.26(4H,m).

### 2) 2,3-Dihydrobenzo[b]thiophene-5-carboxylic acid

In a similar manner to that described in Example 8-6), a reaction was carried out using 5-bromo-2,3-dihydrobenzo[b]thiophene, which was obtained in 1), instead of 6-bromothiochroman to afford the desired compound (yield 70%) as a white powder.
Nuclear magnetic resonance spectrum (270MHz, CD₃OD) δppm:
   7.81(1H,s), 7.77(1H,d,J=8Hz), 7.23(1H,d,J=8Hz),
   3.39-3.31(4H,m).

### 3) Methyl (2,3-dihydrobenzo[b]thiophene-5-carbonyl)acetate (a mixture of tautomers of enol form)

In a similar manner to that described in Example 1-5), a reaction was carried out using 2,3-dihydrobenzo[b]thiophene-5-carboxylic acid, which was obtained in 2), instead of 4-(2-acetoxyethylthio)-3-fluorobenzoic acid to afford the desired compound (yield 76%) as a red oil.
Nuclear magnetic resonance spectrum (270MHz, CDCl₃) δppm:
   12.50(0.1H,s), 7.76(1H,s), 7.69(1H,d,J=8Hz),
   7.28(1H,d,J=8Hz), 5.61(0.1H,s), 3.95(1.8H,s),
   3.79-3.75(3H,m), 3.43-3.35(4H,m).

### 4) 2-(3,4-Difluorophenyl)-5-(2,3-dihydrobenzo[b]thiophen-5-yl)-4-methoxycarbonyl-3-(pyridin-4-yl)-1H-pyrrole

In a similar manner to that described in Example 1-6), a reaction was carried out using methyl (2,3-dihydrobenzo[b]thiophene-5-carbonyl)acetate, which was obtained in 3), instead of ethyl[4-(2-acetoxyethylthio)-3-fluoro]benzoylacetate, and using 2-(t-butyldimethylsilyloxy)-3',4'-difluoro-2-(pyridin-4-yl)acetophenone, which was obtained in Example 16-1), instead of 2-(t-butyldimethylsilyloxy)-4'-fluoro-2-(pyridin-4-yl)acetophenone to afford the desired compound (yield 21%) as a yellow powder.
Nuclear magnetic resonance spectrum (270MHz, CDCl₃) δppm:
   9.19(1H,s), 8.49(2H,m), 7.70-6.88(8H,m), 3.53-3.35(7H,m).

### 5) 2-(3,4-Difluorophenyl)-5-(2,3-dihydrobenzo[b]thiophen-5-yl)-3-(pyridin-4-yl)-1H-pyrrole

In a similar manner to that described in Example 1-7a), hydrolysis and decarboxylation were carried out using 2-(3,4-difluorophenyl)-5-(2,3-dihydrobenzo[b]thiophen-5-yl)-4-methoxycarbonyl-3-(pyridin-4-yl)-1H-1-pyrrole, which was obtained in 4), to afford the desired compound (yield 61%) as a yellow powder.
Nuclear magnetic resonance spectrum (270MHz, CDCl₃) δppm:
   8.66-8.49(3H,m), 7.54-7.13(8H,m), 6.66(1H,m),
   3.75-3.36(4H,m).

### 6) 2-(3,4-Difluorophenyl)-5-(2,3-dihydro-1-oxobenzo[b]thiophen-5-yl)-3-(pyridin-4-yl)-1H-pyrrole

In a similar manner to that described in Example 1-8), oxidation was carried out using 2-(3,4-difluorophenyl)-5-(2,3-dihydrobenzo[b]thiophen-5-yl)-3-(pyridin-4-yl)-1H-pyrrole to afford the title compound (yield 30%) as a yellow powder.
Melting point: 152 - 155 °C
   Nuclear magnetic resonance spectrum (270MHz, CD₃OD) δppm:
      8.39-8.36(2H,m), 7.99-7.81(3H,m), 7.47-7.21(5H,m), 7.03(1H,s), 4.58(1H,s), 3.91-3.83(1H,m), 3.58-3.30(3H,m).

### [Example 19]

### 5-(1-Oxothiochroman-6-yl)-3-(pyridin-4-yl)-2-(3,4,5-trifluorophenyl)-1H-pyrrole (Exemplification compopund No. 2-87)

### 1) 2-(t-Butyldimethylsilyloxy)-2-(pyridin-4-yl)-3',4',5'-trifluoroacetophenone

In a similar manner to that described in Example 1-1), a reaction was carried out using (N-methoxy-N-methyl)-3,4,5-trifluorobenzamide instead of 4-fluoro-(N-methoxy-N-methyl)benzamide to afford the desired compound (yield 98%) as an orange oil.
Nuclear magnetic resonance spectrum (270MHz, CDCl₃) δppm:
   8.62(2H,d,J=6Hz), 7.73(2H,t,J=6Hz), 7.40(2H,d,J=6Hz),
   5.58(1H,s), 1.92(9H,s), 0.11(6H,s).

### 2) 4-(p-Nitrobenzyloxycarbonyl)-3-(pyridin-4-yl)-5-(thiochroman-6-yl)-2-(3,4,5-trifluorophenyl)-1H-pyrrole

In a similar manner to that described in Example 8-8), cyclization was carried out using 2-(t-butyldimethylsilyloxy)-2-(pyridin-4-yl)-3',4',5'-trifluoroacetophenone, which was obtained in 1), instead of 2-(t-butyldimethylsilyloxy)-4'-fluoro-2-(pyridin-4-yl)acetophenone to afford the desired compound (yield 14%) as a amorphous yellow solid.
Nuclear magnetic resonance spectrum (270MHz, CDCl₃) δppm:
   9.13(1H,br.s), 8.74(2H,d,J=6Hz), 8.30(2H,d,J=9Hz),
   7.48-7.41(4H,m), 7.33(1H,d,J=8Hz), 7.19(2H,d,J=9Hz),
   6.98(2H,t,J=6Hz), 5.29(2H,s), 3.31-3.19(2H,m),
   3.02-2.92(2H,m), 2.38-2.26(2H,m).

### 3) 3-(Pyridin-4-yl)-5-(thiochroman-6-yl)-2-(3,4,5-trifluorophenyl)-1H-pyrrole

In a similar manner to that described in Example 8-9), a reaction was carried out using 4-(p-nitrobenzyloxycarbonyl)-3-(pyridin-4-yl)-5-(thiochroman-6-yl)-2-(3,4,5-trifluorophenyl)-1H-pyrrole, which was obtained in 2), instead of 2-(4-fluorophenyl)-4-(p-nitrobenzyloxycarbonyl)-3-(pyridin-4-yl)-5-(thiochroman-6-yl)-1H-pyrrole to afford the desired compound (yield 81%) as a brown powder.
Nuclear magnetic resonance spectrum (270MHz, CDCl₃) δppm:
   8.65(1H,br.s), 8.52(2H,d,J=6Hz), 7.26(2H,d,J=6Hz),
   7.30-7.22(4H,m), 7.15(1H,d,J=8Hz), 7.00(2H,t,J=6Hz),
   6.65(1H,d,J=3Hz), 3.19-3.00(2H,m), 2.91-2.80(2H,m),
   2.20-2.11(2H,m).

### 4) 5-(1-Oxothiochroman-6-yl)-3-(pyridin-4-yl)-2-(3,4,5-trifluorophenyl)-1H-pyrrole

In a similar manner to that described in Example 1-8), oxidation was carried out using 3-(pyridin-4-yl)-5-(thiochroman-6-yl)-2-(3,4,5-trifluorophenyl)-1H-pyrrole, which was obtained in 3), to give the title compound (yield 43%) as a pale yellow powder.
Melting point: >290 °C
Nuclear magnetic resonance spectrum (270MHz, CDCl₃-DMSO-d₆) δppm:
   11.4(1H,br.s), 8.51(2H,d,J=6Hz), 7.72(2H,m), 7.63(1H,br.s),
   7.26(2H,d,J=6Hz), 7.14(2H,t,J=6Hz), 6.77(2H,d,J=3Hz),
   3.25-2.85(2H,m), 2.80-2.52(2H,m), 2.34-2.02(2H,m).

### [Example 20]

### 5-(1,1-Dioxothiochroman-6-yl)-3-(pyridin-4-yl)-2-(3,4,5-trifluorophenyl)-1H-pyrrole

### (Exemplification compound No. 2-184)

In a similar manner to that described in Example 1-8), oxidation was carried out using the compound of Example 19-3) and m-chloroperbenzoic acid (70%, two equivalents to the compound of Example 19-3)) to give the title compound (yield 33%) as a yellow powder.
Melting point: 243 - 251 °C (dec)
Nuclear magnetic resonance spectrum (270MHz, CDCl₃-DMSO-d₆) δppm:
   11.3(1H,br.s), 8.50(2H,d,J=6Hz), 7.88(1H,d,J=8Hz),
   7.78(1H,d,J=8Hz), 7.61(1H,br.s), 7.24(2H,d,t=6Hz),
   7.12(2H,t,J=6Hz), 6.78(1H,d,J=3Hz), 3.45-3.33(2H,m),
   3.18-3.00(2H,m), 2.62-2.48(2H,m).

### [Example 21]

### 5-(3,3-Dimethyl-1-oxothiochroman-6-yl)-2-(4-fluorophenyl)-3-(pyridin-4-yl)-1H-pyrrole (Exemplification compound No.2-24)

### 1) 3-(4-Bromophenylthio)-2,2-dimethylpropionic acid

In a similar manner to that described in Example 8-1), a reaction was carried out using 3-chloro-2,2-dimethylpropionic acid instead of 3-bromopropionic acid to afford the desired compound (yield 72%) as a colorless oil.
Nuclear magnetic resonance spectrum (270MHz, CDCl₃) δppm:
   7.37(2H,d,J=9Hz), 7.25(2H,d,J=9Hz), 3.16(2H,s), 1.31(6H,s).

### 2) 6-Bromo-3,3-dimethyl-4-oxothiochroman

In a similar manner to that described in Example 8-2), a reaction was carried out using 3-(4-bromophenylthio)-2,2-dimethylpropionic acid obtained in 1) instead of 3-(4-bromophenylthio)propionic acid to afford the desired compound (yield 93%) as a colorless oil.
Nuclear magnetic resonance spectrum (270MHz, CDCl₃) δppm:
   8.21(1H,m), 7.47-7.40(1H,m), 7.11(1H,d,J=9Hz), 3.08(2H,s),
   1.32(6H,s).

### 3) 6-Bromo-3,3-dimethyl-4-hydroxythiochroman

In a similar manner to that described in Example 8-3), a reaction was carried out using 6-bromo-3,3-dimethyl-4-oxothiochroman obtained 2) instead of 6-bromo-4-oxothiochroman to afford the desired compound (yield 93%) as a white powder.
Nuclear magnetic resonance spectrum (270MHz, CDCl₃) δppm:
   7.44(1H,m), 7.28-7.26(1H,m), 7.01(1H,d,J=9Hz), 4.14(1H,s),
   3.22-2.52(2H,m), 1.18(3H,s), 0.99(3H,s).

### 4) 6-Bromo-3,3-dimethylthiochroman

A solution of triethylsilane (3.2 ml, 20 mmol) in dichloromethane (10 ml) was added to a solution of 6-bromo-3,3-dimethyl-4-hydroxythiochroman (0.55 g, 2 mmol), which was obtained in 3), in dichloromethane (20 ml). To the mixture was added dropwise trifluoroacetic acid (3.1 ml, 40 mmol) at room temperature with stirring and this was stirred at room temperature for 30 minutes. The reaction mixture was poured into a mixture of ice and water. To this mixture, saturated aqueous sodium hydrogencarbonate solution was added and then it was extracted with chloroform. The organic layer was washed with water and dried over magenesium sulfate and then concentrated under reduced pressure to afford the desired compound (0.51 g, quantitative yield) as a colorless oil.
Nuclear magnetic resonance spectrum (270MHz, CDCl₃) δppm:
   7.24-7.13(2H,m), 6.97(1H,d,J=8Hz), 2.73(2H,s), 2.52(2H,s),
   1.09(6H,s).

### 5) 3,3-Dimethylthiochroman-6-carboxylic acid

In a similar manner to that described in Example 8-6), a reaction was carried out using 6-bromo-3,3-dimethylthiochroman, which was obtained in 4), instead of 6-bromothiochroman to afford the desired compound (yield 55%) as a white powder.
Nuclear magnetic resonance spectrum (270MHz, CDCl₃) δppm:
   7.78-7.73(2H,m), 7.18(1H,d,J=8Hz), 2.80(2H,s), 2.63(2H,s),
   1.12(6H,s).

### 6) Methyl (3,3-dimethylthiochroman-6-carbonyl)acetate

In a similar manner to that described in Example 1-5), a reaction was carried out using 3,3-dimethylthiochroman-6-carboxylic acid, which was obtained in 5), instead of 4-(2-acetoxyethylthio)-3-fluorobenzoic acid to afford the desired compound (yield 93%) as an orange oil.
Nuclear magnetic resonance spectrum (270MHz, CDCl₃) δppm:
   7.81-7.58(2H,m), 7.46-7.17(1H,m), 3.94(2H,s),
   3.79-3.75(3H,m), 2.80(2H,s), 2.62(2H,s), 1.11(6H,s).

### 7) 5-(3,3-Dimethylthiochroman-6-yl)-2-(4-fluorophenyl)-4-methoxycarbonyl-3-(pyridin-4-yl)-1H-pyrrole

In a similar manner to that described in Example 1-6), cyclization was carried out using methyl (3,3-dimethylthiochroman-6-carbonyl)acetate, which was obtained in 6), instead of methyl [4-(2-acetoxyethylthio)-3-fluoro]benzoylacetate to afford the desired compound (yield 18%) as ang amorphous yellow solid.
Nuclear magnetic resonance spectrum (270MHz, CDCl₃) δppm:
   9.49(1H,br.s), 8.43-8.41(2H,m), 7.42-6.91(9H,m),
   3.53(3H,s), 2.78(2H,s), 2.59(2H,s), 1.12(6H,s).

### 8) 5-(3,3-Dimethylthiochroman-6-yl)-2-(4-fluorophenyl)-3-(pyridin-4-yl)-1H-pyrrole

In a similar manner to that described in Example 1-7a), hydrolysis and decarboxylation were carried out using 5-(3,3-dimethylthiochroman-6-yl)-2-(4-fluorophenyl)-4-methoxycarbonyl-3-(pyridin-4-yl)-1H-pyrrole, which was obtained in 7), to afford the desired compound (yield 39%) as a brown powder.
Nuclear magnetic resonance spectrum (270MHz, CDCl₃) δppm:
   8.48-8.44(3H,m), 7.46-7.05(9H,m), 6.67(1H,d,J=3Hz),
   2.79(2H,s), 2.61(2H,s), 1.14(6H,s).

### 9) 5-(3,3-Dimethyl-1-oxothiochroman-6-yl)-2-(4-fluorophenyl)-3-(pyridin-4-yl)-1H-pyrrole

In a similar manner to that described in Example 1-8), oxidation was carried out using 5-(3,3-dimethylthiochroman-6-yl)-2-(4-fluorophenyl)-3-(pyridin-4-yl)-1H-pyrrole, which was obtained in 8), to afford the title compound (yield 25%) as a yellow powder.
Melting point: 138 - 142 °C
Nuclear magnetic resonance spectrum (270MHz, CDCl₃) δppm:
   10.13(1H,br.s), 7.67-7.62(2H,m), 7.46-7.08(9H,m),
   6.79(1H,m), 3.12-2.55(4H,m), 1.13(3H,s), 1.11(3H,s).

### [Example 22]

### 5-(3,3-Dimethyl-1,1-dioxothiochroman-6-yl)-2-(4-fluorophenyl)-3-(pyridin-4-yl)-1H-pyrrole (Exemplification compound No. 2-121)

By-products obtained in Example 25-9) were purified by chromatography on a silica gel column using hexane/ethyl acetate = 1/2 as the eluant to give the title compound (yield 36%) as a white powder.
Melting point: 230 - 237 °C
Nuclear magnetic resonance spectrum (270MHz, CDCl₃) δppm:
   9.09(1H,br.s), 8.46(2H,d,J=5Hz), 7.87(1H,d,J=8Hz),
   7.60-7.56(1H,m), 7.43-7.38(3H,m), 7.23(2H,d,J=6Hz),
   7.10(2H,t,J=9Hz), 6.85(1H,d,J=3Hz), 3.20(2H,s), 2.87(2H,s),
   1.24(6H,s).

### [Example 23]

### 2-(3-Fluorophenyl)-5-(1-oxothiochroman-6-yl)-3-(pyridin-4-yl)-1H-pyrrole

### (Exemplification compound No. 2-42)

### 1) 2-(t-Butyldimethylsilyloxy)-3'-fluoro-2-(pyridin-4-yl)acetophenone

In a similar manner to that described in Example 1-1), a reaction was carried out using 3-fluoro-(N-methoxy-N-methyl)benzamide instead of 4-fluoro-(N-methoxy-N-methyl)benzamide to afford the desired compound (yield 98%) as an orange oil
Nuclear magnetic resonance spectrum (270MHz, CDCl₃) δppm:
   8.58(2H,d,J=6Hz), 7.80-7.75(1H,m), 7.72-7.65(1H,m),
   7.44(1H,d,J=6Hz), 7.38-7.29(1H,m), 7.24-7.16(1H,m),
   5.62(1H,s), 0.88(9H,s), 0.10(6H,s).

### 2) 2-(3-Fluorophenyl)-4-(p-nitrobenzyloxycarbonyl)-3-(pyridin-4-yl)-5-(thiochroman-6-yl)-1H-pyrrole

In a similar manner to that described in Example 8-8), a cyclization was carried out using 2-(t-butyldimethylsilyloxy)-3'-fluoro-2-(pyridin-4-yl)acetophenone, which was obtained in 1), instead of 2-(t-butyldimethylsilyl)-4'-fluoro-2-(pyridin-4-yl)acetophenone to afford the desired compound (yield 22%) as a yellow powder.
Nuclear magnetic resonance spectrum (270MHz, CDCl₃) δppm:
   9.10(1H,br.s), 8.48(2H,d,J=6Hz), 8.07(2H,d,J=9Hz),
   7.80-7.68(3H,m), 7.73(2H,d,J=6Hz), 7.12(2H,d,J=8Hz),
   6.97(2H,d,J=9Hz), 6.91(2H,d,J=8Hz), 5.10(2H,s),
   3.09-3.01(2H,m), 2.82-2.80(2H,m), 2.16-2.02(2H,m).

### 3) 2-(3-Fluorophenyl)-3-(pyridin-4-yl)-5-(thiochroman-6-yl)-1H-pyrrole

In a similar manner to that described in Example 8-9), a reaction was carried out using 2-(3-fluorophenyl)-4-(p-nitrobenzyloxycarbonyl)-3-(pyridin-4-yl)-5-(thiochroman-6-yl)-1H-pyrrole, which was obtained in 2), instead of 2-(4-fluorophenyl)-4-(p-nitrobenzyloxycarbonyl)-3-(pyridin-4-yl)-5-(thiochroman-6-yl)-1H-pyrrole to afford the desired compound (yield 99%) as a brown powder.
Nuclear magnetic resonance spectrum (270MHz, CDCl₃) δppm:
   8.52(1H,br.s), 8.48(2H,d,J=6Hz), 7.40-7.21(3H,m),
   7.25(2H,d,J=6Hz), 7.23-6.93(4H,m), 6.68(1H,d,J=3Hz),
   3.10-3.01(2H,m), 2.91-2.82(2H,m), 2.20-2.11(2H,m).

### 4) 2-(3-Fluorophenyl)-5-(1-oxothiochroman-6-yl)-3-(pyridin-4-yl)-1H-pyrrole

In a similar manner to that described in Example 1-8), oxidation was carried out using 2-(3-fluorophenyl)-3-(pyridin-4-yl)-5-(thiochroman-6-yl)-1H-pyrrole, which was obtained in 3), to afford the title compound (yield 42%) as a pale yellow powder.
Melting point: 254 - 257 °C
Nuclear magnetic resonance spectrum (270MHz, CDCl₃-DMSO-d₆) δppm:
   10.90(1H,br.s), 8.48(2H,d,J=6Hz), 7.68(1H,d,J=8Hz),
   7.63(1H,d,J=8Hz), 7.56(1H,br.s), 7.36-7.19(3H,m),
   7.27(2H,d,J=6Hz), 7.05-6.98(1H,m), 6.78(1H,d,J=3Hz),
   3.20-3.00(2H,m), 2.98-2.79(2H,m), 2.64-2.47(1H,m),
   2.17-1.99(1H,m).

### [Example 24]

### 5-(1,1-Dioxothiochroman-6-yl)-2-(3-fluorophenyl)-3-(pyridin-4-yl)-1H-pyrrole

### (Exemplification compound No. 2-139)

In a similar manner to that described in Example 1-8), oxidation was carried out using the compound of Example 23-3) and m-chloroperbenzoic acid (70%, two equivalents to the compound of Example 23-3)) to give the title compound (yield 27%) as a pale yellow powder.
Melting point: 274 °C (decomposition)
Nuclear magnetic resonance spectrum (270MHz, CDCl₃-DMSO-d₆) δppm:
   11.20(1H,br.s), 8.47(2H,d,J=6Hz), 7.88(1H,d,J=8Hz),
   7.78(1H,d,J=8Hz), 7.62(1H,br.s), 7.38-7.29(1H,m),
   7.26-7.20(2H,m), 7.26(2H,d,J=6Hz), 7.08-6.99(1H,m),
   6.82(1H,d,J=3Hz), 3.44-3.30(2H,m), 3.11-3.01(2H,m),
   2.63-2.48(2H,m).

### [Example 25]

### 2-(3-Chlorophenyl)-5-(1-oxothiochroman-6-yl)-3-(pyridin-4-yl)-1H-pyrrole

### (Exemplification compound No. 2-57)

### 1) 2-(t-Butyldimethylsilyloxy)-3'-chloro-2-(pyridin-4-yl)acetophenone

In a similar manner to that described in Example 1-1), a reaction was carried out using 3-chloro-(N-methoxy-N-methyl)benzamide instead of 4-fluoro-(N-methoxy-N-methyl)benzamide to afford the desired compound (yield 84%) as an orange oil.
Nuclear magnetic resonance spectrum (270MHz, CDCl₃) δppm:
   8.60(2H,d,J=6Hz), 7.98(1H,d,J=2Hz), 7.86(1H,d,J=8Hz),
   7.43(2H,d,J=6Hz), 7.33-7.21(2H,m), 5.61(1H,s), 0.90(9H,s),
   0.11(6H,s).

### 2) Methyl (thiochroman-6-carbonyl)acetate

In a similar manner to that described in Example 8-7), a reaction was carried out using potassium mono-methyl malonate instead of magnesium mono-(p-nitrobenzyl)malonate to afford the desired product (quantitative yield) as a brown oil.
Nuclear magnetic resonance spectrum (270MHz, CDCl₃) δppm:
   7.62-7.58(2H,m), 7.17(1H,d,J=8Hz), 3.93(2H,s), 3.75(3H,s),
   3.12-3.02(2H,m), 2.92-2.83(2H,m), 2.21-2.09(2H,m).

### 3) 2-(3-Chlorophenyl)-4-methoxycarbonyl-3-(pyridin-4-yl)-5-(thiochroman-6-yl)-1H-pyrrole

In a similar manner to that described in Example 1-6), cyclization was carried out using 2-(t-butyldimethylsilyloxy)-3'-chloro-2-(pyridin-4-yl)acetophenone, which was obtained in 1), and methyl (thiochroman-6-carbonyl)acetate, which was obtained in 2), to afford the desired compound (yield 28%) as a pale yellow oil.
Nuclear magnetic resonance spectrum (270MHz, CDCl₃) δppm:
   8.89(1H,br.s), 8.58(2H,d,J=6Hz), 7.58-7.51(2H,m),
   7.43-7.34(3H,m), 7.24(2H,d,J=6Hz),7.20-7.13(2H,m),
   3.56(3H,s), 3.11-3.01(2H,m), 2.91-2.83(2H,m),
   2.18-2.08(2H,m).

### 4) 2-(3-Chlorophenyl)-3-(pyridin-4-yl)-5-(thiochroman-6-yl)-1H-pyrrole

In a similar manner to that described in Example 1-7a), hydrolysis and decarboxylation were carried out using 2-(3-chlorophenyl)-4-methoxycarbonyl-3-(pyridin-4-yl)-5-(thiochroman-6-yl)-1H-pyrrole, which was obtained in 3), to afford the desired compound (yield 43%) as a pale yellow powder.
Nuclear magnetic resonance spectrum (270MHz, CDCl₃) δppm:
   8.60(1H,br.s), 8.48(2H,d,J=6Hz), 7.48-7.43(1H,m),
   7.42-7.40(1H,m), 7.38-7.34(1H,m),7.31-7.20(5H,m),
   7.14(1H,d,J=9Hz), 6.65(1H,d,J=3Hz), 3.11-3.02(2H,m),
   2.92-2.82(2H,m), 2.21-2.09(2H,m).

### 5) 2-(3-Chlorophenyl)-5-(1-oxothiochroman-6-yl)-3-(pyridin-4-yl)-1H-pyrrole

In a similar manner to that described in Example 1-8), oxidation was carried out using 2-(3-chlorophenyl)-3-(pyridin-4-yl)-5-(thiochroman-6-yl)-1H-pyrrole, which was obtained in 4) to give the title compound (yield 74%) as a pale yellow powder.
Melting point: 260 - 263 °C
Nuclear magnetic resonance spectrum (270MHz, CDCl₃) δppm:
   10.40(1H,br.s), 8.59(2H,d,J=6Hz), 7.82-7.57(3H,m),
   7.48(1H,br.s), 7.43-7.17(5H,m), 6.84(1H,d,J=3Hz),
   3.32-2.83(4H,m), 2.68-2.45(1H,m), 2.20-2.02(1H,m).

### [Example 26]

### 2-(3-Chlorophenyl)-5-(1,1-dioxothiochroman-6-yl)-3-(pyridin-4-yl)-1H-pyrrole

### (Exemplification compound No. 2-154)

In a similar manner to that described in Example 1-8), oxidation was carried out using the compound of Example 25-4) and m-chloroperbenzoic acid (70%, two equivalents to the compound of Example 25-4)) to afford the title compound (yield 40%) as a pale yellow powder.
Melting point: 271 - 273 °C
Nuclear magnetic resonance spectrum (270MHz, CDCl₃-DMSO-d₆) δppm:
   11.30(1H,br.s), 7.93(1H,d,J=8Hz), 7.82(1H,d,J=8Hz),
   7.68(1H,br.s), 7.61(1H,br.s), 7.39-7.21(5H,m),
   6.88(1H,d,J=3Hz), 3.50-3.31(2H,m), 3.1 9-2.98(2H,m),
   2.68-2.44(2H,m).

### [Example 27]

### 2-(4-Fluorophenyl)-5-(2-methyl-1-oxothiochroman-6-yl)-3-(pyridin-4-yl)-1H-pyrrole

### (Exemplification compound No. 2-21)

### 1) 3-(4-Bromophenylthio)butanoic acid

In a similar manner to that described in Example 8-1), a reaction was carried out using 3-chlorobutanoic acid instead of 3-bromopropionic acid to afford the desired compound (yield 67%) as a colorless oil.
Nuclear magnetic resonance spectrum (270MHz, CDCl₃) δppm:
   7.44(2H,d,J=9Hz), 7.31(2H,d,J=9Hz), 3.65-3.44(1H,m),
   2.70-2.44(2H,m), 1.36(3H,d,J=7Hz).

### 2) 6-Bromo-2-methyl-4-oxothiochroman

In a similar manner to that described in Example 8-2), a reaction was carried out using 3-(4-bromophenylthio)butanoic acid, which was obtained in 1), instead of 3-(4-bromophenylthio)propionic acid to afford the desired compound (yield 60%) as a purple oil.
Nuclear magnetic resonance spectrum (270MHz, CDCl₃) δppm:
   8.21(1H,m), 7.50-7.47(1H,m), 7.14(1H,d,J=8Hz),
   3.66-3.60(1H,m), 3.06-2.70(2H,m), 1.44(3H,d,J=7Hz).

### 3) 6-Bromo-2-methyl-4-hydroxythiochroman

In a similar manner to that described in Example 8-3), a reaction was carried out using 6-bromo-2-methyl-4-oxothiochroman, which was obtained in 2), instead of 6-bromo-4-oxothiochroman to afford the desired compound (yield 92%) as a white powder.
Nuclear magnetic resonance spectrum (270MHz, CDCl₃) δppm:
   7.73(1H,m), 7.25-7.21(1H,m), 6.94(1H,d,J=9Hz),
   4.80-4.75(1H,m), 3.54-3.36(1H,m), 2.47-2.43(1H,m),
   1.86-1.73(1H,m), 1.37(3H,d,J=7Hz).

### 4) 6-Bromo-2-methylthiochroman

In a similar manner to that described in Example 21-4), a reaction was carried out using 6-bromo-2-methyl-4-hydroxythiochroman, which was obtained in 3), instead of 6-bromo-3,3-dimethyl-4-hydroxythiochroman to afford the desired compound (yield 74%) as a yellow powder.
Nuclear magnetic resonance spectrum (270MHz, CDCl₃) δppm:
   7.18-7.15(2H,m), 6.93(1H,d,J=8Hz), 3.43-3.31(1H,m),
   2.85-2.81(2H,m), 2.22-2.12(1H,m), 1.79-1.61(1H,m),
   1.36(3H,d,J=7Hz).

### 5) 2-Methylthiochroman-6-carboxylic acid

In a similar manner to that described in Example 8-6), a reaction was carried out using 6-bromo-2-methylthiochroman, which was obtained in 4), instead of 6-bromothiochroman to afford the desired compound (yield 91%) as a white powder.
Nuclear magnetic resonance spectrum (270MHz, CDCl₃) δppm:
   7.77-7.75(2H,m), 7.14(1H,d,J=8Hz), 3.78-3.75(2H,m),
   3.47-3.42(3H,m), 2.96-2.87(2H,m), 1.40(3H,d,J=7Hz).

### 6) Methyl (2-methylthiochroman-6-carbonyl)acetate

In a similar manner to tat described in Example 1-5), a reaction was carried out using 2-methylthiochroman-6-carboxylic acid, which was obtained in 5), instead of 4-(2-acetoxyethylthio)-3-fluorobenzoic acid to afford the desired compound (yield 80%) as an orange oil.
Nuclear magnetic resonance spectrum (270MHz, CDCl₃) δppm:
   7.89-7.64(2H,m), 7.32-7.21(1H,m), 3.99(2H,s),
   3.85-3.80(3H,m), 3.66-3.50(1H,m), 3.05-2.88(2H,m),
   2.30-1.73(2H,m), 1.45(3H,d,J=7Hz).

### 7) 2-(4-Fluorophenyl)-5-(2-methylthiochroman-6-yl)-4-methoxycarbonyl-3-(pyridin-4-yl)-1H-pyrrole

In a similar manner to that described in Example 1-6), cyclization was carried out using methyl (2-methylthiochroman-6-carbonyl)acetate, which was obtained in 6), instead of methyl [4-(2-acetoxyethylthio)-3-fluoro]benzoylacetate to afford the desired compound (yield 23%) as a yellow powder.
Nuclear magnetic resonance spectrum (270MHz, CDCl₃) δppm:
   9.77(1H,br.s), 8.62(2H,d,J=5Hz), 7.91-7.22(7H,m),
   7.16(2H,t,J=9Hz), 3.76(3H,s), 3.67-3.61(1H,m),
   3.16-3.08(2H,m), 2.45-1.92(2H,m), 1.60(3H,d,J=7Hz).

### 8) 2-(4-Fluorophenyl)-5-(2-methylthiochroman-6-yl)-3-(pyridin-4-yl)-1H-pyrrole

In a similar manner to that described in Example 1-7a), hydrolysis and decarboxylation were carried out using 2-(4-fluorophenyl)-5-(2-methylthiochroman-6-yl)-4-methoxycarbonyl-3-(pyridin-4-yl)-1H-pyrrole, which was obtained in 7), to afford the desired compound (yield 24%) as a yellow powder.
Nuclear magnetic resonance spectrum (270MHz, CDCl₃) δppm:
   8.58-8.46(3H,m), 7.65-7.01(9H,m), 6.68(1H,m),
   3.49-3.43(1H,m), 2.92(2H,m), 2.25-1.68(2H,m),
   1.40(3H,d,J=7Hz).

### 9) 2-(4-Fluorophenyl)-5-(2-methyl-1-oxothiochroman-6-yl)-3-(pyridin-4-yl)-1H-pyrrole

In a similar manner to that described in Example 1-8), oxidation was carried out using 2-(4-fluorophenyl)-5-(2-methylthiochroman-6-yl)-3-(pyridin-4-yl)-1H-pyrrole, which was obtained in 8), to give the title compound (yield 83%) as a white powder.
Melting point: 238 - 243 °C
Nuclear magnetic resonance spectrum (270MHz, CDCl₃) δppm:
   11.00(0.4H,br.s), 10.51(0.6H,br.s), 8.43(2H,m),
   7.54-7.24(7H,m), 7.07(2H,t,J=9Hz), 6.75(1H,m),
   2.97-1.76(5H,m), 1.29(1.8H,d,J=7Hz), 1.21(1.2H,d,J=7Hz).

### [Example 28]

### 2-(4-Fluorophenyl)-5-(1,1-dioxo-2-methylthiochroman-6-yl)-3-(pyridin-4-yl)-1H-pyrrole (exemplification compound No. 2-118)

In a similar manner to that described in Example 1-8), oxidation was carried out using the compound of Example 27-8) and m-chloroperbenzoic acid (70%, two equivalents to the compound of Example 27-8) to give the title compound (yield 57%) as a white powder.
Melting point: >290 °C
Nuclear magnetic resonance spectrum (270MHz, DMSO-d₆) δppm:
   11.90(1H,br.s), 8.43(2H,d,J=5Hz), 7.90-7.80(3H,m),
   7.52-7.46(2H,m), 7.34-7.24(4H,m), 7.15(1H,d,J=2Hz),
   3.57-3.03(3H,m), 2.29-2.17(2H,m), 1.35(3H,d,J=7Hz).

### [Example 29]

### 2-(4-Fluorophenyl)-5-(1-oxohomothiochroman-7-yl)-3-(pyridin-4-yl)-1H-pyrrole

### (Exemplification compound No. 6-2)

### 1) 7-Bromothiochroman

Under ice-cooling, bromine (52µ1, 1 mmol) was added to a mixture of homothiochroman (known compound, 0.16 g, 1 mmol) and iron powder (2.5 mg). The mixture was stirred at the same temperature for 4 hours and then allowed to stand at room temperature overnight. Saturated aqueous sodium hydrogencarbonate was added to the reaction mixture and then it was extracted with diethyl ether. The organic layer was washed with water, dried over magnesium sulfate and then concentrated under reduced pressure to afford the desired compound (0.2 g, quantitative yield) as a pale yellow powder.
Nuclear magnetic resonance spectrum (270MHz, CDCl₃) δppm:
   7.39-7.36(2H,m), 7.22-7.19(1H,m), 2.99-2.95(2H,m),
   2.73-2.69(2H,m), 2.17-2.06(2H,m), 1.90-1.69(2H,m).

### 2) Homothiochroman-7-carboxylic acid

In a similar manner to that described in Example 8-6), a reaction was carried out using 7-bromohomothiochroman, which was obtained in 1), instead of 6-bromothiochroman to afford the desired compound (yield 51%) as a white powder.
Nuclear magnetic resonance spectrum (270MHz, CDCl₃) δppm:
   7.93(1H,m), 7.84-7.81(1H,m), 7.60(1H,d,J=8Hz),
   3.09-3.00(2H,m), 2.81-2.77(2H,m), 2.18-2.08(2H,m),
   1.76-1.72(2H,m).

### 3) Methyl (homothiochroman-7-carbonyl)acetate

In a similar manner to that described in Example 1-5), a reaction was carried out using homothiochroman-7-carboxylic acid, which was obtained in 2), instead of 4-(2-acetoxyethylthio)-3-fluorobenzoic acid to afford the desired compound (quantitative yield) as an orange oil.
Nuclear magnetic resonance spectrum (270MHz, CDCl₃) δppm:
   7.76(1H,m), 7.76-7.46(2H,m), 3.98(2H,s), 3.80-3.74(3H,m),
   3.08-3.04(2H,m), 2.81-2.74(2H,m), 2.10-2.03(2H,m),
   1.78-1.72(2H,m).

### 4) 2-(4-Fluorophenyl)-5-(homothiochroman-7-yl)-4-methoxycarbonyl-3-(pyridin-4-yl)-1H-pyrrole

In a similar manner to that described in Example 1-6), cyclization was carried out using methyl (homothiochroman-7-carbonyl)acetate, which was obtained in 3), instead of methyl [4-(2-acetoxyethylthio)-3-fluoro]benzoylacetate to afford the desired compound (yield 67%) as a yellow oil.
Nuclear magnetic resonance spectrum (270MHz, CDCl₃) δppm:
   8.95(1H,br.s), 8.49-8.45(2H,m), 7.65-6.90(9H,m),
   3.62-3.55(3H,m), 3.08-2.97(2H,m), 2.79-2.75(2H,m),
   2.12-2.10(2H,m), 1.75-1.64(2H,m).

### 5) 2-(4-Fluorophenyl)-5-(homothiochroman-7-yl)-3-(pyridin-4-yl)-1H-pyrrole

In a similar manner to that described in Example 1-7a), hydrolysis and decarboxylation were carried out using 2-(4-fluorophenyl)-5-(homothiochroman-7-yl)-4-methoxycarbonyl-3-(pyridin-4-yl)-1H-pyrrole, which was obtained in 4), to afford the desired compound (yield 15%) as a pale brown powder.
Nuclear magnetic resonance spectrum (270MHz, DMSO-d₆) δppm:
   8.36(2H,d,J=5Hz), 7.70(2H,m), 7.51-7.18(7H,m),
   6.92(1H,d,J=2Hz), 2.96-2.93(2H,m), 2.69-2.67(2H,m),
   1.98-1.94(2H,m), 1.71-1.60(2H,m).

### 6) 2-(4-Fluorophenyl)-5-(1-oxohomothiochroman-7-yl)-3-(pyridin-4-yl)-1H-pyrrole

In a similar manner to that described in Example 1-8), oxidation was carried out using 2-(4-fluorophenyl)-5-(homothiochroman-7-yl)-3-(pyridin-4-yl)-1H-pyrrole, which was obtained in 5), to give the title compound (yield 35%) as a white powder.
Melting point: 265 - 269 °C
Nuclear magnetic resonance spectrum (270MHz, DMSO-d₆) δppm:
   11.8(1H,br.s), 8.40(2H,m), 7.86(1H,d,J=8Hz), 7.75(1H,s),
   7.63(1H,d,J=8Hz), 7.50-7.45(2H,m), 7.32-7.23(4H,m),
   7.08(1H,m), 3.23-3.14(2H,m), 3.05-2.92(2H,m),
   2.72-2.63(1H,m), 2.21-2.14(2H,m), 1.89-1.86(1H,m).

### [Example 30]

### 5-(1,1-Dioxohomothiochroman-7-yl)-2-(4-fluorophenyl)-3-(pyridin-4-yl)-1H-pyrrole

### (Exemplification compound No. 6-9)

In a similar manner to that described in Example 1-8), oxidation was carried out using the compound of Example 29-5) and m-chloroperbenzoic acid (70%, two equivalents to the compound of Example 29-5)) to give the title compound (yield 38%) as a white powder.
Melting point: >290 °C
Nuclear magnetic resonance spectrum (270MHz, DMSO-d₆) δppm:
   11.89(1H,br.s), 8.43(2H,d,J=5Hz), 7.87-7.83(3H,m),
   7.51-7.46(2H,m), 7.37-7.19(5H,m), 3.35(2H,m),
   3.28-3.13(2H,m), 2.11(2H,m), 1.79-1.78(2H,m).

### [Example 31]

### 5-(2,2-Dimethyl-1-oxothiocbroman-6-yl)-2-(4-fluorophenyl)-3-(pyridin-4-yl)-1H-pyrrole (Exemplification compound No. 2-26)

### 1) 2,2-Dimethyl-4-hydroxythiochroman

In a similar manner to that described in Example 8-3), a reaction was carried out using 2,2-dimethyl-4-oxothiochroman (known compound) instead of 6-bromo-3,3-dimethyl-4-oxothiochroman to afford the desired compound (yield 93%) as a colorless oil.
Nuclear magnetic resonance spectrum (270MHz, CDCl₃) δppm:
   7.61-7.59(1H,m), 7.15-7.12(3H,m), 4.92-4.90(1H,m),
   2.29-1.86(3H,m), 1.45(6H,s).

### 2) 2,2-Dimethylthiochroman

In a similar manner to that described in Example 21-4), a reaction was carried out using 2,2-dimethyl-4-hydroxythiochroman, which was obtained in 1), instead of 6-bromo-3,3-dimethyl-4-hydroxythiochroman to afford the desired compound (yield 87%) as a colorless oil.
Nuclear magnetic resonance spectrum (270MHz, CDCl₃) δppm:
   7.11-6.95(4H,m), 2.92(2H,t,J=7Hz), 1.91(2H,t,J=7Hz),
   1.42(6H,s).

### 3) 6-Bromo-2,2-dimethylthiochroman

In a similar manner to that described in Example 29-1), a reaction was carried out using 2,2-dimethylthiochroman, which was obtained in 2), instead of homothiochroman to afford the desired compound (yield 85%) as a colorless oil.
Nuclear magnetic resonance spectrum (270MHz, CDCl₃) δppm:
   7.24(1H,d,J=2Hz), 7.16(1H,dd,J=8Hz,2Hz), 6.92(1H,d,J=8Hz),
   2.89(2H,t,J=6Hz), 1.89(2H,t,J=6Hz), 1.40(6H,s).

### 4) 2,2-Dimethylthiochroman-6-carboxylic acid

In a similar manner to that described in Example 8-6), a reaction was carried out using 6-bromo-2,2-dimethylthiochroman, which was obtained in 3), instead of 6-bromothiochroman to afford the desired compound (yield 84%) as a white powder.
Nuclear magnetic resonance spectrum (270MHz, CDCl₃) δppm:
   7.84(1H,m),7.79-7.76(1H,m), 7.13(1H,d,J=8Hz),
   2.99(2H,t,J=6Hz), 1.94(2H,t,J=6Hz), 1.44(6H,s).

### 5) Methyl (2,2-dimethylthiochroman-6-carbonyl)acetate

In a similar manner to that described in Example 1-5), a reaction was carried out using 2,2-dimethylthiochroman-6-carboxylic acid, which was obtained in 4), instead of 4-(2-acetoxyethylthio)-3-fluorobenzoic acid to afford the desired compound (yield 99%) as an orange oil.
Nuclear magnetic resonance spectrum (270MHz, CDCl₃) δppm:
   12.50(0.1H,s), 7.69-7.54(2H,m), 7.14-7.07(1H,m),
   5.61(0.1H,s), 3.95(1.8H,s), 3.79(0.3H,s), 3.75(2.7H,s),
   2.98(2H,t,J=6Hz), 1.93(2H,t,J=6Hz), 1.44(6H,s).

### 6) 5-(2,2-Dimethylthiochroman-6-yl)-2-(4-fluorophenyl)-4-methoxycarbonyl-3-(pyridin-4-yl)-1H-pyrrole

In a similar manner to that described in Example 1-6), cyclization was carried out using methyl (2,2-dimethylthiochroman-6-carbonyl)acetate, which was obtained in 5), instead of methyl [4-(2-acetoxyethylthio)-3-fluoro]benzoylacetate to afford the desired compound (yield 17%) as a yellow oil.
Nuclear magnetic resonance spectrum (270MHz, CDCl₃) δppm:
   8.86(1H,br.s), 8.47(2H,d,J=4Hz), 7.34-7.03(7H,m),
   6.97(2H,t,J=9Hz), 3.55(3H,s), 2.97(2H,t,J=6Hz),
   1.94(2H,t,J=6Hz), 1.44(6H,s).

### 7) 5-(2,2-Dimethylthiochroman-6-yl)-2-(4-fluorophenyl)-3-(pyridin-4-yl)-1H-pyrrole

In a similar manner to that described in Example 1-7a), hydrolysis and decarboxylation were carried out using 5-(2,2-dimethylthiochroman-6-yl)-2-(4-fluorophenyl)-4-methoxycarbonyl-3-(pyridin-4-yl)-1H-pyrrole, which was obtained in 6), to afford the desired compound (yield 71%) as a brown powder.
Nuclear magnetic resonance spectrum (270MHz, CDCl₃) δppm:
   8.66(1H,br.s), 8.43(2H,d,J=6Hz), 7.40-7.17(6H,m),
   7.11-7.01(3H,m), 6.68(1H,d,J=2Hz), 2.97(2H,t,J=6Hz),
   1.95(2H,t,J=6Hz), 1.44(6H,s).

### 8) 5-(2,2-Dimethyl-1-oxothiochroman-6-yl)-2-(4-fluorophenyl)-3-(pyridin-4-yl)-1H-pyrrole

In a similar manner to that described in Example 1-8), oxidation was carried out using 5-(2,2-dimethylthiochroman-6-yl)-2-(4-fluorophenyl)-3-(pyridin-4-yl)-1H-pyrrole, which was obtained in 7), to give the title compound (yield 45%) as a pale yellow powder.
Melting point: 236 - 239 °C (decomposition)
Nuclear magnetic resonance spectrum (270MHz, DMSO-d₆) δppm:
   11.80(1H,br.s), 8.42(2H,d,J=6Hz), 7.84-7.74(2H,m),
   7.66(1H,d,J=8Hz), 7.51-7.46(2H,m), 7.33-7.24(4H,m),
   7.10(1H,m), 3.02-2.95(2H,m), 2.26-2.15(1H,m),
   1.87-1.77(1H,m), 1.23-1.22(6H,m).

### [Example 32]

### 5-(2,2-Dimethyl-1,1-dioxothiochroman-6-yl)-2-(4-fluorophenyl)-3-(pyridin-4-yl)-1H-pyrrole (Exemplification compound No. 2-123)

In a similar manner to that described in Example 1-8), oxidation was carried out using the compound of Example 31-7) and m-chloroperbenzoic acid (70%, two equivalents to the compound of Example 31-7)) to give the title compound (yield 65%) as a white powder.
Melting point: >290 °C
Nuclear magnetic resonance spectrum (270MHz, DMSO-d₆) δppm:
   11.90(1H,s), 8.41(2H,d,J=5Hz), 7.89-7.78(3H,m),
   7.50-7.45(2H,m), 7.32-7.23(4H,m),
   7.14(1H,s), 3.04(2H,t,J=6Hz), 2.26(2H,t,J=6Hz), 1.36(6H,s).

### [Example 33]

### 2-(4-Fluorophenyl)-5-(4-fluoro-1-oxothiochroman-6-yl)-3-(pyridin-4-yl)-1H-pyrrole

### (Exemplification compound No. 2-30)

(Diethylamino)sulfur trifluoride (DAST, 63µl, 0.48 mmol) was added to a solution of the compound of Example 13 (200 mg, 0.48 mmol) in dichloromethane (20 ml) at -78°C. The mixture was stirred from -78°C to room temperature for 8 hours. To the reaction mixture, saturated aqueous sodium hydrogencarbonate solution was added and then it was extracted with ethyl acetate. The organic layer was washed with water and dried over anhydrous magnesium sulfate and concentrated under reduced pressure. The residue was purified by chromatography on a silica gel column using ethyl acetate as the eluant to give the title compound (83 mg, yield 42%) as a yellow powder.
Melting point: 175 - 180 °C
Nuclear magnetic resonance spectrum (270MHz, CDCl₃) δppm:
   9.27(1H,br.s), 8.48(2H,d,J=5Hz), 7.72-7.65(3H,m),
   7.43(2H,dd,J=9Hz,5Hz), 7.23(2H,d,J=6Hz), 7.11(2H,t,J=9Hz),
   6.86(1H,d,J=3Hz), 5.63(1H,dt,J=49Hz,3Hz), 3.33-3.02(2H,m),
   2.93-2.80(1H,m), 2.50-2.37(1H,m).

### [Example 34]

### 2-(4-Fluorophenyl)-5-(4-hydroxyimino-1-oxothiochroman-6-yl)-3-(pyridin-4-yl)-1H-pyrrole (Exemplification compound No. 6-16)

Sodium carbonate (69 mg, 0.5 mmol) and hydroxyamine hydrochloride (69 mg, 1.0 mmol) were added to a solution of the compound of Example 12-6) (200 mg, 0.5 mmol) in a mixture of tetrahydrofuran (7 ml), ethanol (7 ml) and water (7 ml). The mixture was stirred at 60°C for 3 hours and further at room temperature overnight. The reaction mixture was concentrated under reduced pressure. To the residue was added water and the precipitate was filtered to afford crude oxime derivative. The crude product was oxidized in a similar manner to that described in Example 1-8) to give the title compound (92 mg, yield 48%) as a pale yellow powder.
Melting point: 257 - 262 °C
Nuclear magnetic resonance spectrum (400MHz, CDCl₃) δppm:
   11.26(1H,br.s), 11.08(1H,s), 8.25-8.23(3H,m),
   7.68(1H,dd,J=8Hz,2Hz), 7.54(1H,d,J=8Hz), 7.31-7.27(2H,m),
   7.06-7.02(2H,m), 6.89(2H,t,J=9Hz), 6.77(1H,d,J=3Hz),
   4.00-3.93(1H,m), 3.48-3.39(1H,m), 3.28-3.16(2H,m).

### [Example 35]

### 2-(4-Fluorophenyl)-5-(4-methoxyimino-1-oxothiochroman-6-yl)-3-(pyridin-4-yl)-1H-pyrrole (Exemplification compound No. 6-30)

In a similar manner to that described in Example 34, a reaction was carried out using O-methylhydroxyamine hydrochloride instead of hydroxyamine hydrochloride and then oxidation of the resulting oxime product was carried out to give the title compound (yield 40%) as a pale yellow powder.
Melting point: 255 - 259 °C
Nuclear magnetic resonance spectrum (270MHz, CDCl₃) δppm:
   9.05(1H,br.s), 8.48(2H,d,J=6Hz), 8.16(1H,d,J=2Hz),
   7.75(1H,d,J=8Hz), 7.66(1H,dd,J=8Hz,2Hz),
   7.43(2H,dd,J=9Hz,5Hz), 7.25(2H,d,J=6Hz),
   7.12(2H,t,J=9Hz), 6.87(1H,d,J=3Hz), 4.08(3H,s),
   3.38-3.02(4H,m).

### [Formulation Example]

Pharmaceutical formulations containing the compound of the present invention or a pharmacologically acceptable salt or derivative thereof as an active ingredient are prepared as follows:

### [Formulation Example 1] Powder

Powders can be obtained by mixing the compound of Example 1 (5 g), lactose (895 g) and corn starch (100 g) in a blender.

### [Formulation Example 2] Granules

Granules can be prepared by mixing the compound of Example 2 (5 g), lactose (865 g) and low-substituted hydroxylpropylcellulose (100 g), adding 300g of a 10% aqueous solution of hydroxypropylcellulose to the mixture, kneading the mixture, granulating the kneaded mass using an extrusion granulator and then drying the granulated product.

### [Formulation Example 3] Capsules

Capsules can be obtained by mixing the compound of Example 3 (5 g), lactose (115 g), corn starch (58 g) and magnesium stearate (2 g) in a V-shaped mixer and then filling the resulting mixture, in 180 mg portions, into No. 3 capsules.

### [Formulation Example 4] Tablets

Tablets can be obtained by mixing the compound of Example 4 (5 g), lactose (90 g), corn starch (34 g), crystalline cellulose (20 g) and magnesium stearate (1 g) in a blender and then tableting the resulting mixture using a tableting machine.

### [Test examples]

### [Test example 1]

### Test on the inhibition of the production of IL-1β and TNFα in human whole blood (in vitro)

The test was carried out in a similar manner to that described by Hartman et al. (D.A. Hartman, S.J. Ochalski and R.P. Carison: The effects of antiinflammatory and antiallergic drugs on cytokine release after stimulation of human whole blood by lipopolysaccharide and zymosan A: Inflamm. Res., 44, 269(1995)).

Blood was collected from the peripheral vessel of healthy volunteers in the presence of heparin. In an Eppendorf tube to which 2 µl of a solution of the test compound in dimethylsulfoxide had been added in advance, 1000 µl of the whole blood were charged, followed by the addition of 10 µl of lipopolysaccharide (LPS) (E. coli 026: derived from B6, product of Difco Laboratories) (final concentration: 10 µg/ml) as a stimulant. The resulting mixture was mixed thoroughly and then incubated under the conditions of 37°C and 5% CO₂ for 6 hours. After the end of this time, the reaction was terminated by cooling to 4°C. Immediately after that, the reaction mixture was centrifuged at 14,000 rpm for 5 minutes and the supernatant plasma was collected. The IL-1β and TNFα released in the plasma were determined using an enzyme immunoassay (ELISA) kit (product of Cayman Corp.and Genzyme Corp.). The inhibition rate was calculated from the amount of each of the produced cytokines in the presence or absence of the test compound.

In the above test, the compounds according to the present invention exhibited excellent inhibitory activity against the production of cytokines.

### [Test example 2]

### Test on the inhibition of the production of TNFα (in vivo)

This test was carried out in a similar manner to that described by Ochalski et al. (S.J. Ochalski, D.A. Hartman, M.T. Belfast, T.L. Walter, K.B. Glaser and R.P. Carlson; Inhibition of endotoxin-induced hypothermia and serum TNF-α levels in CD-1 mice by various pharmacological agents: Agents Actions 39, C52-C54(1993)).

The production of TNFα was induced by the intravenous injection of LPS to a mouse. To the caudal vein of a Balb/c mouse (male, 5 to 7 weeks old, about 22 g in weight, purchased from Nippon Charles River) fasted overnight from the day before the test, 10 ml/kg body weight of LPS (E. coli O26: derived from B6, product of Difco Laboratories) prepared to give a concentration of 0.045 mg/ml with physiological saline was administered. One hour after the administration, the mouse was subjected to laparotomy under anesthesia with ether and the blood was collected from the abdominal vein. For the blood collection, a 1-ml disposable syringe equipped with a 23G needle and having an inner wall wetted with heparin was employed. Immediately after the blood collection, the blood was transferred into a 1.5-ml Eppendorf tube, followed by centrifugation under the conditions of 4°C and 14000 rpm to separate the plasma. The plasma was stored at -20°C until the assay of TNFα.

The TNFα was quantitatively analyzed using an enzyme immunoassay (ELISA) kit (mouse TNFα ELISA KIT, product of Genzyme Corp.).

The test compound was suspended in a 0.5% tragacanth solution. The resulting suspension was orally administered at a dose of 10 ml/kg weight 30 minutes before the injection of LPS. Each of at least 3 doses per test compound was administered to 5 mice. For each dose, an average inhibition rate relative to the control group was calculated.

In the above test, the compound of the present invention exhibited excellent inhibitory activity against the production of TNFα.

### [Test example 3]

### Test on the inhibition of the production of IL-1β (in vivo)

This test was carried out in a similar manner to that described by Griffiths et al. (Richard J. Griffiths, Ethan J. Stam, James T. Downs and Ivan G. Otterness; ATP Induces the Release of IL-1 from LPS-Primed Cells In Vivo: J. Immunol., 154, 2821-2828(1995)).

LPS and adenosine triphosphate (ATP) were intraperitoneally administered to a mouse to induce the production of IL-1β. LPS (E. coli O26: derived from B6, product of Difco Laboratories), prepared to give a concentration of 0.0045 mg/ml with physiological saline, was intraperitoneally administered to a Balb/c mouse (male, 5 to 7 weeks old, about 22 g, purchased from Nippon Charles River), which had been fasted overnight from the day before the test, at a dose of 10 ml/kg body weight. Two hours later, 0.5 ml of ATP prepared to give a concentration of 6.03 mg/ml with physiological saline were intraperitoneally administered. The mouse was stifled with dry ice 0.5 hour after the administration of ATP and, immediately after that, 3 ml of PBS (containing 10 U/ml of heparin, 0.25 mM of PMSF, 1 µg/ml of leupepsin, 1 µg/ml of pepstatin and 1 mM of EDTA) were intraperitoneally injected to wash the abdominal cavity. The washing solution was collected by a 1-ml disposable syringe equipped with a 21G needle. The washing solution from the abdominal cavity was transferred to a 1.5-ml Eppendorf tube just after the collection and centrifuged at 4°C and 7,500 rpm, to separate the supernatant. The resulting supernatant was stored at -20°C until the assay of IL-1β.

The amount of IL-1β was assayed using an enzyme immunoassay (ELISA) kit (mouse ELISA KIT, product of Genzyme Corp.).

The test compound was suspended in a 0.5% tragacanth solution. The resulting suspension was orally administered at a dose of 10 ml/kg 30 minutes before the injection of LPS. Each of at least 3 doses per test compound was administered to 5 mice. For each dose, an average inhibition rate relative to the control group was calculated.

In the above test, the compound of the present invention exhibited excellent inhibitory activity against the production of IL-1β

### [Capability of Utility in Industry]

The compounds according to the present invention have excellent inhibitory activity against inflammatory cytokines (particularly, inhibitory activity against the production of IL-1β and TNFα), good oral absorption and low toxicity and they are effective as a medicament, particularly as a drug for the prevention or treatment of the diseases which inflammatory cytokines take part in. More specifically, the compounds of the present invention are useful as an analgesic, an antiinflammatory drug, a virucide, and an agent for the prevention or treatment of chronic rheumatism, osteoarthritis, allergosis, asthma, sepsis, psoriasis, osteoporosis, autoimmune diseases (e.g. systemic lupus erythematosus, ulcerative colitis, Crohn's disease etc.), diabetes, glomerular nephritis or arteriosclerosis, particularly as an analgesic, an anti-inflammatory drug and an agent for the prevention or treatment of chronic rheumatism, osteoarthritis, allergosis, sepsis, psoriasis, osteoporosis, ulcerative colitis, diabetes or arteriosclerosis.

## Claims

1. A compound represented by the following formula (I): 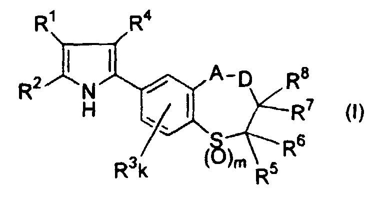 [wherein,
A represents a single bond, an oxygen atom, a sulfur atom, a carbonyl group, -SO-, -SO₂-, -C(R⁹)(R¹⁰)- (in which R⁹ and R¹⁰ are the same or different and each independently represents a hydrogen atom, a hydroxyl group, a halogen atom or a lower alkyl group), -N(R¹¹)- (in which R¹¹ represents a hydrogen atom or a lower alkyl group), or =C=NOR¹¹ (in which R¹¹ has the same meaning as described above),
D represents a single bond or -C(R¹²)(R¹³)- (in which R¹² and R¹³ are the same or different and each independently represents a hydrogen atom, a hydroxyl group, a halogen atom or a lower alkyl group),
R¹ represents an unsubstituted pyridyl group or a pyridyl group substituted with at least one group selected from Substituent group α,
R² represents an unsubstituted phenyl group or a phenyl group substituted with at least one group selected from Substituent group α,
R³ represents a halogen atom, a lower alkyl group, a lower halogeno alkyl group, a lower alkoxy group or a lower halogeno alkoxy group,
R⁴, R⁵, R⁶, R⁷ and R⁸ are the same or different and each independently represents a hydrogen atom or a lower alkyl group,
k is an integer of 0 to 3 (when k is 2 or 3, the plural R³ groups may be the same or different) and
m is 1 or 2;
Substituent group α:
a halogen atom, a lower alkyl group, a lower halogeno alkyl group, a lower alkoxy group, a lower halogeno alkoxy group, a lower alkylthio group];
and a pharmacologically acceptable salt or derivative thereof.

2. A compound or a pharmacologically acceptable salt or derivative thereof according to claim 1, wherein R¹ represents an unsubstituted 4-pyridyl group or a 4-pyridyl group substituted with at least one group selected from Substituent group α.

3. A compound or a pharmacologically acceptable salt or derivative thereof according to claim 1, wherein R¹ represents an unsubstituted 4-pyridyl group.

4. A compound or a pharmacologically acceptable salt or derivative thereof according to any one of claims 1 to 3, wherein R² represents an unsubstituted phenyl group or a phenyl group substituted with 1 to 3 substituents selected from Substituent group α.

5. A compound or a pharmacologically acceptable salt or derivative thereof according to any one of claims 1 to 3, wherein R² represents an unsubstituted phenyl group or a phenyl group substituted with 1 to 3 substituents selected from the below-described Substituent group α¹;
Substituent group α¹:
a halogen atom, a C₁₋₄ alkyl group, a C₁₋₄ halogenoalkyl group, a C₁₋₄ alkoxy group, a C₁₋₄ halogenoalkoxy group, C₁₋₄ alkylthio group.

6. A compound or a pharmacologically acceptable salt or derivative thereof according to any one of claims 1 to 3, wherein R² represents an unsubstituted phenyl group or a phenyl group substituted with 1 to 3 substituents selected from the below-described Substituent group α²;
Substituent group α²:
a fluorine atom, a chlorine atom, a difluoromethoxy group.

7. A compound or a pharmacologically acceptable salt or derivative thereof according to any one of claims 1 to 6, wherein R³ represents a halogen atom, a C₁₋₄ alkyl group, a C₁₋₄ halogenoalkyl group, a C₁₋₄ alkoxy group or a C₁₋₄ halogenoalkoxy group.

8. A compound or a pharmacologically acceptable salt or derivative thereof according to any one of claims 1 to 6, wherein R³ represents a fluorine atom, a chlorine atom, a methyl group, a methoxy group or a difluoromethoxy group.

9. A compound or a pharmacologically acceptable salt or derivative thereof according to any one of claims 1 to 8, wherein k is 0.

10. A compound or a pharmacologically acceptable salt or derivative thereof according to any one of claims 1 to 9, wherein R⁴ represents a hydrogen atom or a C₁₋₄ alkyl group.

11. A compound or a pharmacologically acceptable salt or derivative thereof according to any one of claims 1 to 9, wherein R⁴ represents a hydrogen atom, a methyl group or an ethyl group.

12. A compound or a pharmacologically acceptable salt or derivative thereof according to any one of claims 1 to 11, wherein R⁵, R⁶, R⁷ and R⁸ are the same or different and each independently represents a hydrogen atom or a C₁₋₄ alkyl group,

13. A compound or a pharmacologically acceptable salt or derivative thereof according to any one of claims 1 to 11, wherein R⁵, R⁶, R⁷ and R⁸ are the same or different and each independently represents a hydrogen atom or a methyl group.

14. A compound or a pharmacologically acceptable salt or derivative thereof according to any one of claims 1 to 13, wherein R⁹ and R¹⁰ are the same or different and each independently represents a hydrogen atom, a hydroxyl group, a halogen atom or a C₁₋₄ alkyl group.

15. A compound or a pharmacologically acceptable salt or derivative thereof according to any one of claims 1 to 13, wherein R⁹ and R¹⁰ are the same or different and each independently represents a hydrogen atom, a hydroxyl group, a fluorine atom, a methyl group or an ethyl group.

16. A compound or a pharmacologically acceptable salt or derivative thereof according to any one of claims 1 to 15, wherein R¹¹ represents a hydrogen atom or a C₁₋₄ alkyl group.

17. A compound or a pharmacologically acceptable salt or derivative thereof according to any one of claims 1 to 15, wherein R¹¹ represents a hydrogen atom, a methyl group or an ethyl group.

18. A compound or a pharmacologically acceptable salt or derivative thereof according to any one of claims 1 to 17, wherein A represents a single bond, an oxygen atom, a carbonyl group, -SO-, SO₂-, -C(R⁹)(R¹⁰)-, -N(R¹¹)- or =C=NOR¹¹.

19. A compound or a pharmacologically acceptable salt or derivative thereof according to any one of claims 1 to 17, wherein A represents a single bond, an oxygen atom, a carbonyl group, -SO₂-, -C(R⁹)(R¹⁰)-, -N(R¹¹)- or =C=NOR¹¹.

20. A compound or a pharmacologically acceptable salt or derivative thereof according to any one of claims 1 to 17, wherein A represents a single bond, an oxygen atom, a carbonyl group, -C(R⁹)(R¹⁰)-, -N(R¹¹)- or =C=NOR¹¹.

21. A compound or a pharmacologically acceptable salt or derivative thereof according to any one of claims 1 to 17, wherein A represents a single bond, an oxygen atom, a carbonyl group, -C(R⁹)(R¹⁰)- or =C=NOR¹¹.

22. A medicament comprising a compound or a pharmacologically acceptable salt or derivative thereof as described in any one of claims 1 to 21 as an effective ingredient.

23. An antiinflammatory drug comprising a compound or a pharmacologically acceptable salt or derivative thereof as described in any one of claims 1 to 21 as an effective ingredient

24. A medicament for prevention or treatment of chronic rheumatism, which comprises a compound or a pharmacologically acceptable salt or derivative thereof as described in any one of claims 1 to 21 as an effective ingredient.

25. A medicament for prevention or treatment of osteoarthritis, which comprises a compound or a pharmacologically acceptable salt or derivative thereof as described in any one of claims 1 to 21 as an effective ingredient.
